# EUROPEAN PATENT SPECIFICATION

(11) **EP 3 143 137 B1**
(45) Date of publication and mention of the grant of the patent: **21.10.2026**
(21) Application number: 15793514.9
(22) Date of filing: 14.05.2015
(51) Int. Cl.: C12N 9/52, A61K 38/48, A61P 31/04

(54) **DEIMMUNIZED LYSOSTAPHIN AND METHODS OF USE**
DEIMMUNISIERTES LYSOSTAPHIN UND VERFAHREN ZUR VERWENDUNG
LYSOSTAPHINE DÉSIMMUNISÉE ET MÉTHODES D'UTILISATION

(30) Priority: 14.05.2014 US 201461993056 P; 27.05.2014 US 201462003256 P; 12.02.2015 US 201562115326 P; 30.04.2015 US 201562155079 P
(43) Date of publication of application: 22.03.2017
(73) Proprietor: Trustees of Dartmouth College, Hanover, NH 03755 (US); Stealth Biologics, LLC, Lyme, NH 03768 (US)
(72) Inventor: GRISWOLD, Karl E., Lyme, NH 03768 (US); BAILEY-KELLOGG, Chris, Strafford, VT 05072 (US); CHOI, Yoonjoo, Namyangju-si Gyeonggi-do 472-905 (KR); BLAZANOVIC, Kristina, 76239 Crkvina (BA); ZHAO, Hongliang, Hanover, NH 03755 (US); VERMA, Deeptak, Wilder, VT 05088 (US)
(74) Representative: Callaghan, Dayle Anne
(86) International application number: PCT/US2015/030765
(87) International publication number: WO 2015/175774

(56) References cited:
- WO-A1-2012/150858
- WO-A1-99/05289
- WO-A2-2008/105826
- US-A- 6 028 051
- US-B1- 7 091 332
- ZHAO, H ET AL.: "Gene And Protein Sequence Optimization For High-Level Production Of Fully Active And Aglycosylated Lysostaphin In Pichia Pastoris.", APPL ENVIRON MICROBIOL., vol. 80, no. 9, 21 February 2014 (2014-02-21), pages 2746 - 2753, XP055237738
- HUANG. C ET AL.: "Site-Specific N-Glycosylation Of Caprine Lysostaphin Restricts Its Bacteriolytic Activity Toward Staphylococcus Aureus.", ANIM BIOTECHNOL., vol. 24, no. 2, 27 March 2013 (2013-03-27), pages 129 - 147, XP055237740
- DE GROOT, AS ET AL.: "Beyond Humanization And De-Immunization: Tolerization As A Method For Reducing The Immunogenicity Of Biologics", EXPERT REV CLIN PHARMACOL., vol. 6, no. 6, November 2013 (2013-11-01), pages 651 - 662, XP055237741

## Description

### Background

*Staphylococcus aureus* colonizes the skin and mucosal membranes of humans and animals, and together with the other members of the genus *Staphylococcus,* has been implicated in a diverse array of infections. *S*. *aureus* contains many virulence factors including surface proteins designated as "microbial surface components recognizing adhesive matrix molecules," which facilitate attachment to surfaces and initiate infection (Gordon & Lowy (2008) Clin. Infect. Dis. 46:S350-S359). *S. aureus* can also form biofilms (Donlan & Costerton (2002) Clin. Microbiol. Rev. 15:167-193), which allow it to evade both the immune system and antibiotics. Most strains have a polysaccharide capsule and secrete a variety of enzymes that are used during infection to enhance bacterial spreading (Foster (2005) Nat. Rev. Microbiol. 3: 948-958). *S. aureus* can also cause toxic shock syndrome, and studies have shown that the peptidoglycan and lipoteichoic acid of the *S. aureus* cell wall act together to cause toxic shock in rats (Kimpe, et al. (1995) Proc. Natl. Acad. Sci. USA 92:10359-10363).

Antibiotic resistance in staphylococci appeared after penicillin was first used for treatment of staphylococcal infections. This development of resistance, which was present in over 80% of clinical isolates by the late 1960s (Lowy (2003) J. Clin. Invest. 111:1265-1273), prompted the development of new, more potent drugs to combat the opportunistic pathogen. These efforts led to production of methicillin, a narrow spectrum penicillinase-resistant drug designed to alleviate the burden of staphylococcal infections. However, it took only a year for the first methicillin-resistant *S. aureus* (MRSA) clinical isolates to be discovered.

Initially, MRSA infections were only associated with prolonged hospital treatment and invasive surgical procedures, and were classified as Health Care-Acquired MRSA (HCA-MRSA). However, in recent years, MRSA has also emerged as a community-acquired infection (CA-MRSA), which affects groups with high-intensity physical contact, such as competitive athletes, military recruits, and children in daycare centers (Romano, et al. (2006) J. Athl. Train. 41:141-145; Kazakova, et al. (2005) New Engl. J. Med. 352:468-475; Zinderman, et al. (2004) Emerg. Infect. Dis. 10:941-944; Adcock, et al. (1998) J. Infect. Dis. 178:577-580).

The *S. aureus* cell wall is composed of alternating polysaccharide subunits of N-acetylglucosamine and N-acetylmuramic acid, wherein each N-acetylmuramic acid is connected to a peptide chain. Cross-linking of the peptidoglycan is achieved by four major penicillin-binding proteins (PBP1, 2, 3 and 4) that connect the muropeptide chains via pentaglycine interpeptide bridge. Methicillin resistance arose when *S. aureus* acquired the mecA gene, which encodes for penicillin-binding protein PBP2A that has transpeptidase activity but lower affinity for penicillin and β-lactam antibiotics. Resistant cells still produce PBPs, but given the expression of PBP2A, peptidoglycan synthesis continues in the presence of methicillin and other β-lactams (Hiramatsu, et al. (2001) Trends Microbiol. 9:486-493).

Lysostaphin is a glycyl-glycine zinc-dependent endopeptidase produced by *Staphylococcus simulans,* which selectively targets pentaglycine interpeptide cross-bridges. The gene for lysostaphin has been isolated and characterized. Genetic truncations have been made to remove the 36-residue signal peptide and 224-residue long propeptide thereby facilitating fusion to either an initiating methionine for intracellular expression or an exogenous signal sequence, *e.g.,* to permit the secretion of a single species of lysostaphin into the periplasmic space of *E. coli* (See, *e.g.,* US 2005/0118159). The mature, 247-residue enzyme is composed of N-terminal catalytic domain (138 amino acids), which is connected to the C-terminal cell wall binding domain (92 amino acids) via an 18-residue linker (Lu, et al. (2013) Antimicrob. Agents Chemother. 57:1872-1881).

Lysostaphin has shown promise as a therapeutic agent for treatment of *S. aureus* infections. The protein has been shown to lyse staphylococcal strains (Schindler & Schuhardt (1964) Proc. Natl. Acad. Sci. USA 51:414421) and clinical isolates (Cropp & Harrison (1964) Can. J. Microbiol. 10:823-828), and demonstrated remarkable efficacy in animal models (Schuhardt & Schindler (1964) J. Bacteriol. 88:815-816; Schaffner, et al. (1967) Yale J. Biol. Med. 39:230-244; Goldberg, et al. (1967) Antimicrob. Agents Chemother. 7:45-53; Kokai-Kun, et al. (2007) J. Antimicrob. Ther. 60:1051-1059; Placencia, et al. (2009) Ped. Res. 65:420-424; Climo, et al. (1998) Antimicrob. Agents Chemother. 42:1355-60), including those of staphylococcal biofilms (Kokai-Kun, et al. (2009) J. Antimicrob. Ther. 64:94-100). In several of these studies, antibodies against lysostaphin were observed in animals subjected to the drug for a prolonged period of time (Climo, et al. (1998) Antimicrob. Agents Chemother. 42:1355-1360). Similarly, human clinical trials with intranasal lysostaphin indicated a slight elevation in anti-lysostaphin antibody titer (Kokai-Kun (2012) in Antimicrobial Drug Discovery: Emerging Strategies (Tegos & Mylonakis, eds) Ch. 10, 147-165).

In an attempt to improve pharmacokinetics and reduce immunogenicity, lysostaphin has been linked to branched polyethylene glycol (PEG). While PEGylation reduced immunoreactivity, PEGylation of the enzyme significantly reduced its activity (Walsh, et al. (2003) Antimicrob. Agents Chemother. 47:554-558). In addition, US 2008/0095756 describes the deimmunization of the cell wall binding domain of lysostaphin. However, variants with a deimmunized catalytic domain are not described.
Zhao et al., "Gene and protein sequence optimization for highlevel production of fully active and aglycosylated Lysostaphin in Pichia Pastoris", Applied and Environmental Microbiology, vol 80, no. 9, February 2014 discloses optimization of lysostaphin variants in a high level expression system.
WO 2008/105826 discloses compositions comprising lysostaphin variants and methods of using the same.
US 7091332 discloses methods and reagents for expressing in mammalian cells proteins that have anti-microbial activity. Huang et al., "Site-specific N-glycosylation of Caprine Lysostaphin restricts its bacteriolytic activity towards Staphylococcus aureus", Animal Biotechnology, vol 24, no. 2, March 2013 discloses site-specific glycosylation and effects on bacteriolytic activity.
De Groot et al., "Beyond humanization and de-immunization: Tolerization as a method for reducing the immunogenicity of biologics", Expert Review of Clinical Pharmacology, vol 6, no. 6, November 2013 discloses a method to reduce immunogenicity of biologics by introduction of Treg epitopes. WO 2012/150858 discloses a polypeptide for use in treating an infectious disease or for use as an antimicrobial.
WO 99/05289 discloses lysostaphin analogs having a molecular structure preventing post-translational modifications while retaining lysostaphin-like biological activity.
US 6028051 discloses administration of lysostaphin for the purpose of treatment of staphylococcus infection.

### Summary of the Invention

The subject matter for which protection is sought is as defined in the claims.

This invention provides a deimmunized lysostaphin having a mutation at Ser124Gly; Ser122Asp, Ser122Gly, or Ser122Thr; Asn121Gly; Val120Asp; Arg118Thr; Ile99Gln; Lys95Glu; Tyr93His; Ser84Tyr or Ser84Gly; Leu83Met; Val75Glu or Val75Gln; Asn72His; Ile70Lys; Lys46His; Ile41Glu; Asn40Thr; Phe38Ser; Tyr33Thr; Asn13His; Asn12Gly; or a combination thereof of SEQ ID NO:49. A pharmaceutical composition containing the deimmunized lysostaphin and an antibiotic is provided, as is a method preventing or treating a microbial infection.

### Brief Description of the Drawings

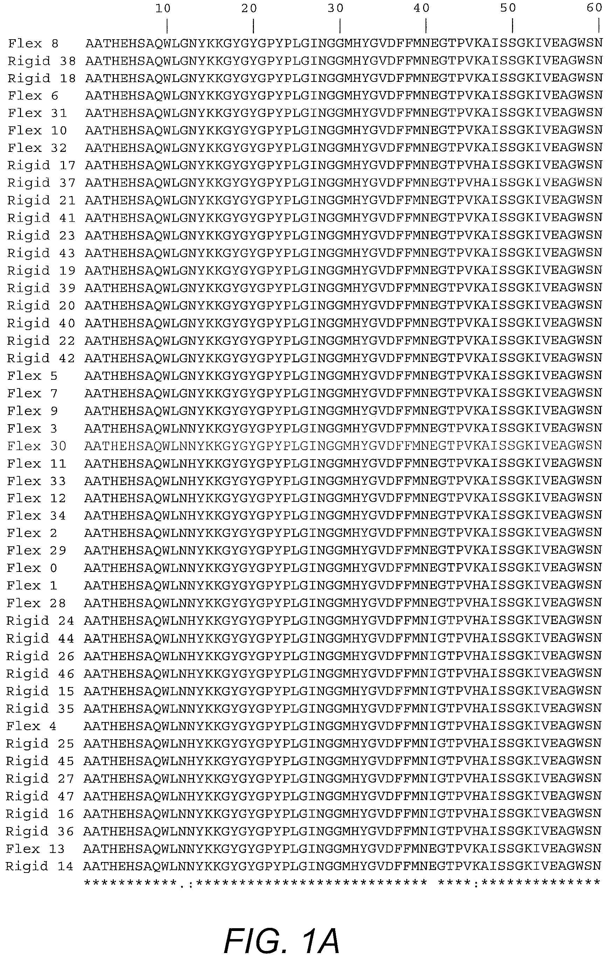

Figures 1A-1E show a sequence alignment of lysostaphin variants of the present invention.

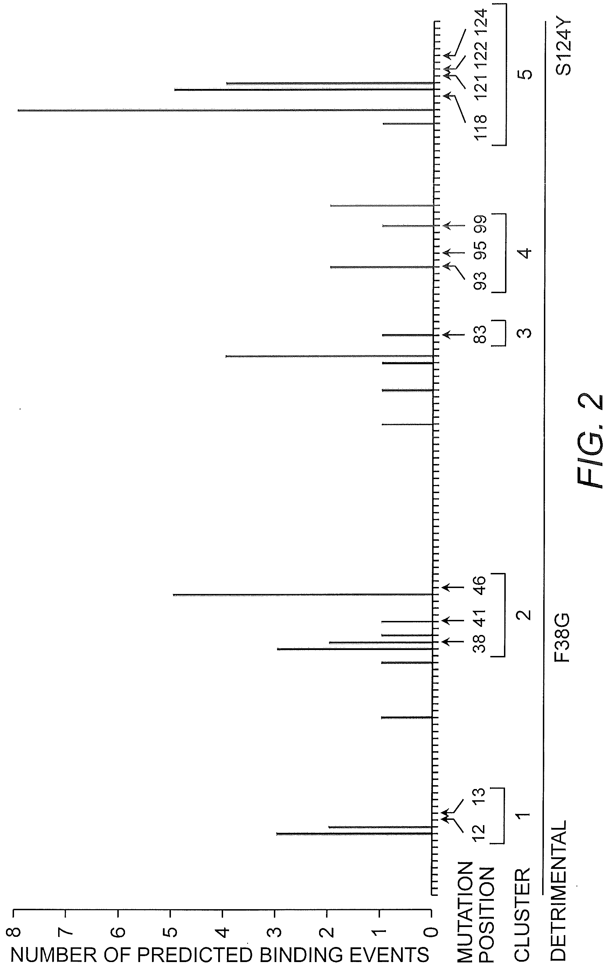

Figure 2 shows an epitope map of the lysostaphin catalytic domain. The total number of predicted binding events is plotted against the lysostaphin primary sequence. Using EpiMatrix, epitopes were predicted for MHC II alleles DRB*0101, 0301, 0401, 0701, 0801, 1101, 1301, and 1501. The maximum score is 8 and represents an epitope that is predicted to bind all 8 alleles. Such an epitope was observed at position 116. The sites of EpiSweep mutations are indicated with arrows and residue numbers. Epitope groups are divided into five distinct clusters. Mutations found to be detrimental for lysostaphin expression and activity that were later dropped (Phe38Gly and Ser124Tyr) are also indicated.

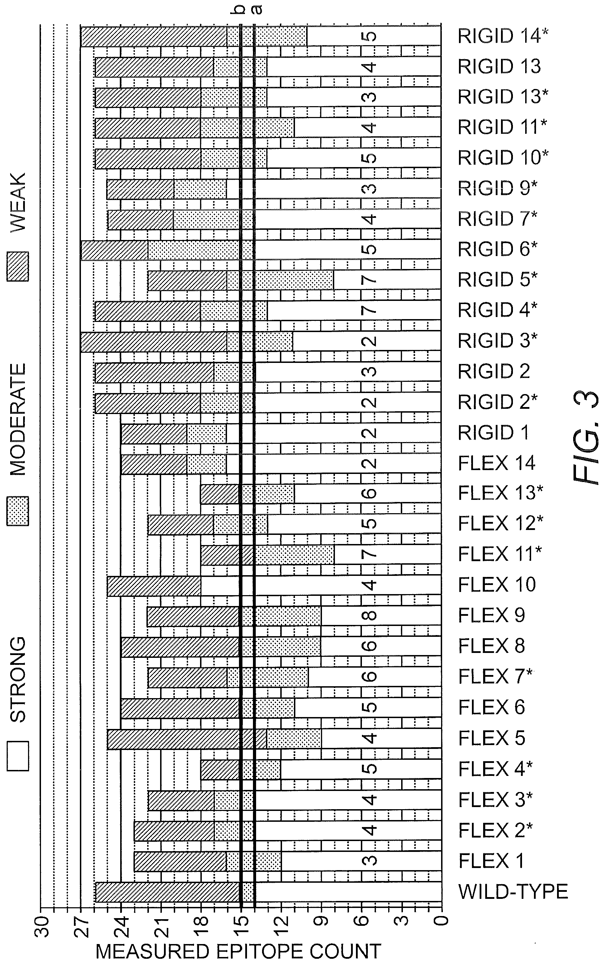

Figure 3 shows an aggregate of immunogenicity scores calculated for full-length designs. Each peptide in a design was evaluated as strong (IC₅₀ < 1 µM), moderate (1 µM < IC₅₀ < 10 µM), or weak (10 µM < IC₅₀ < 100 µM) binder to the eight MHC class II alleles. Strong, moderate and weak binders were then summed to obtain the aggregate score shown in the figure. The line with an "a" indicates the number of strong binders in the wild type lysostaphin catalytic domain, while the line with a "b" shows the number of moderate binders. Numbers on the bars represent the mutational load of each design. *Indicates reverted designs.

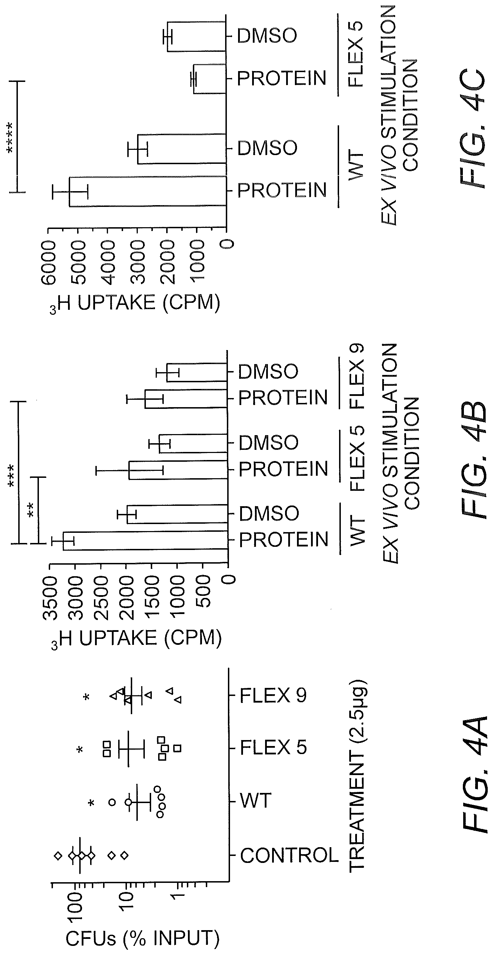

Figures 4A-4C shows *in vivo* efficacy and immunogenicity analysis of Flex 5 and Flex 9 variants. Figure 4A, Bacterial burden in the lungs of C57Bl/6 mice following infection with *S. aureus* and treatment with wild-type LST, variant Flex 5, variant Flex 9, or a PBS control. N=6 per group. Figure 4B, HUMI mice (all humanized from a single donor) were immunized subcutaneously with either wild-type LST (WT), variant Flex 5, or variant Flex 9, and splenocytes were harvested and restimulated *ex vivo* with the same protein or DMSO. Proliferation was measured as uptake of tritiated thymidine. N=4 per group, pooled and measured in triplicate. Figure 4C, Transgenic DR4 mice were immunized with multiple subcutaneous injections of wild-type LST. Following the final boost, mice were allowed to recover for 20 weeks, divided into two groups, and rechallenged with either wild-type LST or variant Flex 5. Splenocytes were harvested and restimulated *ex vivo* with the rechallenge protein or DMSO, and proliferation was measured as uptake of tritiated thymidine. N=5 per group, pooled and measured in triplicate. Statistical significance was assessed by one way ANOVA (Figure 4A) or two way ANOVA (Figures 4B and 4C). *P<0.05; **P<0.01; ***P<0.001; ****P<0.0001.

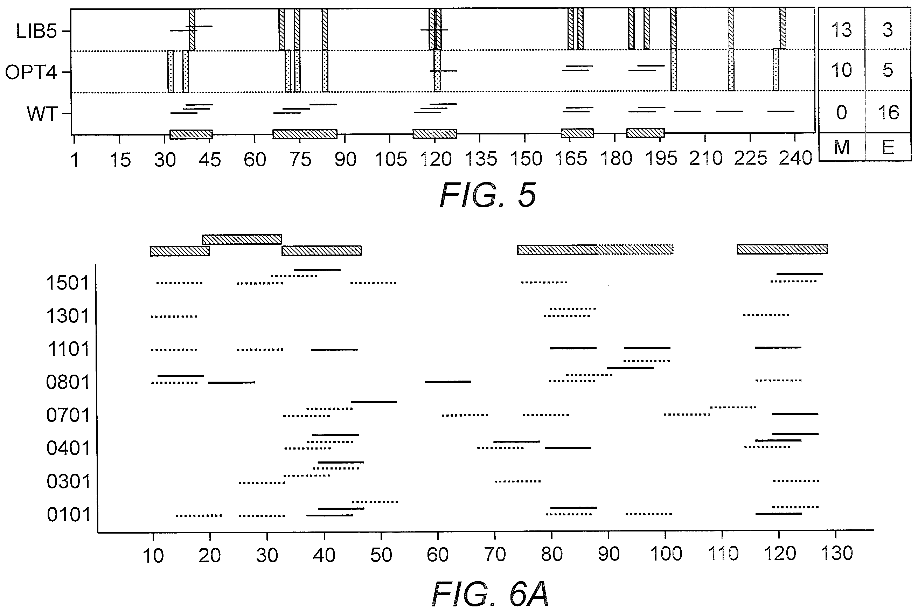

Figure 5 shows the predicted DR4 T cell epitopes for variants Lib5 (top), Opt4 (middle), and LST^{WT} (bottom). Nonamer epitopes are shown as horizontal lines at the corresponding position in the LST amino acid sequence (X-axis). LST^{WT} contains 16 putative epitopes, Opt4 contains 5, and Lib5 contains only three. Regions of overlapping epitope density in LST^{WT} are highlighted with filled blocks on the x-axis. Positions of deimmunizing mutations in Lib5 (top) and in Opt4 (middle) are shown as filled vertical bars. The mutation (M) and epitope (E) counts for each design are indicated at right.

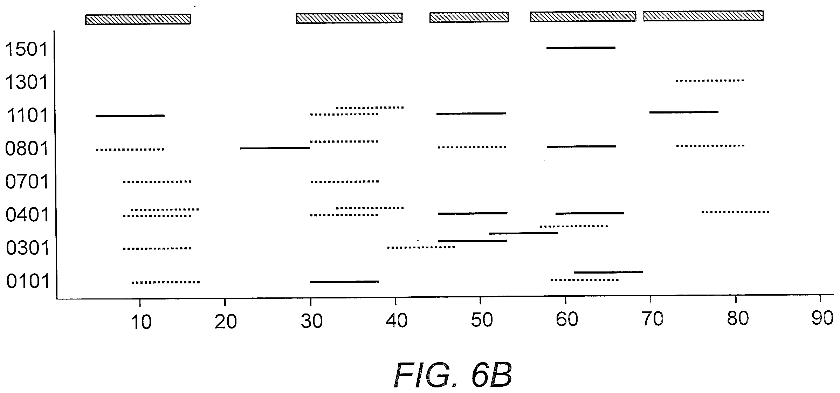

Figures 6A and 6B show the global epitope map of the lysostaphin catalytic and cell wall binding domain, respectively. Epitopes were predicted for HLA alleles DRB1*0101, 0301, 0401, 0701, 0801, 1101, 1301, and 1501. Nine residue peptide epitopes are indicated by solid lines (1-2% threshold) and dashed lines (3-5% threshold). The Lysostaphin catalytic domain primary sequence is indicated on the x-axis, and the HLA alleles associated with each peptide epitope are shown on the y-axis. Higher risk regions for designability analysis are marked at top with filled boxes. The box with the broken line represents a moderate risk region that was also redesigned during development of the deimmunized Flex 9 deimmunized catalytic domain.

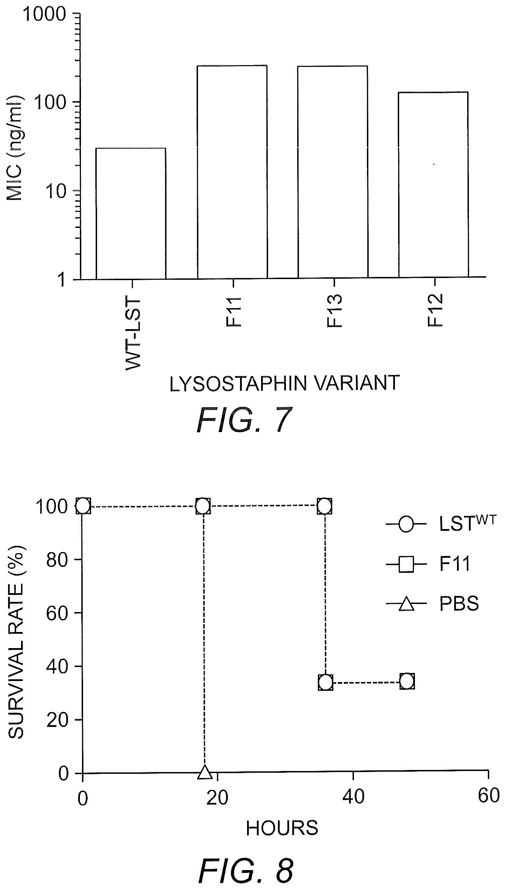

Figure 7 shows the minimum inhibitory concentration (MIC) for F11, F12 and F13 against MRSA strain USA400.
Figure 8 shows the *in vivo* efficacy of the F11 variant in C57BL/6 mice. Mice were challenged with an intraperitoneal injection of 2x10⁸ MRSA strain USA400, and 1 hour later the mice were treated with 100 µg wild-type LST, F11 or PBS. The percent survival is shown.

### Detailed Description of the Invention

It has now been shown that the catalytic domain of lysostaphin can be deimmunized without significantly altering enzymatic activity. In particular, EpiSweep analysis of lysostaphin was used to identify MHC II binding events. Deimmunized lysostaphin variants containing mutations in the catalytic domain were generated, expressed, purified and shown to have an activity level comparable to commercially-sourced lysostaphin. By combining mutations in the catalytic domain with mutations in the cell wall binding domain, this invention provides a fully deimmunized lysostaphin variant and pharmaceutical compositions comprising the same for use to treat a microbial infection.

As used herein, the term "deimmunized" when used in reference to lysostaphin, relates to lysostaphin (*e.g.,* lysostaphin variants, derivatives and/or homologues thereof), wherein the specific removal and/or modification of highly immunogenic regions or residues has occurred. The term "deimmunized" is well-known in the art and, among other things, has been employed for the removal of T-cell epitopes from other therapeutic molecules including antibodies (See, *e.g.,* WO 98/52976 or WO 00/34317).

Humoral antibody formation requires the cooperation of helper T-cells with antigen-specific B-cells. To reduce immunogenicity of a molecule, one approach is to reduce the ability of the antigen to interact with and stimulate B-cells and/or reduce their ability to stimulate helper T-cells. The identification of B-cell epitopes is problematic, however, given the fact that they are of indeterminate length, and often dependent on the tertiary structure of the target antigen. In contrast, T cell epitopes are short (9-15 amino acid), linear peptides (See, *e.g.*, Doytchinova & Flower (2006) Mol. Immunol. 43(13):2037-44). In addition, evidence suggests that reduction of T-cell activation is easier to achieve and has the ability to greatly impact antibody production (see, *e.g.,* Tangri, et al. (2005) J. Immunol. 174:3187-3196). The amino acid sequences that include the antigenic determinants that stimulate T-cells are referred to as T-cell epitopes and are displayed in the context of major histocompatibility complex (MHC) molecules on antigen presenting cells. Altering the ability of T cell epitopes to bind MHC molecules (*e.g*., by inhibiting the binding of the epitope to the MHC molecule, altering the affinity between the epitope and the MHC molecule, altering the epitope in a manner such that the epitope's orientation is altered while within the binding region of the MHC molecule, or altering the epitope in such a way that its presentation by the MHC molecule is altered) has the potential to render the altered epitopes unable to or less able to stimulate an immunogenic response *(e.g.,* stimulate helper T-cells and B cell responses). Accordingly, using the methods described herein, epitopes of lysostaphin were identified and subsequently altered in an effort to reduce the immunogenicity of lysostaphin and its ability to induce humoral antibody responses.

Thus, deimmunization involves the identification, modification and/or removal of T-cell epitopes, preferably helper T-cell epitopes. In this context, the term T-cell epitope relates to T-cell epitopes (*i.e.,* small peptides) that are recognized by T-cells in the context of MHC class I and/or class II molecules. Methods for the identification of T-cell epitopes are known in the art (see, *e.g.*, WO 98/52976, WO 00/34317, and US 2004/0180386). Various methods of identification include, but are not limited to, peptide threading, peptide-MHC binding, human T-cell assays, analysis of cytokine expression patterns, ELISPOT assays, class II tetramer epitope mapping, search of MHC-binding motif databases and the additional removal/modification of T-cell epitopes. In particular embodiments, a structure-guided deimmunization approach, such as that employed by the EpiSweep method, is used. EpiSweep integrates structure-based protein design, sequence-based protein deimmunization, and algorithms for finding the Pareto frontier of a design space (Parker, et al. (2013) J. Comput. Biol. 20:152-65).

Having identified T cell epitopes by application of the above-recited technologies, the epitopes can be eliminated, substituted and/or modified from lysostaphin or fragment(s) thereof (e.g., the catalytic domain) by one or more amino acid substitutions within an identified MHC binding peptide as further described herein. In some embodiments, one or more amino acid substitutions are generated that eliminate or greatly reduce binding to MHC class I and/or class II molecules, or alternatively, altering the MHC binding peptide to a sequence that retains its ability to bind MHC class I or class II molecules but fails to trigger T cell activation and/or proliferation.

Mature lysostaphin has been shown to have two functional domains, a C-terminal domain of 92 residues that binds the *S. aureus* outer cell wall and the N-terminal active site having endopeptidase activity (Baba & Schneewind (1996) EMBO J. 15:4789-4797). Lysostaphin has not been successfully crystallized in part due to the differing solvent characteristics of its two separate domains. However, using the *in silico* methods described herein, highly functional lysostaphin proteins, including various combinations of mutations, have been produced. Mutable amino acids at each position in the catalytic domain (except active site residues) were selected lysostaphin variants were produced that were predicted to have lower immunogenicity while retaining stability. Each mutation was evaluated for expression and activity. Only the mutations that were deemed satisfactory in both regards were selected, and the deimmunization process was repeated again. After the appropriate energy minimizations of the resulting plans, designs with the best predicted energy scores were chosen and experimentally tested. Lysostaphin variants that were capable of being expressed were then purified and further characterized for activity, stability and immunogenicity.

Accordingly, the present invention provides a variety of lysostaphin variants, including modification (e.g., mutations such as amino acid substitutions) of immunogenic epitopes, which retain activity while concurrently displaying reduced immunogenicity. As used herein, the term "lysostaphin" refers to amino acid sequence and/or nucleic acid sequence encoding full length lysostaphin or portion thereof, any lysostaphin mutant or variant (e.g., lysostaphin of any one of SEQ ID NOs: 1-48 or 218-220), any lysostaphin truncation (e.g., in which one or more amino acids have been removed from the protein's amino terminus, carboxy terminus, or both), and any recombinantly expressed lysostaphin protein, that retains the proteolytic ability, *in vitro* and *in vivo,* of proteolytic attack against glycine-containing bridges in the cell wall peptidoglycan of staphylococci. Lysostaphin variants (*e.g.*, deimmunized lysostaphin described herein) may also be expressed in a truncated form. Modified full-length lysostaphin or lysostaphin variants may be generated by post-translational processing of the protein (either by enzymes present in a host cell strain or by means of enzymes or reagents introduced at any stage of the process) or by mutation of the structural gene. Lysostaphin variants, as describe herein, may include deletion, insertion, domain removal, point and replacement/substitution mutations.

A variety of variants are described including, but not limited to, those described in the Examples and depicted in Figures 1A-1E. In some embodiments, a lysostaphin variant has a single amino acid substitution (*e.g.*, any one of the amino acid substitutions described herein) when compared with the wild-type sequence:
AATHEHSAQWLNNYKKGYGYGPYPLGINGGMHYGVDFFMNIGTPVKAISSGKIVEAGWS NYGGGNQIGLIENDGVHRQWYMHLSKYNVKVGDYVKAGQIIGWSGSTGYSTAPHLHFQR MVNSFSNPTAQDPMPFLKSAGYGKAGGTVTPTPNTGWKTNKYGTLYKSESASFTPNTDI ITRTTGPFRSMPQSGVLKAGQTIHYDEVMKQDGHVWVGYTGNSGQRIYLPVRTWNKSTN TLGVLWGTIK (SEQ ID NO:49). In some embodiments, a lysostaphin variant has two amino acid substitutions when compared with the wild-type sequence. In other embodiments, a lysostaphin variant has three amino acid substitutions when compared with the wild-type sequence. In further embodiments, a lysostaphin variant has four or more amino acid substitutions when compared with the wild-type sequence. In certain embodiments, a lysostaphin variant has one or more amino acid substitutions in the catalytic domain. Described is , a lysostaphin mutant having a mutation at Ser124, Ser122, Asn121, Arg118, Ile99, Lys95, Tyr93, Leu83, Lys46, Ile41, Asn13, Asn12 or a combination thereof. In some embodiments, a lysostaphin variant has one or a combination of the following mutations: Ser124Gly, Ser122Asp, Asn121Gly, Arg118Thr, Ile99Gln, Lys95Glu, Tyr93His, Leu83Met, Lys46His, Ile41Glu, Asn13His, and Asn12Gly. In some embodiments, a lysostaphin variant also has one or more amino acid substitutions in the C-terminal binding domain. In some embodiments, a lysostaphin variant also has a C-terminal binding domain mutation at Asn236, Arg186, Ala169, Ser166, Tyr160 or a combination thereof. In some embodiments, a lysostaphin variant also has one or a combination of the following mutations in the C-terminal binding domain mutation: Asn236Asp, Arg186Thr, Ala169Gly, Ser166Asn and Tyr160His. Other suitable amino acid substitutions in the C-terminal binding domain include, but are not limited to those disclosed in US 2008/0095756.

Mutations described herein include, but not limited to, amino acid exchange(s), insertion(s), deletion(s), addition(s), substitution(s), inversion(s) and/or duplication(s). These mutations/modification(s) also include conservative and/or homologue amino acid exchange(s). Guidance concerning how to make phenotypically/functionally silent amino acid substitution has been described (see, *e.g.,* Bowie (1990), Science 247:1306-1310).

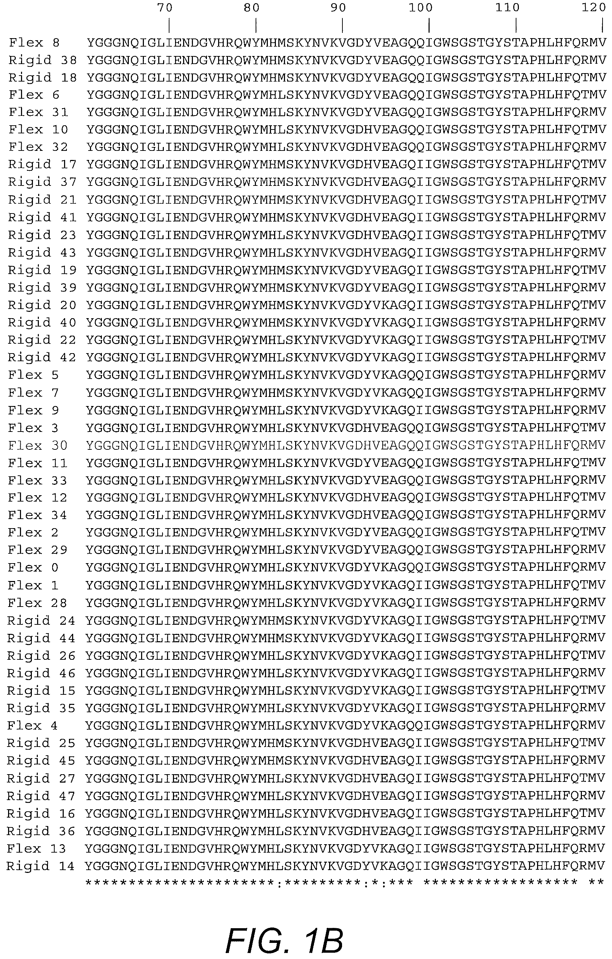

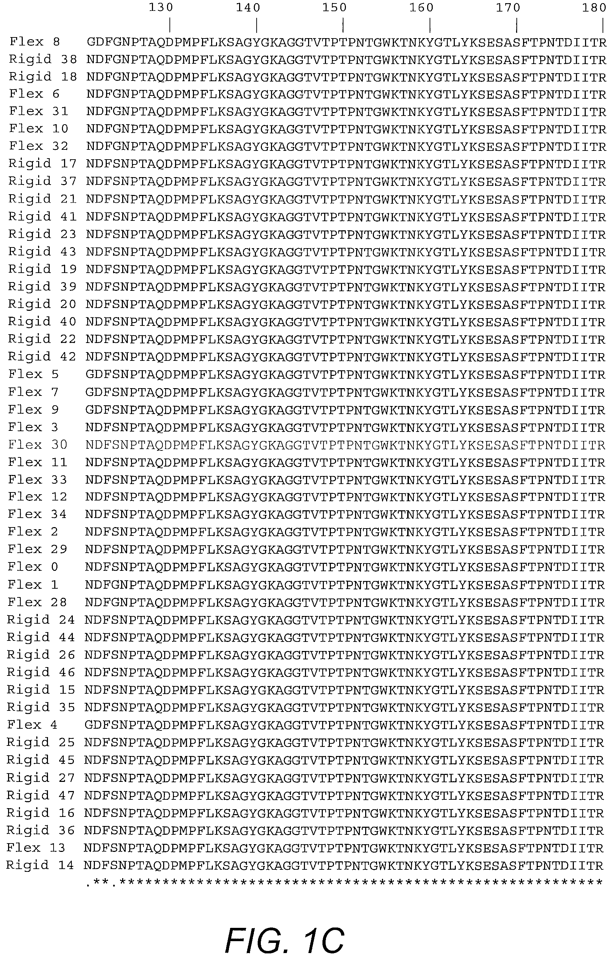

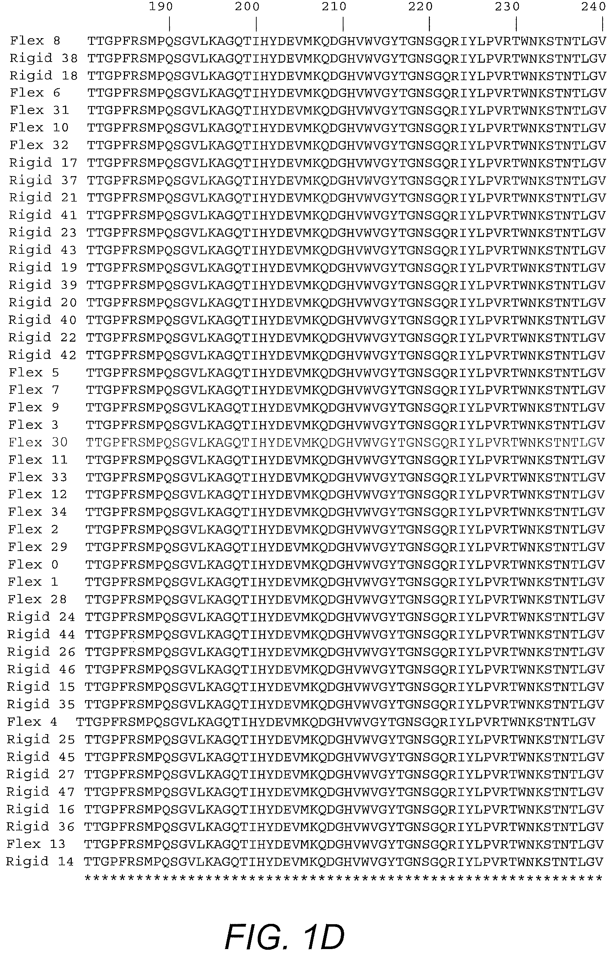

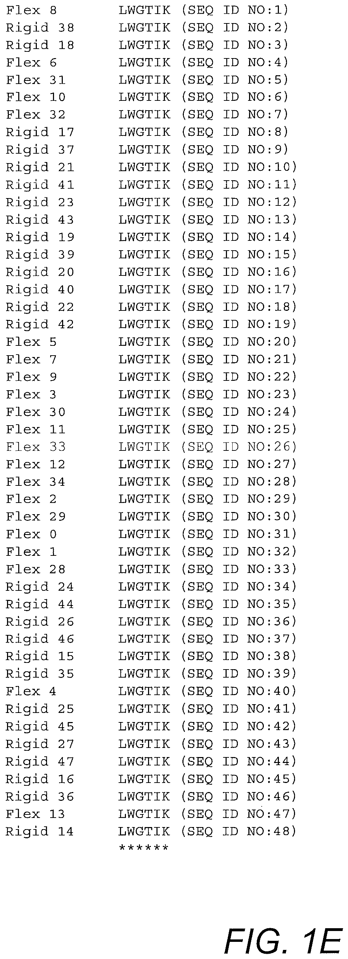

Also described is lysostaphin variants having an amino acid sequence that is at least 60%, more preferably at least 70%, more preferably at least 80%, more preferably 90%, more preferably at least 95% and most preferably 99% identical or homologous to the polypeptide sequences shown in Figure 1 (SEQ ID NOs: 1-48) or in SEQ ID NO:218-220.

Also described is a lysostaphin variant of the present invention that elicits less than 90%, more preferably less than 80%, more preferably less than 70%, more preferably less than 60%, more preferably less than 50%, more preferably less than 40%, more preferably less than 30%, more preferably less than 20%, and even more preferably less than 10% of the immune response (*e.g.,* as measured by anti-lysostaphin antibody titers) elicited by non-deimmunized lysostaphin.

Also described is a plasmid harboring a nucleic acid sequence encoding deimmunized a lysostaphin variant. The plasmid may be an expression vector harboring a nucleic acid sequence encoding a lysostaphin variant (*e.g.*, that displays bactericidal activity and reduced immunogenicity and). In some embodiments, the lysostaphin variant is expressed as a fusion protein, *e.g*., fused to sequences that facilitate purification (*e.g.*, histidine stretches). Also described is an expression vector which harbors a nucleic acid sequence encoding a deimmunized lysostaphin variant having an amino acid sequence as set forth in SEQ ID NO:1-48 (Figures 1A-1E) or SEQ ID NO:218-220.

In addition to lysostaphin variant nucleic acids, a plasmid may also include regulatory sequences, *e.g.,* promoters, transcriptional enhancers and/or sequences that allow for induced expression of lysostaphin variants. For example, one suitable inducible system is a tetracyclineregulated gene expression system (see, *e.g.,* Gossen & Bujard (1992) Proc. Natl. Acad. Sci. USA 89:5547-5551; Gossen et al. (1994) Trends Biotech. 12:58-62). The inducible system may be an isopropyl-b-D-thiogalactoside (IPTG)-inducible promoter.

Using expression plasmids, the lysostaphin variant of this invention can be produced by a number of known methods. For example, the lysostaphin variant can be expressed and isolated from *Bacillus sphaericus* (US 4,931,390); *Lactococcus lactis* NICE expression system (NIsin-Controlled gene Expression) (Mierau, et al. (2005) Microb. Cell Fact. 4:1-9); pET23b(+) and pBAD/Thio-TOPO *E*. *coli* expression systems (Szweda, et al. (2005) J. Biotechnol. 117:203-213); BL21 (DE-*3) E. coli* (Sharma, et al. (2006) Prot. Exp. Purific. 45:206-215); or *Pichia pastoris,* as described herein and elsewhere for the production of therapeutic proteins (Gasser, et al. (2013) Future Microbiol. 8:191-208; Walsh (2010) Nature Biotechnol. 28:917-924; Shekhar (2008) Chem. Biol. 15:201-202; Meyer, et al. (2008) Bioproc. Internat. 6:10-21). In particular embodiments, the lysostaphin variant of this invention is obtained by expression in *P. pastoris,* which is characterized with efficient and selective secretion, high protein titers, and high cell density cultivations (Vogl, et al. (2013) Curr. Opin. Biotechnol. 24:1094-1101). Furthermore, *P. pastoris* is considered as a safe (GRAS) organism, has several signal sequences that can be used for protein secretion, and has one of the strongest promoters known (AOX). Because it allows for protein secretion directly into media, *P. pastoris* greatly simplifies protein recovery and downstream purification.

The lysostaphin variants of this invention can be purified by a number of known methods. For example, due to its high positive charge, lysostaphin has been purified from bacterial hosts such as *S. simulans, B. sphaericus,* or *L. lactis* using a cation exchange step (Recsei, et al. (1990) supra; Mierau, et al. (2005) *supra*; Fedorov, et al. (2003) Biochemistry (Moscow) 68:50-53). When expressed in *E. coli,* lysostaphin has been purified using affinity chromatography (Szweda, et al. (2005) *supra*; Sharma, et al. (2006) *supra*)*.*

Lysostaphin activity can be determined in several different ways: minimum inhibitory concentration (MIC), minimum bactericidal concentration (MBC), disk diffusion, and turbidity reduction (Kusuma & Kokai-Kun (2005) Antimicrob. Agents Chemother. 49:3256-3263). The MIC assay is performed to obtain the minimum concentration of lysostaphin that is necessary to prevent growth of *S. aureus* cells, while MBC assay is usually done after a MIC assay to determine the minimal concentration of the drug necessary to kill *S. aureus.* MIC assays are considered to be the golden standard of determining the value of a therapeutic. The disk diffusion assay is conducted to measure activity by determining the diameter of zone of clearance that is created when a lysostaphin-containing disk is placed on a lawn of *S. aureus* and allowed to diffuse into the plate media over time. The turbidity reduction assay involves measuring the decrease in optical absorbance of *S. aureus* culture over time as lysis of the cells progresses (Schindler & Schuhardt (1964) Proc. Natl. Acad. Sci. USA 51:414-421).

Protein stability can be determined using several different methods. Three well-established methods for measuring thermostability include, *e.g.,* differential scanning calorimetry (DSC), differential scanning light scattering (DSLS), and differential scanning fluorimetry (DSF). All methods are based on determining the rate of protein unfolding with increasing temperature, which is a measure of protein stability. For instance, if a small increase in temperature results in protein unfolding, the protein is not considered to be very stable. DSC directly measures the heat absorption associated with thermal denaturation and has been proven to be sufficiently quantitative for evaluation of stability of protein therapeutics (Wen, et al. (2011) J. Pharmaceut. Sci. 101:955-964). The DSLS method measures protein stability based on the assumption that proteins denature irreversibly as they are exposed to increasing temperatures. Using light-scattering, this method monitors the aggregation that occurs as a consequence of denaturation. In DSF, a fluorescent dye is used that fluoresces upon binding hydrophobic residues. As temperature increases, the protein starts to unfold and exposes the hydrophobic residues found in its core, causing an increase in the fluorescent signal. This increase in signal is monitored over a range of temperatures and is used to determine the Tm value.

To assess immunogenicity, *in vitro* and *in vivo* models have been generated. Since MHC molecules play an important role in T cell dependent immune responses, *in vitro* assays can be used to test the ability of a peptide to bind MHC (Salvat, et al. (2014) J. Vis. Exp. 85). In addition, several animal models such as rats, mice, and non-human primates are currently used in the pre-clinical evaluation of protein therapeutics, wherein the closer a model is to humans, the more accurate it will be at predicting unwanted antibody production in patients (Brinks, et al. (2013) Pharma. Res. 30:1719-1728).

In some embodiments, the present invention provides a pharmaceutical composition containing a lysostaphin variant of the present invention. For example, in some embodiments, the present invention provides a composition containing a lysostaphin variant and a pharmaceutically acceptable carrier. In certain embodiments, the present invention provides a lysostaphin variant (*e.g.,* deimmunized lysostaphin) of use in a pharmaceutical composition for treatment or prevention of staphylococcal infection (*e.g.*, of the skin, of a wound, or of an organ) or as a therapy for various active *S*. *aureus* infections. In preferred embodiments, a pharmaceutical composition of the present invention includes a therapeutically effective amount of a lysostaphin of the invention, together with a pharmaceutically acceptable carrier. The present invention is not limited by the types of pharmaceutically acceptable carrier utilized. Indeed, a variety of carriers are well known in the art including, but not limited to, sterile liquids, such as water, oils, including petroleum oil, animal oil, vegetable oil, peanut oil, soybean oil, mineral oil, sesame oil, and the like. Saline solutions, aqueous dextrose, and glycerol solutions can also be employed as liquid carriers, particularly for solution preparations for injection. Suitable pharmaceutical carriers are described in Remington's Pharmaceutical Sciences, 18th Edition.

A therapeutically effective amount is an amount of lysostaphin variant reasonably believed to provide some measure of relief, assistance, prophylaxis, or preventative effect in the treatment of infection. A therapeutically effective amount may be an amount believed to be sufficient to block a bacterial colonization or infection. Similarly, a therapeutically effective amount may be an amount believed to be sufficient to alleviate (*e.g.*, eradicate) an existing bacterial infection. A pharmaceutical composition of the present invention may be particularly useful in preventing, ameliorating and/or treating bacterial infection.

The compositions of the invention may be administered locally (*e.g*., topically) or systemically (*e.g*., intravenously). Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents depending on the intended use of the pharmaceutical composition.

In accordance with this invention, the terms "treatment", "treating" and the like are used herein to generally mean obtaining a desired pharmacological and/or physiological effect. The effect may be prophylactic in terms of completely or partially preventing an infection and/or may be therapeutic in terms of completely or partially treating (*e.g*., eradicating) a bacterial infection. The term "treatment" as used herein includes preventing bacterial infection from occurring in a subject (*e.g*., that may be predisposed to infection (*e.g.*, nosocomial infection) but has not yet been diagnosed as having infection); inhibiting bacterial infection; and/or (c) relieving infection (*e.g*., completely or partially reducing the presence of bacteria responsible for infection.

Staphylococcal infections, such as those caused by *S. aureus,* are a significant cause of morbidity and mortality, particularly in settings such as hospitals, schools, and infirmaries. Patients particularly at risk include infants, the elderly, the immunocompromised, the immunosuppressed, and those with chronic conditions requiring frequent hospital stays. Patients also at risk of acquiring staphylococcal infections include those undergoing inpatient or outpatient surgery, those within an Intensive Case Unit (ICU), on continuous hemodialysis, with HIV infection, with AIDS, burn victims, people with diminished immunity (*e.g*., resulting from drug treatment or disease), the chronically ill or debilitated patients, geriatric subjects, infants with immature immune systems, and people with intravascular (*e.g.*, implanted) devices. Thus, in some embodiments, a composition containing a lysostaphin variant is administered to any one of these types of subject as well as to other subjects that have or are susceptible to bacterial infection (*e.g*., caused by *S. aureus* or *S. epidermidis*)*.*

In some embodiments, a lysostaphin variant of the present invention is formulated as either an aqueous solution, semi-solid formulation, or dry preparation (*e.g.*, lyophilized, crystalline or amorphous, with or without additional solutes for osmotic balance) for reconstitution. Formulations may be in, or reconstituted in, for example, a non-toxic, stable, pharmaceutically acceptable, aqueous carrier medium, at a pH of about 3 to 8, typically 5 to 8, for administration by conventional protocols and regimes or in a semi-solid formulation such as a cream. Delivery can be via, for example, ophthalmic administration, intravenous (iv), intramuscular, subcutaneous or intraperitoneal routes or intrathecally or by inhalation or used to coat medical devices, catheters and implantable devices, or by direct installation into an infected site so as to permit blood and tissue levels in excess of the minimum inhibitory concentration (MIC) of the active agent to be attained (*e.g.*, to effect a reduction in microbial titers in order to cure, alleviate or prevent an infection). In some embodiments, the antimicrobial agent is formulated as a semi-solid formulation, such as a cream (*e.g.*, that is used in a topical or intranasal formulation).

Furthermore, the lysostaphin variant can be co-administered, simultaneously or alternating, with other antimicrobial agents so as to more effectively treat an infectious disease. Formulations may be in, or be reconstituted in, semi-solid formulations for topical, ophthalmic, or intranasal application, liquids suitable for ophthalmic administration, bolus iv or peripheral injection or by addition to a larger volume iv drip solution, or may be in, or reconstituted in, a larger volume to be administered by slow iv infusion. For example, a lysostaphin variant can be administered in conjunction with antibiotics that interfere with or inhibit cell wall synthesis, such as penicillins, nafcillin, and other alpha- or beta-lactam antibiotics, cephalosporins such as cephalothin, aminoglycosides, sulfonamides, antifolates, macrolides, quinolones, glycopepetides such as vancomycin and polypeptides. In some embodiments, a lysostaphin variant is administered in conjunction with one or more antibiotics that inhibit protein synthesis (*e.g*., aminoglycosides such as streptomycin, tetracyclines, and streptogramins). The present invention is not limited by the type of agent co-administered with deimmunized lysostaphin. Indeed, a variety of agents may be co-administered including, but not limited to, those agents described in US 6,028,051, US 6,569,830, and US 7,078,377. In some embodiments, a lysostaphin variant is administered with monoclonal antibodies; other non-conjugated antibacterial enzymes such as lysostaphin, lysozyme, mutanolysin, and cellozyl muramidase; peptides (*e.g*., defensins); and lantibiotics (*e.g.,* nisin); or any other lanthione-containing molecules (*e.g.*, subtilin).

Agents co-administered with a lysostaphin variant may be formulated together with the lysostaphin variant as a fixed combination or may be used extemporaneously in whatever formulations are available and practical and by whatever routes of administration are known to provide adequate levels of these agents at the sites of infection.

In preferred embodiments, lysostaphin variants according to the present invention possess at least a portion of the antimicrobial activity of the corresponding non-deimmunized antimicrobial agent. A lysostaphin variant of the present invention may be administered in increased dosages and/or at less frequent intervals due to the decreased immunogenicity. In some embodiments, a lysostaphin variant retains at least 10% of the activity of the non-deimmunized antimicrobial agent. In some embodiments, a lysostaphin variant retains at least 20% of the activity of the non-deimmunized antimicrobial agent. In some embodiments, a lysostaphin variant retains at least 30% of the activity of the non-deimmunized antimicrobial agent. In some embodiments, a lysostaphin variant retains at least 40% of the activity of the non-deimmunized antimicrobial agent. In some embodiments, a lysostaphin variant retains at least 50% of the activity of the non-deimmunized antimicrobial agent. In some embodiments, a lysostaphin variant retains at least 60% of the activity of the non-deimmunized antimicrobial agent. In some embodiments, a lysostaphin variant retains at least 70% of the activity of the non-deimmunized antimicrobial agent. In some embodiments, a lysostaphin variant retains at least 80% of the activity of the non-deimmunized antimicrobial agent. In some embodiments, a lysostaphin variant retains at least 90% of the activity of the non-deimmunized antimicrobial agent. In some embodiments, a lysostaphin variant retains 90% or more (e.g., 95%, 97%, 99% or more) of the activity of the non-deimmunized antimicrobial agent.

Suitable dosages and regimes of a deimmunized lysostaphin may vary with the severity of the infection and the sensitivity of the infecting organism and, in the case of combination therapy, may depend on the particular agent (*e.g.*, anti-staphylococcal agent) co-administered. Dosages may range from about 0.05 to about 500 mg/kg/day (*e.g.*, in some embodiments, range from 0.1-10 mg/kg/day, in some embodiments, range from 10-100 mg/kg/day, in some embodiments, range from 100-200 mg/kg/day, in some embodiments, range from 200-400 mg/kg/day, in some embodiments, range from 400-500 mg/kg/day), although higher (*e.g.,* 500-1000 mg/kg/day) or lower (*e.g.,* 0.1-0.5 mg/kg/day doses may be provided, given as single or divided doses, or given by continuous infusion. In some embodiments, deimmunized lysostaphin is administered once a day, twice a day, three times a day or more frequently (*e.g.,* four or more times a day). In some embodiments, deimmunized lysostaphin is administered once a week, twice a week, or every other day. In some embodiments, deimmunized lysostaphin is administered once every other week, once a month, once every two months, once every three months, once every four months, once every five months, once every six months, once every 9 months, once every year or less frequently.

In certain embodiments, a deimmunized lysostaphin of this invention is aglycosylated. Aglycosylation can be carried out as described here, and can include mutations at residues Ser126 and/or Thr127. Exemplary mutations include, Ser126Pro and Thr127Ala.

In some embodiments, a deimmunized lysostaphin of the present invention may be further modified in order to further decrease immunogenicity of the lysostaphin molecule while retaining antimicrobial activity. For example, in some embodiments, a deimmunized lysostaphin is conjugated to a water soluble polymer. The present invention is not limited by the type of water soluble polymer to which a deimmunized lysostaphin is conjugated. Indeed, a variety of water soluble polymers may be used including, but not limited to, poly(alkylene oxides), polyoxyethylated polyols and poly(vinyl alcohols). Poly(alkylene oxides) include, but are not limited to, polyethylene glycols (PEGs), poloxamers and poloxamines. The present invention is not limited by the type of conjugation used (*e.g*., to connect a deimmunized lysostaphin to one or more water-soluble polymers (*e.g*., PEG)). In some embodiments, a poly(alkylene oxide) is conjugated to a free amino group via an amide linkage (e.g., formed from an active ester such as the N-hydroxysuccinimide ester) of the poly(alkylene oxide). In some embodiments, an ester linkage remains in the conjugate after conjugation. In some embodiments, linkage occurs through a lysine residue present in the deimmunized lysostaphin molecule. In some embodiments, conjugation occurs through a short-acting, degradable linkage. The present invention is not limited by the type of degradable linkage utilized. Indeed, a variety of linkages are contemplated to be useful in the present invention including, but not limited to, physiologically cleavable linkages including ester, carbonate ester, carbamate, sulfate, phosphate, acyloxyalkyl ether, acetal, and ketal linkages. In some embodiments, deimmunized lysostaphin is conjugated to PEG utilizing any of the methods, reagents and/or linkages described in US 4,424,311; US 5,672,662; US 6,515,100; US 6,664,331; US 6,737,505; US 6,894,025; US 6,864,350; US 6,864,327; US 6,610,281; US 6,541,543; US 6,515,100; US 6,448,369; US 6,437,025; US 6,432,397; US 6,362,276; US 6,362,254; US 6,348,558; US 6,214,966; US 5,990,237; US 5,932,462; US 5,900,461; US 5,739,208; US 5,446,090 and US 6,828,401; and WO 02/02630 and WO 03/031581. In some embodiments, a deimmunized lysostaphinwater soluble polymer conjugate of the present invention is produced by a third party (*e.g.*, NEKTAR, San Carlos, CA). In some embodiments, the conjugate includes a cleavable linkage present in the linkage between the polymer and deimmunized lysostaphin (*e.g.*, such that when cleaved, no portion of the polymer or linkage remains on the deimmunized lysostaphin molecule). In some embodiments, the conjugate includes a cleavable linkage present in the polymer itself (*e.g.*, such that when cleaved, a small portion of the polymer or linkage remains on the deimmunized lysostaphin molecule).

Also described is, a deimmunized lysostaphin of the present invention that is used for the treatment and/or prevention of a biofilm (*e.g.*, as described in US 2003/0215433 and WO 03/082148). In other embodiments, a deimmunized lysostaphin of the present invention is for use in the prevention and/or treatment of a microbial infection, including bacterial infections by members of the genus *Staphylococcus.* A subject in need of treatment (*e.g.,* a subject with or at risk of developing an *S. aureus* infection) is administered an effective amount of a deimmunized lysostaphin so that the microbial infection is prevented or treated. Subjects benefiting from this treatment include those exhibiting clinical signs or symptoms of an infection, a subject exposed to a bacterium (*e.g., S. aureus*)*,* or a subject suspected of being exposed to a bacterium (*e.g., S. aureus*)*.* Effective treatment will result in a decrease, attenuation, inhibition or amelioration of the well-known signs or symptoms of infection. In some embodiments, treatment includes nasal applications, *e.g*., as described in US 2003/0211995; or topical applications, *e.g.*, as described in US 2004/0192581.

The selected dosage level will depend upon a variety of factors including the activity of the particular deimmunized lysostaphin employed, the route of administration, the time of administration, the rate of excretion or metabolism of the particular deimmunized lysostaphin, the duration of the treatment, other drugs, compounds and/or materials used in combination with the particular deimmunized lysostaphin employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of a deimmunized lysostaphin at levels lower than that required in order to achieve the desired therapeutic effect and gradually increase the dosage until the desired effect is achieved. This is considered to be within the skill of the artisan.

Effective doses can also be determined in an artrecognized model of *S. aureus* infection. There are many different *in vivo* model systems that can be used by one of skill in the art to further demonstrate efficacy and aid in identification of doses that will be both safe and effective in humans. Such animal model systems are well-accepted and used during development of new human pharmaceuticals. Examples of such model systems include, but are not limited to, a guinea pig model of *S. aureus* wound infection (Kernodle & Kaiser (1994) Antimicrob. Agents Chemother. 38:1325-1330); a rabbit model of *S. aureus* abscess in rabbits (Fernandez, et al. (1999) Antimicrob. Agent Chemother. 43:667-671); a mouse model of *S. aureus* skin infection (Gisby & Bryant (2000) Antimicrob. Agents Chemother. 44:255-260); a mouse model of deep dermal *S. aureus* infection (Godin, et al. (2005) J. Antimicrob. Chemother. 55:989-994); and a mouse intraperitoneal infection model (Patel, et al. (2004) Antimicrob. Agents Chemother. 48:4754-4761). In such models, therapeutics can be tested against infections where the infection established is from inoculation of the animal with various strains of *S. aureus.* Demonstration of efficacy in such models is measured in many ways and would include but not be limited to a reduction in mortality rate, a reduction in bacterial cell counts determined by microscopic examination of tissue or blood samples taken from the animals, or even assessment of wound healing in the animals.

The following non-limiting examples are provided to further illustrate the present invention.

### Example 1: Deimmunization of Lysostaphin Catalytic Domain

### Materials and Methods

*Reagents and Media.* Primers were ordered with standard desalting from IDT Technologies (Coralville, IA). PCR cleanup and gel extraction kits were from Zymo Research (Irvine, CA). Commercial lysostaphin was purchased from Sigma (St. Louis, MO). Plasmid purification was performed using QIAPREP Spin Miniprep Kit (Qiagen; Valencia, CA). All enzymes were obtained from New England BioLabs (Ipswich, MA), and all reagents from VWR Scientific (Philadelphia, PA), unless otherwise noted. Peptides derived from the lysostaphin catalytic domain were ordered from GenScript (Piscataway, NJ), and were greater than 85% pure. MHC-II DR molecules were purchased from Benaroya Research Institute (Seattle, WA), anti-MHC-IIDR antibody from Biolegend (San Diego, CA), and DELFIA Eu-labeled Streptavidin was from PerkinElmer (Boston, MA) .

*Epitope Prediction.* The T cell epitope content of the lysostaphin catalytic domain was predicted using EpiMatrix, a scoring matrix whose predictions have been shown to correlate well with the clinically observed immunogenicity of therapeutics. EpiMatrix is a pocket profile method used to predict HLA binding (Groot & Moise (2007) Curr. Opin. Drug Discover. Dev. 10). In this approach, a protein is divided into overlapping 9-mer peptides, each of which is then evaluated for its binding potential to HLA alleles. The eight most common HLA alleles (DRB1*0101, DRB1*0301, DRB1*0401, DRB1*0701, DRB1*0801, DRB1*1101, DRB1*1301, DRB1*1501), which are representative of more than 90% of the human population (Southwood, et al. (1998) J. Immunol. 160:3363-3373), were considered. Based on binding potential, each peptide was assigned a corresponding standardized Z score and then mapped onto the cluster immunogenicity scale, which represents the deviation in epitope content from what would be expected for a randomly generated peptide (Groot, et al. (2013) Exp. Rev. Clin. Pharmacol. 6:651-662). Peptides scoring above 1.64 on the EpiMatrix "Z" scale (approximately the top 5%) were considered to be likely to bind to the corresponding MHC molecule, while peptides scoring in the top 1% (above 2.32 on the scale) were extremely likely to bind (Koren, et al. (2007) Clin. Immunol. 124:26-32). If a peptide had a score higher than 1.64 for four or more alleles, it was said to contain an EpiBar (Weber, et al. (2009) Adv. Drug Deliv. Rev. 61:965-976).

*Homology Modeling of Lysostaphin.* EpiSweep analyzes deimmunized variants based on two quantified measures: epitope score and force field energy value. However, the algorithm uses the structure of the protein to calculate energy. Since the crystal structure of lysostaphin was not known, a lysostaphin model was constructed based on the available crystal structures of proteins similar to the catalytic domain of lysostaphin. The templates were selected by comparing the sequence of lysostaphin to the PDB database and selecting three highly similar protein structures (PDB accession code: 2B0PA, 2B44A, and 1QWYA). For homology modeling purposes, sequence identity above 30% is considered sufficiently accurate (Rost (1999) Prot. Eng. Design Select. 12:85-94). All of the template structures belong to LytM, an autolysin from *S. aureus,* which has 48% sequence identity and 63% similarity to the catalytic domain of lysostaphin (Lu, et al. (2013) *supra*).

The catalytic domain models were built using LytM crystal structures by employing MODELLER, a homology modeling protocol that builds a three-dimensional structure of proteins based on coordinates of template structures (Shen & Sali (2006) Protein Sci. 15:2507-2524). Two hundred-fifty models were generated and the most accurate one was selected in terms of the DOPE statistical potential (Shen & Sali (2006) *supra*) *.*

For regions of the protein for which there was no sufficient coordinate information to allow complete and accurate construction of the model, a protein loop modeling method, FREAD, was used to remodel the existing gaps (Choi & Deane (2010) Proteins 78:1431-1440). The obtained homology model was minimized against AMBER99sb with an implicit solvent model (GB/SA). The aglycosylated wild-type (125 NPT) was modeled by applying *in silico* mutations to the original model using Scwrl4 (Krivov, et al. (2009) Proteins: Struct. Funct. Bioinform. 77:778-795), followed by energy reminimization.

*Evolutionary Information.* The process of deimmunization requires mutation of residues that are predicted to contribute to MHC binding. However, T cell epitopes can be present in any part of the protein. Thus, random selection of mutations could lead to disruption of proper folding and function. This problem can be mitigated by adopting point mutations found in sequences remotely similar to the target sequence. To determine which mutations could be used in the deimmunization process, a total of 10,000 homologs to LST^{CAT} were collected by running PSI-BLAST (3 iterations, e-value <0.001). These sequences were filtered to remove those with >50% gaps or <35% sequence identity to the wild-type. A diverse set of 218 representative sequences was subselected so as to have at most 90% sequence identity to each other. Allowed mutations were those predicted to delete at least one putative epitope while appearing as frequently as expected in terms of a background probability distribution (McCaldon & Argos (1988) Proteins 4:99-122). Additional filters excluded mutations to/from Pro and Cys, mutations involving active site residues (32His, 36Asp, 82His, 113His and 115His), and mutations previously found to be detrimental (Thr43Asp, Ser50Asp, Asn121Asp, and Leu135Ser).

*EpiSweep.* Structure-based EpiSweep is a protein redesign tool that allows for protein deimmunization while retaining protein stability and functionality. The algorithm combines validated immunoinformatics and structural modeling to produce Pareto optimal designs that can then be selected experimentally for the best immunogenicity, stability, and activity scores. The method by which EpiSweep selects the optimal designs has been described (Parker, et al. (2013) J. Computation. Biol. 20:152-165). Briefly, the algorithm addresses the stability concern by assuming the protein backbone as rigid and selecting the best side-chain conformations from a discrete set of rotamers, which are chosen to minimize total protein energy. All rotamers and rotamer pairs are evaluated for potential clashes with the backbone and with each other. For example, conformations found to contain rotamers with a significant van der Waals radii overlap or with exceptionally high intra- or interrotamer energies are discarded (Parker, et al. (2013) *supra).*

For EpiSweep analysis of the lysostaphin catalytic domain, the mutational load was allowed to vary from two to eight mutations and the algorithm was constrained to disallow mutations at the active site (His32, Asp36, His82, His113, and His115). Furthermore, the algorithm was to generate not only Pareto optimal plans at each mutational load (designs with the lowest possible rotamer energies), but also the additional 19 suboptimal plans (designs that have successively worse rotamer energies as compared to the Pareto optimal plans). This analysis was performed to correct for any possible mistakes that may arise due to the rigid backbone assumption, since proteins are characterized by a high degree of flexibility. In fact, it has been observed that further side chain optimization changes Pareto optimality of EpiSweep designs, and thus may affect design selection (Parker, et al. (2013) *supra).*

*EpiSweep Analysis of Ser126Pro Backbone.* For analysis using the Ser126Pro lysostaphin backbone, the algorithm considered only the 12 well-tolerated mutations (Ser124Gly, Ser122Asp, Asn121Gly, Arg118Thr, Ile99Gln, Lys95Glu, Tyr93His, Leu83Met, Lys46His, Ile41Glu, Asn13His, and Asn12Gly). Since Ser122Asp mutation was an extremely efficient epitope remover (Table 6), the algorithm was also constrained to include this mutation in all generated plans. Additional post-processing energy minimization of the EpiSweep designs was performed using molecular modeling software TINKER against AMBER (AMBER99sb) force field and an implicit solvent model (GB/SA).

*Plasmids and Strains. P. pastoris* strain GS115 and expression vector pPIC9 were obtained from Invitrogen (Grand Island, NY). *S. aureus* strain SA113 and *S. aureus subsp. aureus* (ATCC 25923) were obtained from the American Type Culture Collection (Manassas, VA). Other strains of *S. aureus* (methicillin sensitive strains 6445 and 3425-1, and MRSA strain 3425-3) were clinical isolates.

*Synthesis of Lysostaphin Gene Optimized for P. pastoris Expression.* Synthesis of a synthetic lysostaphin gene was performed as described (Zhao, et al. (2014) Appl. Environ. Microbiol. 80:2746-53). The majority of the codons was replaced to reflect the codon preference by *P. pastoris* (Zhao, et al. (2000) Sheng Wu Gong Cheng Xue Bao 16:308-11). To disrupt long A+T nucleotide stretches in the gene sequence, second-most frequent codons were introduced as needed.

*PCR-Based Synthesis of Single Point Mutants.* Lysostaphin single point mutants were synthesized as described (Zhao, et al. (2014) *supra).* Briefly, the mutations were introduced using splice overlap extension PCR with primers listed in Table 1. For instance, the Ser122Gly mutation was introduced by first amplifying lysostaphin gene using Syn_F and S122G_R, and S122G_F and Syn_R primers. The resulting (gel-purified) gene fragments were then mixed at an equimolar ratio, and used as a template in a subsequent reaction using Syn_F and Syn_R primers. The final product was the full-length lysostaphin gene with the Ser122Gly mutation. All PCR reactions were performed using PHUSION High-Fidelity DNA polymerase. The lysostaphin gene harboring the desired mutation was then digested with *Eco*RI and *Xho*I, and ligated into the pPIC9 plasmid using T4 DNA ligase. The end product of ligation was the lysostaphin gene fused to the alpha mating factor secretion signal from *Saccharomyces cerevisiae.* The resulting plasmid was transformed into *E. coli* DH5α electrocompetent cells (*F*-ϕ80*lac*ZΔM15Δ(*lacZYA-argF*) U169 *recA1 endA1 hsdR17* (*r⁻_{K}m⁺_{K}*) *phoA supE44 λ⁻thil gyrA96 relA1*) *.* Clones were evaluated for the presence of lysostaphin gene using Syn_F and Syn_R primers and sequenced to confirm the presence of mutations (primers AOX1_F and AOX1_R) .

**TABLE 1**

| Primer | Sequence (5'->3') | SEQ ID NO: |
|---|---|---|
| N12G_R | ACCCTTCTTGTAGTTACCCAACCATTGAGCGGA | 50 |
| N12G_F | TCCGCTCAATGGTTGGGTAACTACAAGAAGGGT | 51 |
| N13H_R | CGATGAATTCTTACTTGATGGTACCCCA | 52 |
| N13H_F | ATCGCTCGAGAAAAGAGCTGCTACCCA CGAGCACTCCGCTCAATGGTTGAACCACTAC | 53 |
| F38GR | GGTACCGATGTTCATACCGAAGTCAACACCGTA | 54 |
| F38G_F | TACGGTGTTGACTTCGGTATGAACATCGGTACC | 55 |
| I41E_R | AGCCTTGACTGGGGTACCCTCGTTCATGAAGAA | 56 |
| I41E_F | TTCTTCATGAACGAGGGTACCCCAGTCAAGGCT | 57 |
| K46H_R | ACCGGAGGAGATAGCGTGGACTGGGGTACCGAT | 58 |
| K46H_F | ATCGGTACCCCAGTCCACGCTATCTCCTCCGGT | 59 |
| L83M_R | TACTTGGACATGTGCATGTACCATTG | 60 |
| L83M_F | CAATGGTACATGCACATGTCCAAGTA | 61 |
| Y93H_R | TTGACCAGCCTTGACGTGGTCACCGACCTTGAC | 62 |
| K95E_R | GATGATTTGACCAGCCTCGACGTAGTCACCGAC | 63 |
| K95E_F | GTCGGTGACTACGTCGAGGCTGGTCAAATCATC | 64 |
| Y93H_F | GTCAAGGTCGGTGACCACGTCAAGGCTGGTCAA | 65 |
| I99Q_R | ACCGGACCAACCGATTTGTTGACCAGCCTTGAC | 66 |
| I99Q_F | GTCAAGGCTGGTCAACAAATCGGTTGGTCCGGT | 67 |
| R118T_R | GAAGGAGTTGACCATGGTTTGGAAGTGCAAGTG | 68 |
| R118TF | CACTTGCACTTCCAAACCATGGTCAACTCCTTC | 69 |
| N121G_R | TGGGTTGGAGAAGGAACCGACCATTCTTTGGAA | 70 |
| N121G_F | TTCCAAAGAATGGTCGGTTCCTTCTCCAACCCA | 71 |
| S122D_R | GGTTGGGTTGGAGAAGTCGTTGACCATTCTTTG | 72 |
| S122D_F | CAAAGAATGGTCAACGACTTCTCCAACCCAACC | 73 |
| S122G_R | GGTTGGGTTGGAGAAACCGTTGACCATTCTTTG | 74 |
| S122G_F | CAAAGAATGGTCAACGGTTTCTCCAACCCAACC | 75 |
| S124G_R | TTGAGCGGTTGGGTTACCGAAGGAGTTGACCAT | 76 |
| S124G_F | ATGGTCAACTCCTTCGGTAACCCAACCGCTCAA | 77 |
| S124Y_R | TTGAGCGGTTGGGTTGTAGAAGGAGTTGACCAT | 78 |
| S124YF | ATGGTCAACTCCTTCTACAACCCAACCGCTCAA | 79 |
| Syn_R | CGATGAATTCTTACTTGATGGTACCCCA | 80 |
| Syn_F | ATCGCTCGAGAAAAGAGCTGCTACCCAC | 81 |
| AOX1_R | GCAAATGGCATTCTGACATCC | 82 |
| AOX1_F | GACTGGTTCCAATTGACAAGC | 83 |
| AflII_R | AACGTAACCAGCGGACTTAAGGAATGGCATTGGGTC | 84 |
| AflII_F | GACCCAATGCCATTCCTTAAGTCCGCTGGTTACGGT | 85 |
| T118R_R | GAAGTCGTTGACCATTCTTTGGAAGTGCAAGTG | 86 |
| T118R_F | CACTTGCACTTCCAAAGAATGGTCAACGACTTC | 87 |

*Cloning of LST Integrated Designs.* Lysostaphin variants that were not expressing in the first round of screening and contained the Arg118Thr mutation were synthesized without that mutation and re-examined for expression. Splice overlap extension PCR was used to revert the Arg118Thr mutation back to wild-type (Thr118) with primers T118R_F and T118R_R (Table 1).

To prepare the pPIC9 plasmid for insertion of synthesized genes, a silent mutation was introduced into the lysostaphin linker (residues 135-136) to accommodate a cutting site for the restriction enzyme *Afl*II. To introduce the necessary mutations, splice overlap extension PCR was used with primers AflII_F and AflII_R (Table 1). The synthetic genes were digested with *Xho*I and *Afl*II restriction enzymes, and ligated in similarly digested pPIC9 plasmid using T4 DNA ligase. The resulting plasmid was transformed into *E. coli* DH5α electrocompetent cells and the resulting clones were sequenced to confirm the presence of mutations.

*P. pastoris Expression and Purification.* After DH5α clones were confirmed for the presence of correct catalytic domain mutation by sequence analysis, the purified plasmid was digested with *Sac*I High-Fidelity restriction enzyme prior to electroporation into *P. pastoris* strain GS115. The resulting transformants were grown on MD plates (1.34% yeast nitrogen base, 0.000004% biotin, 2% dextrose and 1% agar). For expression studies, clones were grown in BMGY media (1% yeast extract, 2% peptone, 1.34% yeast nitrogen base, 0.000004% biotin, 1% glycerol, 100 mM phosphate buffer, pH 6) at 30°C in 500 ml shake flasks covered with four layers of cheese cloth for enhanced oxygen flow to the yeast. After 24 hours, cells were centrifuged at 3,000 rpm in a table top centrifuge for 10 minutes. The cells were then resuspended in 100 ml of BMMY induction media (1% yeast extract, 2% peptone, 1.34% yeast nitrogen base, 0.000004% biotin, 0.5% methanol, 100 mM phosphate buffer, pH 6) and allowed to grow for the next 48 hours at 30°C. At 12-hour intervals, 100% methanol was added for a final concentration of 1%. After 48 hours of induction, shake flask culture was centrifuged in a table top centrifuge at 3,000 rpm for 15 minutes. The resulting supernatant was filtered to remove any yeast cells and diluted 1:5 with 10 mM KH₂PO₄ buffer at pH 7.5. Diluted supernatant was flowed over a gravity column packed with 500 µl SP-SEPHAROSE Fast Flow resin (GE Healthcare; Cleveland, OH). The column was washed with 5 ml of 50 mM NaCl in 10 mM KH₂PO₄ at pH 7.5. Protein was eluted with 500 µl aliquots of 200 mM NaCl in 10 mM KH₂PO₄, pH 7.5. The purity of lysostaphin was determined using SDS-PAGE. The protein concentration was quantified using ND-1000 Spectrophotometer (NanoDrop Technologies; Wilmington, DE). To ensure accuracy, protein absorbance measure was adjusted using the absorbance adjustment factor of 0.4 for both wild-type lysostaphin and its variants. Briefly, the adjustment factor was calculated as the inverse of the reported Abs 0.1% (=1 g/L) value (ProtParam, ExPASy).

*Lytic Assay of Culture Supernatant. S. aureus* cells were grown either to mid-log or to saturation in tryptic soy broth (TSB) at 37°C with shaking. The cells were harvested by centrifugation and washed once in phosphate buffered saline (PBS: 2.7 mM KCl, 1.5 mM KH₂PO₄, 8.9 mM Na₂HPO₄, 136.9 mM NaCl, pH 7.4). The assay was performed in 96-well, black, clear bottom plates from Greiner Bio-One (Monroe, NC). The final (250 µl) reaction was composed of 10 µl of *P. pastoris* culture supernatant, *S. aureus* cells at OD600 = 1.5, and 5 µM SYTOX Green (Thermo Fisher Scientific; Waltham, MA), all in PBS. Data were collected using SPECTRAMAX GEMINI Fluorescence Microplate Reader (Molecular Devices; Sunnyvale, CA) using an excitation/emission of 504/523 nm, and rates were determined from the slope of the steepest linear portion of the trace. Each assay was performed with 500 ng of commercially-sourced lysostaphin (ssLys) as an internal control.

The amount of protein used in each assay was estimated from SDS-PAGE gels. Briefly, 10 µl of culture supernatant was run on a SDS-PAGE gel together with standards of 0.5 µg, 0.7 µg, and 1 µg of ssLys in separate lanes. The gel was stained using GELCODE Blue Stain Reagent from Thermo Fisher Scientific (Carlsbad, CA) and bands quantified using Quantity Tools from Image Lab 5.1 software (Bio-Rad Laboratories; Hercules, CA).

*Lytic Assay Using Purified Protein.* The activity of purified lysostaphin variants was examined using the SYTOX^{®} kinetic assay as previously described in Chapter 2, Section 3.2.6. 200 ng of purified enzyme (instead of culture supernatant) was used in each reaction.

*MIC Assay.* The MICs of wild-type lysostaphin and its variants were determined by adding 2-fold serial dilutions of enzymes into wells of a polypropylene 96-well plate (Costar 3879) containing ~40,000 *S. aureus* SA113 (or *S. aureus* 6445, 3425-1, 3425-3) cells in Muller Hinton broth (BD) supplemented with 2% NaCl and 0.1% bovine serum albumin, yielding a total volume of 100 µl. Plates were grown overnight at 37°C with shaking at 900 rpm on an Orbit P4 orbital shaker (Labnet; Edison, NJ). The inhibitory activity of purified lysostaphin was determined by the concentration of enzyme that completely inhibited bacterial growth. The assay was performed in triplicate for each enzyme.

*PNGase F Treatment. P. pastoris* culture supernatant was treated with 1 µl of 10x G7 buffer and the same volume of REMOVE-IT PNGase F. The reaction was incubated for 1 hour at 37°C and the results analyzed by SDS-PAGE.

*Saturation Mutagenesis.* Saturation mutagenesis at position 125 of the lysostaphin catalytic domain was carried out using known methods (Zhao, et al. (2014) *supra*)*.* Briefly, saturation mutagenesis was performed by splice overlap extension PCR using the lysostaphin synthetic gene as a template and degenerate NNK primers. The resulting 32-member library was transformed into *P. pastoris,* and transformants were grown on YPD medium (1% yeast extract, 2% peptone, 1% methanol, 1% agar) at 30°C for 48 hours. To find yeast clones expressing the active enzymes, molten top agar (0.5% yeast extract, 1% peptone, 1% NaCl, 0.75% agar) containing *S. aureus* SA113 cells was poured over YPM yeast plates and incubated at 37°C for 10 hours. Halo-forming colonies were picked out and amplified using primers Syn_F and Syn_R, and the genes were sequenced using primers AOX1_F and AOX1_R.

*MIC Assay Using P. pastoris Culture Supernatant.* Lysostaphin MIC was determined essentially as described (Zhao, et al. (2014) *supra*)*.* Briefly, 100 µl aliquots of *P*. *pastoris* culture supernatant were serially diluted in TSB. Each well was inoculated with 100 µl of ~10⁶ CFU/ml *S. aureus* SA113 in TSB. Microplates were incubated at 37°C for 24 hours. The inhibitory activity in culture supernatants was assessed as the MIC₅₀, the treatment dilution yielding 50% inhibition of growth. MIC₅₀ was quantified by measuring light scattering at 650 nm in a microplate reader.

*Thermostability.* The relative thermostability of the lysostaphin variants was determined by differential scanning fluorimetry, as previously described (Niesen, et al. (2007) Nat. Protocols 2:2212-2221). Proteins and SYPRO Orange were diluted in PBS (final concentrations of 100 µg/ml and 5x in 20 µl reaction volume, respectively), and fluorescence was quantified at 1-degree increments from 25 to 94°C using an Applied Biosystems ABI 7500 fast real-time PCR system. The reactions were performed using PCR Plates for Fast Thermocyclers (VWR; Radnor, PA). Fluorescence was quantified using the preset TAMRA parameters. Melting temperatures were determined by data analysis with the 'DSF Analysis v3.0.xlsx' EXCEL sheet and GraphPad Prism v.6.02 software.

*MHC Binding Assays.* MHC II competition binding assays were performed using a 384-well high throughput assay as previously described (Salvat, et al. (2014) *supra*)*.* Binding assays were performed for the eight alleles: DRB1*0101, 0301, 0401, 0701, 0801, 1101, 1301, and 1501. Briefly, 100 nM biotinylated control peptides composed of known peptide antigens for each MHC II allele were incubated in polypropylene 384-well plates with 50 nM purified recombinant MHC II protein and serial dilutions of LST or variant peptide fragments (100 µM to 10 nM). Peptide-MHC II complexes were captured from equilibrated solutions using the conformation specific anti-HLA-DR antibody L243 coated on high binding ELISA plates. Bound control peptide was quantified using the DELFIA streptavidin-Europium conjugate and time resolved fluorescence (SpectraMax Gemini Fluorescence Microplate Reader).

*Biofilm Degradation Assay. S. aureus* SA113 was grown overnight in TSB at 37°C shaking. The cells were then diluted 1:100 in TSB supplemented with 5% ethanol and 0.1% glucose and 100 µl of cell suspension was added to wells of a 96-well plate (Costar 3595). The cells were left to form biofilms overnight at 37°C without shaking. The resulting biofilms were then washed three times in water and treated with 200 ng of enzyme in 100 µl for 75 minutes. No-treatment wells contained PBS with 0.1% BSA. The plates were washed three times in water after the treatment and stained with 0.1% crystal violet for 15 minutes. The plates were then washed again three times in water and allowed to dry. Two hundred µl of 30% acetic acid was added to each well and allowed to dissolve the crystal violet stain for 15 minutes at 25°C with shaking. Destain (150 µl) was transferred to a new 96-well plate and the absorbance of each well was measured in a SPECTRAMAX 190 spectrophotometer (Molecular Devices; Sunnyvale, CA) at 550 nm.

*Murine Lung Infection Model.* Overnight LB cultures of *S. aureus* strain ATCC 25923 were pelleted, washed twice with PBS, and resuspended to give 10⁸-10⁹ colony forming units (CFU) in 40 µl of PBS. The actual inoculum was determined by serial dilution of the input bacterial suspension on LB agar (DIFCO), followed by incubation at 37°C for 24 hours. Adult female C57BL/6J mice (age, 8 to 12 weeks; Jackson Laboratories, Detroit, MI) were anesthetized briefly with isoflurane and inoculated with 40 µl of bacterial suspension via oropharyngeal aspiration. At 1 hour post-infection, a second 40 µl PBS inoculation containing either 2.5 µg wild-type LST, 2.5 µg variant Flex 5, 2.5 µg variant Flex 9, or a blank control. At 24 hours post-infection, mice were sacrificed and lungs were excised, placed into 1 ml of cold PBS, and homogenized. Viable bacterial counts in the lung homogenate were determined by plating serial dilutions onto LB agar, followed by incubation at 37°C for 24 hours.

*HUMI Murine Immunogenicity Studies.* HUMI mice were constructed by surgical transplantation of human bone marrow, liver, and thymus tissues into NOD/SCID/γ_{c}^{-/-} mice (Dartmouth Transgenics & Genetic Constructs Shared Resource) as described (Brainard, et al. (2009) J. Virol. 83:7305-21). All animals were humanized from the same human donor. Mice used experimentally had human lymphocytes as a minimum of 25% of their total peripheral blood leukocytes. Fourteen weeks postengraftment, 12 female HUMI mice were divided into 3 groups of 4 each and immunized with a single 50 µl subcutaneous injection of 100 µg wild-type LST, 100 µg variant Flex 5, or 100 µg variant Flex 9 in complete Freund's adjuvant (CFA). Two weeks following the immunization, mice were sacrificed and splenocytes were harvested and pooled for each group. Pooled splenocytes (5x10⁵/well) were plated in triplicate into 96-well plates with medium containing 5% fetal calf serum, l-glutamine, antibiotics, and a final concentration of 10 µg/ml LST or variants (or 1% DMSO as a control). After 72 hours of incubation, wells were pulsed with 1 µCi of [³H]thymidine (Dupont NEN, Boston, Mass.) and harvested 6 hours later onto UNIFILTER 96-well GF/C plates for assessment of thymidine incorporation by scintillation counting (Packard MicroSant NXT counter).

*Transgenic DR4 Murine Immunogenicity Studies.* Twelve female 6-8 week old DR4 transgenic mice (Abb Knockout/Transgenic HLA-DR4; B6.129S2-H2-Ab1tm1GruTg(HLA-DRA/H2-Ea,HLA-DRB1*0401/H2-Eb)1Kito; Taconic Farms, Germantown, NY) were divided into four groups of three each and immunized with 50 µl subcutaneous injections of wild-type LST using one of the following four schemes: (i) initial immunization with 100 µg enzyme in CFA, followed by 100 µg boosts in incomplete Freund's adjuvant (IFA) on days 14 and 28; (ii) initial immunization with 20 µg enzyme in CFA, followed by 20 µg boosts in IFA on days 14 and 28; (iii) initial immunization with 100 µg enzyme in PBS buffer, followed by 100 µg boosts in PBS buffer on days 7, 14, 21 and 28; (iv) initial immunization with 20 µg enzyme in PBS buffer, followed by 20 µg boosts in PBS buffer on days 7, 14, 21 and 28. Serum IgG antibody titers against wild-type LST were measured on days 13, 20, 27, 34, and 62. Five weeks after the final boost, all 12 mice exhibited equivalent maximum ELISA signals at a 1:40 serum dilution and all signals were within 20% at a 1:160 dilution. Mice were housed without further manipulation until week 23 of the study, at which time serum IgG antibody titers were again measured and mice were divided into two experimental arms having equivalent average antibody titers. Note that during the week 9 to week 23 recovery period, two mice (the lowest titer 100 µg no adjuvant and one of the high titer 100 µg adjuvant) began suffering hair loss, weight loss, and reduced mobility and were sacrificed as per the IACUC approved protocol. At week 24, one arm was rechallenged with 100 µg wild-type LST in IFA and the other arm with 100 µg variant Flex 5 in IFC. At week 26, mice were sacrificed and splenocytes were harvested and pooled for each group. Proliferation assays were conducted as described above.

*Bioinformatics Analysis.* Sequence alignment of lysostaphin and its homologous sequences ALE-1 and LytM was performed using ClustalW.

### Epitope Prediction

EpiMatrix analysis of the lysostaphin catalytic domain of an Asn125Gln mutant (Zhao, et al. (2014) *supra*) showed that the domain had many predicted T cell epitopes, with a total epitope score of 46. The protein had 14 instances of predicted top 1% binders (score>2.32), and 32 instances of top 5% binders (score>1.64). The sequence was also found to contain three EpiBars (peptides which have a score of 1.64 or higher for a minimum of four alleles), with the peptide ¹¹⁶FQRMVNSFS¹²⁴ (SEQ ID NO:88) predicted as highly immunogenic for all eight alleles (Table 2).

**TABLE 2**

| Peptide | SE Q ID NO : | Z Scores | | | | | | | |
|---|---|---|---|---|---|---|---|---|---|
| | | DRB1 * 0101 | DRB1 * 0301 | DRB1 * 0401 | DRB1 * 0701 | DRB1 * 0801 | DRB1 * 1101 | DRB1 * 1301 | DRB1 * 1501 |
| WLNNYKKG Y | 89 | | | | | 1.69 | 2.12 | 1.73 | 1.93 |
| LNNYKKGY G | 90 | | | | | 2.46 | | | 1.9 |
| INGGMHYG V | 91 | | | | | | | | 1.93 |
| VDFFMNIG T | 92 | | | | | | | | |
| FFMNIGTP V | 93 | 2.41 | | 1.79 | 1.93 | | | | |
| FMNIGTPV K | 94 | | | 2.19 | | | 2.06 | | |
| MNIGTPVK A | 95 | | 1.68 | | | | | | |
| IGTPVKAI S | 96 | | | | | | | 1.85 | |
| VKAISSGK I | 97 | 2.35 | 2.02 | 1.65 | 2.9 | | | | 2.75 |
| IENDGVHR Q | 98 | | | 2.06 | | | | | |
| VHRQWYMH L | 99 | | | | | | | | 1. 99 |
| WYMHLSKY N | 10 0 | | | | 2.72 | | | | |
| YMHLSKYN V | 10 1 | 1.86 | | | | 2.29 | 2.72 | | 2.01 |
| LSKYNVKV G | 10 2 | | | | | 1. 67 | | | |
| YVKAGQII G | 10 3 | 1.66 | | | | 1. 97 | | | |
| IIGWSGST G | 10 4 | | | 1.76 | | | | | |
| WSGSTGYS T | 10 5 | 1.95 | | | 1.92 | | | | |
| LHFQRMVN S | 10 6 | | | | | | | 2.05 | |
| FQRMVNSF S | 88 | 3.4 | 2.54 | 3.41 | 2.27 | 2.66 | 3.37 | 2.24 | 2.86 |
| QRMVNSFS Q | 10 7 | | | | 1.71 | | 1.8 | | |
| MVNSFSQS T | 10 8 | 1.91 | | | 2.41 | | | | 2.12 |

### Selection of the Most Frequent Mutations for a Preliminary Design Analysis

EpiSweep yielded a total of 1,533 plans, 81 of which were evaluated as Pareto optimal plans at mutational loads of two to eight mutations. At this point, it was possible to simply select a set of designs, which would then be subjected to experimental analysis for proper folding and activity. However, previous efforts to produce deimmunized lysostaphin based on sequence-based EpiSweep did not yield active variants. Structure-based EpiSweep was designed as an alternative method, which was supposed to produce better results. Given that inaccurate lysostaphin representation may have resulted in errors, an iterative feedback strategy was used, wherein the 15 most frequent mutations (individually) were tested for their impact on protein folding (expression) and activity.

The results (Table 3) showed that the most frequent 15 mutations were present in 9-67% of total plans, and in 1-65% of Pareto optimal plans. The mutations were all found on the surface of the catalytic domain. Buried residues were not frequently used by EpiSweep. Indeed, buried residues Ser49Gly and Ser49Ala take positions as the 18^{th} and 20^{th} mutation, respectively. Amino acids that were predicted to contribute to MHC binding included basic (Arg/Lys), polar uncharged (Ser/Asn/Tyr), and non-polar residues (Phe/Leu/Ile). These residues were replaced with non-polar (Gly/Met), acidic (Asp/Glu), basic (His), or polar uncharged (Thr/Gln/Tyr) amino acids.

**TABLE 3**

| Mutation | Total Number of Plans with Mutation | Number of Optimal Plans with Mutation | Surface/Buried Residue |
|---|---|---|---|
| Arg118Thr | 1031 (67%) | 53 (65%) | Surface |
| Ser124Gly | 1012 (66%) | 59 (73%) | Surface |
| Ser122Gly | 954 (62%) | 57 (70%) | Surface |
| Lys95Glu | 861 (56%) | 50 (62%) | Surface |
| Phe38Gly | 848 (55%) | 46 (57%) | Surface |
| Asn12Gly | 604 (39%) | 48 (59%) | Surface |
| Lys46His | 516 (34%) | 25 (31%) | Surface |
| Tyr93His | 516 (34%) | 26 (32%) | Surface |
| Ser122Asp | 348 (23%) | 18 (22%) | Surface |
| Leu83Met | 336 (22%) | 15 (19%) | Surface |
| Asn121Gly | 262 (17%) | 11 (14%) | Surface |
| Ser124Tyr | 246 (16%) | 7 (9%) | Surface |
| Asn13His | 179 (12%) | 4 (5%) | Surface |
| Ile41Glu | 161 (11%) | 10 (12%) | Surface |
| Ile99Gln | 143 (9%) | 1 (1%) | Surface |
| Thr110Glu | 136 (9%) | 1 (1%) | Surface |
| Leu83Asn | 136 (9%) | 5 (6%) | Surface |
| Ser49Gly | 117 (8%) | 0 | Buried |
| Val120Gln | 102 (7%) | 3 (4%) | Surface |
| Ser49Ala | 69 (5%) | 0 | Buried |

The mutations were predicted to significantly reduce the binding of peptides to the MHC and produce less immunogenic variants (Table 4). In all generated peptides, it could be seen that the number of mutant hits (the number of alleles a peptide was predicted to bind) was lower than that of the wild-type peptides. For instance, the Arg to Thr mutation at position 118 in the wild-type QRMVNSFSQ (SEQ ID NO:107) peptide was predicted to delete both epitopes and resulted in a Z score of 0 across all the alleles. Some of the more immunogenic regions were harder to tackle with a single mutation. Yet, it was observed that even a single mutation could have a meaningful impact on reducing the total immunogenicity score. For instance, the Ser122Asp mutation deleted two epitopes and reduced the overall hit number from 14 to 8.

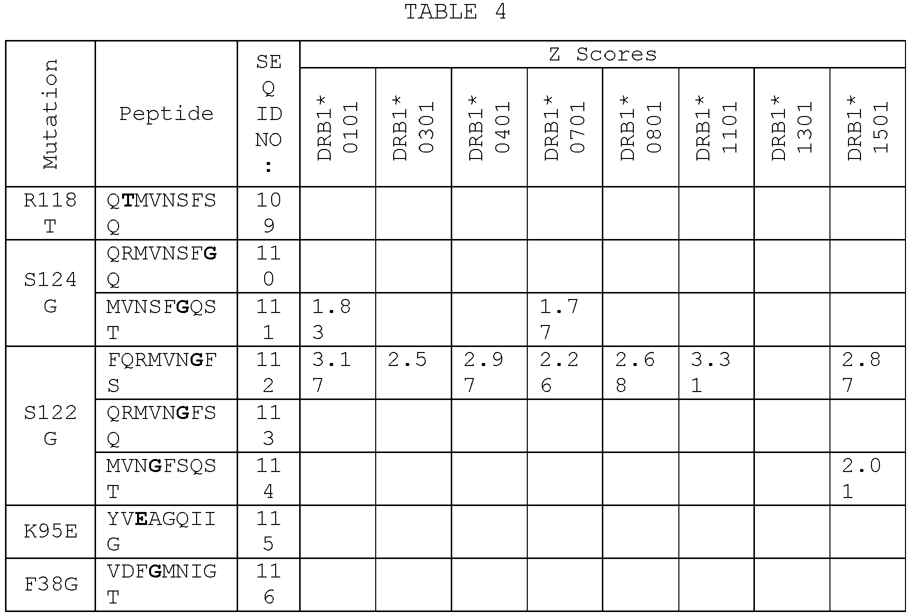

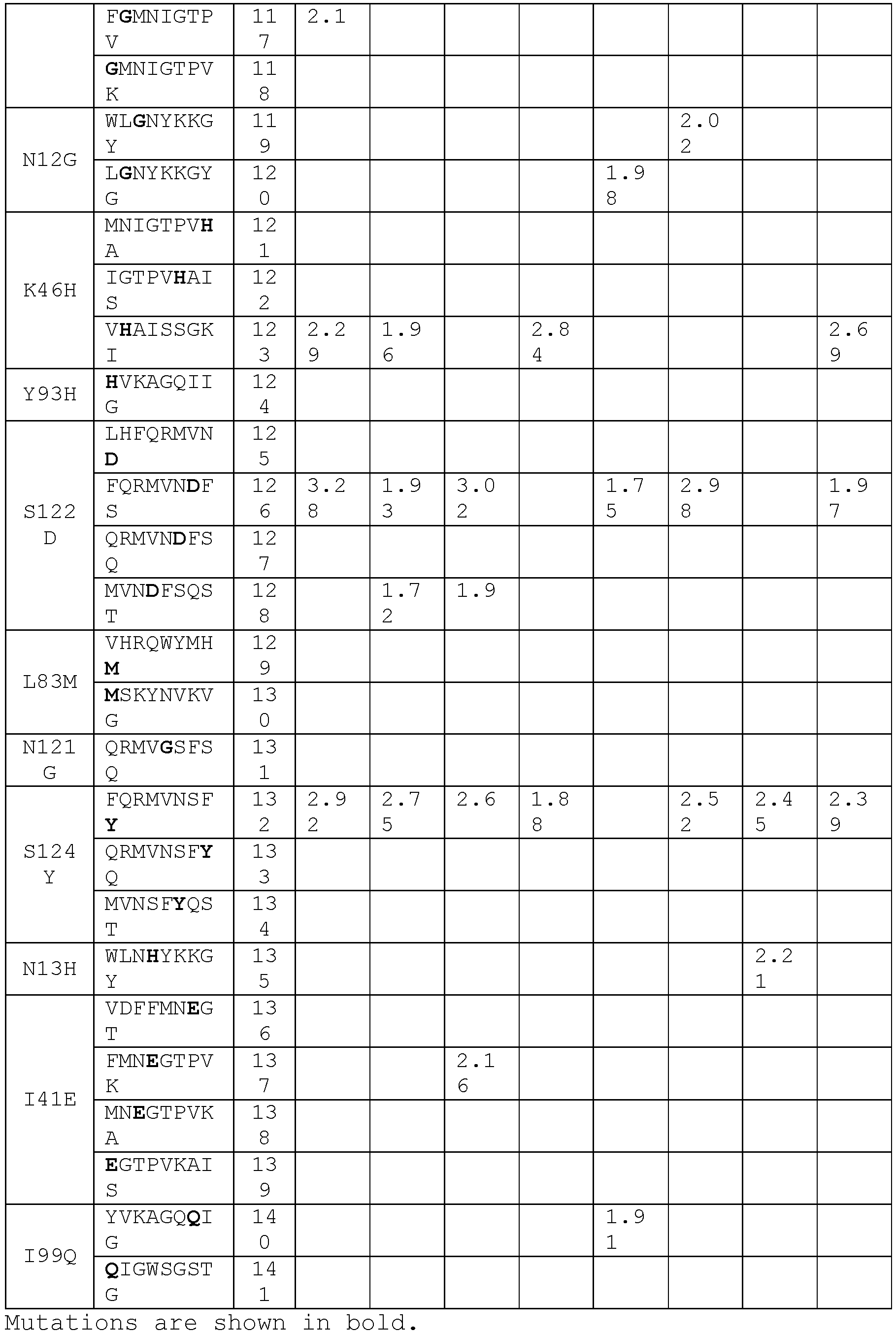

### Modification of Lysostaphin Sequence for P. pastoris Expression

Initial attempts to make *S. simulans* lysostaphin in *P. pastoris* were hampered by the lack of protein expression. Thus, the gene was modified for expression in *P. pastoris* (Zhao, et al. (2014) *supra).* Briefly, the sequence of the wild-type lysostaphin was adjusted to reflect the codon preference of *P. pastoris.* Additionally, a long segment with disproportionate A+T content in the sequence was identified and disrupted. The new version of the gene (SYN lysostaphin) was found to yield up to 80 mg/L of protein in shake flask culture, and 500 mg/L in a 2 L bioreactor (Zhao, et al. (2014) *supra) .*

Subsequent expression experiments conducted with SYN lysostaphin showed that the protein migrated as a doublet in SDS-PAGE. It was suspected that the observed doublets were due to protein N-glycosylation. Close examination of the lysostaphin sequence revealed that it contained a glycosylation sequon at position 125. The presence of the N-glycan was confirmed by PNGase treatment of the culture supernatant. Once treated, the protein migrated as a singlet in SDS-PAGE.

An attempt to disrupt the Asn125 glycosylation sequon was made by introducing conservative Asn->Gln, Asn->Ser, and Asn->Asp single point mutations. The results of this analysis indicated that these mutants exhibited similar expression levels but had 10-, 20- and 40-fold lower activity than the wild-type enzyme, respectively.

In subsequent studies, a library was constructed by saturation mutagenesis to determine which other residues besides Asn could be tolerated at position 125 and still allow for disruption of N-glycosylation sequence (Zhao, et al. (2014) *supra*)*.* The library results, and subsequent alignment of lysostaphin sequence with its homologues (ALE-1 and LytM), showed that Asn125 is a conserved residue, such that focus needed to be placed on mutating other residues of the glycosylation sequon. Thus, two other mutants, Ser126Pro and Thr127Ala, were synthesized and found to successfully prevent N-glycosylation. The mutants were compared to the wild-type lysostaphin and found to exhibit equivalent activity. Since a fully active aglycosylated mutant was desired, the lysostaphin Ser126Pro was selected for further experimental evaluation with the EpiSweep algorithm.

### EpiSweep Correction for Aglycosylated Lysostaphin

The Ser126Pro lysostaphin backbone was analyzed with EpiMatrix and EpiSweep to determine the epitope score and compare the most frequent mutations. The results showed that switching to the Ser126Pro mutant increased the predicted epitope score. Ser126Pro had a total epitope score of 50, as compared to epitope score of 46 for the Asn125Gln backbone (Table 2). EpiMatrix analysis also showed that Ser126Pro had five EpiBars (peptides which have a Z score of 1.64 or higher for a minimum of four alleles), while Asn125Gln had three. The Ser126Pro backbone had 15 instances of predicted top 1% binders (score>2.32), and 35 instances of top 5% MHC binders (score>1.64). Peptide ¹¹⁶FQRMVNSFS¹²⁴ (SEQ ID NO:88) remained equally problematic, as in the Asn125Gln backbone, and was predicted to be highly immunogenic for all eight alleles. The four new epitopes present in Ser126Pro backbone were introduced in peptides ¹¹⁹MVNSFSNPT¹²⁷ (SEQ ID NO:142) and ¹²⁰VNSFSNPTA¹²⁸ (SEQ ID NO:143) (Table 5).

**TABLE 5**

| Peptide | SE Q ID NO : | Z Scores | | | | | | | |
|---|---|---|---|---|---|---|---|---|---|
| | | DRB1 * 0101 | DRB1 * 0301 | DRB1 * 0401 | DRB1 * 0701 | DRB1 * 0801 | DRB1 * 1101 | DRB1 * 1301 | DRB1 * 1501 |
| WLNNYKKGY | 89 | | | | | 1.69 | 2.12 | 1.73 | |
| LNNYKKGYG | 90 | | | | | 2.46 | | | 1.93 |
| INGGMHYGV | 91 | | | | | | | | 1.9 |
| VDFFMNIGT | 92 | | | | | | | | 1.93 |
| FFMNIGTPV | 93 | 2.41 | | 1.79 | 1.93 | | | | |
| FMNIGTPVK | 94 | | | 2.19 | | | 2.06 | | |
| MNIGTPVKA | 95 | | 1.68 | | | | | | |
| IGTPVKAIS | 96 | | | | | | | 1.85 | |
| VKAISSGKI | 97 | 2.35 | 2.02 | 1.65 | 2.9 | | | | 2.75 |
| IENDGVHRQ | 98 | | | 2.06 | | | | | |
| VHRQWYMHL | 99 | | | | | | | | 1.99 |
| WYMHLSKYN | 10 0 | | | | 2.72 | | | | |
| YMHLSKYNV | 10 1 | 1.86 | | | | 2.29 | 2.72 | | 2.01 |
| LSKYNVKVG | 10 2 | | | | | 1. 67 | | | |
| YVKAGQIIG | 10 3 | 1.66 | | | | 1.97 | | | |
| IIGWSGSTG | 10 4 | | | 1.76 | | | | | |
| WSGSTGYST | 10 5 | 1.95 | | | 1.92 | | | | |
| LHFQRMVNS | 10 6 | | | | | | | 2.05 | |
| FQRMVNSFS | 88 | 3.4 | 2.54 | 3.41 | 2.27 | 2.66 | 3.37 | 2.24 | 2.86 |
| MVNSFSNP T | 14 2 | 2 | | 1.9 | 2.79 | | 1.78 | | 2.25 |
| VNSFSNPT A | 14 3 | | | 2.03 | | | 1.96 | 2.05 | 2.55 |

EpiSweep analysis of the Ser126Pro backbone yielded a total of 2,333 plans, 96 of which were evaluated as Pareto optimal plans at mutational loads of two to eight mutations (compared with the Asn125Gln backbone which yielded a total of 1,533 plans, 81 of which were Pareto optimal). Table 6 compares the most frequent mutations found in the Asn125Gln and Ser126Pro backbones. The mutation pattern was not significantly changed after switching to the new backbone. Most mutations remained in the top 15, with exception of only four mutations: Ser122Gly, Ser124Gly, Leu83Met and Ile99Gln moved to positions 16, 18, 23 and 24, respectively. The most frequent mutation, Arg118Thr, remained as dominant in both backbones. While in the Asn125Gln backbone all 15 most frequent mutations were surface exposed residues, the new backbone pushed a buried residue, Ser49Gly, to the top 15. Most mutations were similarly represented in total plans and optimal plans, with an exception of Ser124Gly, which changed from being present in 72% of the plans, to not being present in any of the optimal plans selected with the Ser126Pro backbone. The most frequent 15 mutations (evaluated based on the Asn125Gln backbone) were present in 2-82% of total plans generated using the Ser126Pro backbone, and in 0-88% of Pareto optimal plans.

**TABLE 6**

| Mutation | Percentage of Plans with Mutation | Percentage of Optimal Plans with Mutation | Ranking Order change |
|---|---|---|---|
| Arg118Thr | 67->82% | 65->88% | 1->1 |
| Ser124Gly | 66->9% | 72->0% | 2->18 |
| Ser122Gly | 62->10% | 70->4% | 3->16 |
| Lys95Glu | 56->53% | 61->58% | 4->2 |
| Phe38Gly | 55->18% | 56->25% | 5->11 |
| Asn12Gly | 39->49% | 59->78% | 6->3 |
| Lys46His | 33->18% | 30->18% | 7->10 |
| Tyr93His | 33->45% | 32->58% | 8->4 |
| Ser122Asp | 22->43% | 22->54% | 9->5 |
| Leu83Met | 21->4% | 18->1% | 10->23 |
| Asn121Gly | 17->26% | 13->32% | 11->7 |
| Ser124Tyr | 16->12% | 8->9% | 12->15 |
| Asn13His | 11->15% | 4->10% | 13->14 |
| Ile41Glu | 10->21% | 12->26% | 14->9 |
| Ile99Gln | 9->2% | 1->3% | 15->24 |
| Thr110Glu | 8->25% | 1->27% | 16->8 |
| Leu83Asn | 8->10% | 6->8% | 17->17 |
| Ser49Gly | 7->18% | 0->27% | 18->12 |
| Val120Gln | 6->6% | 3->0% | 19->21 |
| Ser49Ala | 4->7% | 0->0% | 20->20 |

The 15 mutations based on the Asn125Gln backbone were still predicted to significantly reduce the binding of residues to MHC II and produce less immunogenic variants in the Ser126Pro backbone (Table 7). The predicted deletions were largely similar to those obtained with Asn125Gln. The overall mutant hit rate with Asn125Gln was 35 (40 with Ser126Pro) and the wild-type hit rate is 84 (88 with Ser126Pro), so the two backbones targeted roughly the same number of epitopes.

The one significant difference observed was that the Arg118Thr mutation, which deleted two epitopes in the Asn125Gln backbone, did not delete any epitopes in the Ser126Pro backbone. However, closer examination showed that Arg118Thr helped other mutations delete more epitopes. For instance, when Arg118Thr was combined with Ser122Asp, the two mutations deleted a total of 13 epitopes. As such, the Arg118Thr mutation was not eliminated from further study. However, the Ser122Gly mutation was excluded since it targeted the same residue as Ser122Asp but only deleted three epitopes (as opposed to 10 that Ser122Asp was predicted to remove) .

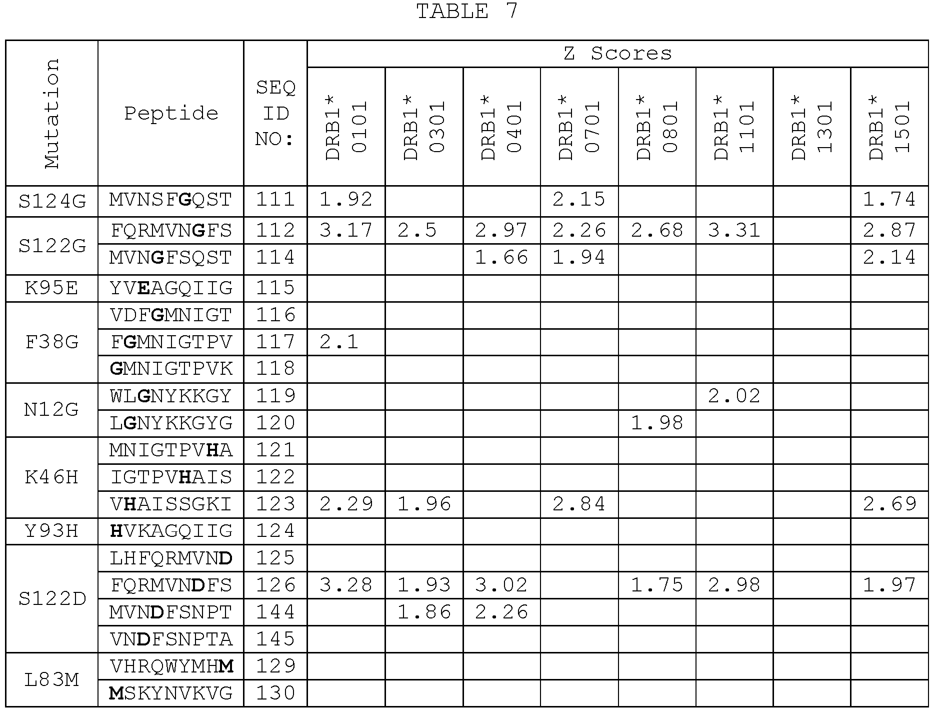

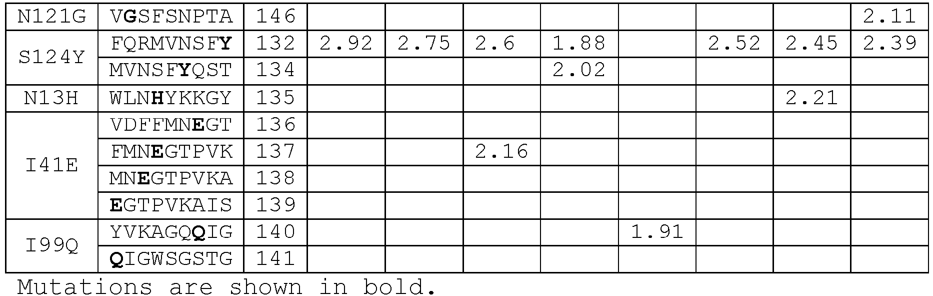

### Expression Level and Activity of Lysostaphin Variants

Even though the algorithm was focused on deimmunization of only the catalytic domain, it is important to note that the single point mutants were tested in the context of a full-length, mature protein to ensure proper protein folding. Since lysostaphin is secreted into media, a simple analysis of lysostaphin activity from the culture supernatant was considered a sufficient evaluation of the effect of single point mutations on the protein.

The analysis of lysostaphin single point mutants revealed that all variants were expressed in *P. pastoris.* Thus, none of the most frequent EpiSweep mutations abolished expression. The two mutations with the lowest expression levels were Arg118Thr and Ser124Tyr, with 47% and 35% of the wild-type expression level, respectively. Furthermore, 13 of the lysostaphin variants had activity that either equaled or exceeded that of the wild-type enzyme. Point mutant Phe38Gly was one of the two mutations with activity lower than the wild-type (69%). The second mutation with low activity was Ser124Tyr, with 16% of the wild-type activity. Since Phe38Gly and Ser124Tyr did not meet threshold activity levels of >70% of the wild-type activity, neither enzyme was included in further studies. While the expression level of the Arg118Thr mutation was less than 50% of the wild-type enzyme, this enzyme was further analyzed given the number of epitopes removed by this mutation.

### Backbone Flexibility Adjustment

All designs obtained using EpiSweep assumed a rigid backbone (referred to herein as "rigid" backbone designs). However, since proteins are known for their high level of flexibility, it was posited that keeping the backbone fixed in the post-processing energy minimization step could compromise the accuracy of deimmunized variants by resulting in imprecise energy assessments. It has been observed that completely rigid designs differ drastically in energy-epitope score landscape from the designs in which the side chains were allowed to relax (while the backbone was fixed) during minimization (Parker, et al. (2013) *supra*). Thus, to achieve flexibility in a computationally-permitting way, additional post-processing energy minimization of the EpiSweep designs was performed (referred to herein as "flexible" backbone designs). The results of this analysis showed that there was indeed a visible movement between the two backbones.

### EpiSweep Analysis of Chosen Mutations

As described above, the expression and activity of the 15 most frequent mutations were analyzed. Based upon this analysis, two mutations were dropped due to unsatisfactory activity values. In addition, Ser122Gly was removed as it targeted the same residue as Ser122Asp, which deleted 10 epitopes compared to the three epitopes of the Ser122Gly mutation. The most frequent mutation, Arg118Thr, did not delete any epitopes in the context of the Ser126Pro backbone and had a relatively low expression level. However, Arg118Thr was maintained as it assisted other mutations in targeting epitopes. For instance, when combined with Ser122Asp, the Arg118Thr mutation targeted a total of 13 epitopes. As a result, the data set contained 12 different mutations: Ser124Gly, Ser122Asp, Asn121Gly, Arg118Thr, Ile99Gln, Lys95Glu, Tyr93His, Leu83Met, Lys46His, Ile41Glu, Asn13His, and Asn12Gly.

EpiSweep analysis was performed once again, but the algorithm was constrained to use only the 12 well-tolerated mutations. The mutational load was allowed to vary from two to eight mutations and the algorithm was forced not to introduce mutations at the active site (His32, Asp36, His82, His113, and His115). Since it was observed that the Ser122Asp mutation was an extremely efficient epitope remover, the algorithm was constrained to include this mutation in all generated plans. As before, the algorithm generated Pareto optimal plans and an additional 19 suboptimal plans at each mutational load. The lowest epitope score was found to be 24, and could only be achieved with plans containing eight mutations. The highest epitope score was 40, and the plans with this score had only the forced Ser122Asp mutation.

A number of plans along the Pareto optimal curve (designs that have the lowest possible rotamer energy at a fixed epitope score and mutational load) were selected. EpiSweep analysis yielded rigid backbone plans, and a total of 14 Pareto optimal plans at mutational loads ranging from two to eight mutations were selected, with the epitope score in the range of 24 (lowest) to 36. Since the wild-type lysostaphin backbone had an epitope score of 50, it was posited that this range covered designs that were significantly deimmunized.

To address the protein flexibility concern, 14 designs that were energy minimized post-processing (flexible backbone designs) were also included. The designs at the same epitope scores and mutational loads as the rigid backbone designs were selected so that the two different methods of obtaining variants could be directly evaluated. For instance, if a Pareto optimal rigid plan with eight mutations and an epitope score of 24 were chosen, a flexible backbone design at the same mutational load and epitope score was selected. When faced with several options, the flexible backbone design of the lowest rotamer energy was chosen. Because they were energy minimized, these designs did not appear on the Pareto frontier. It should be noted that the two mutation plan was shared between the rigid and flexible backbone designs.

### Expression Level and Activity of Synthesized Variants

Each lysostaphin variant was grown in shake flask culture and examined for expression level by SDS-PAGE. The plans that were found to express at a meaningful level in culture supernatants (by SDS-PAGE detection) were further characterized for their relative specific activities. The results showed that out of 28 rigid and flexible backbone plans, only 11 were expressed. Expression levels of all variants except Rigid 1 were found to be lower than that of the wild-type lysostaphin.

All of the plans (except Flex 1; SEQ ID NO:32) had activity lower than that of the wild-type enzyme, but only two had activity lower than the commercial E. coli-produced lysostaphin. It was noted that there was a significant difference between the activity of the wild-type lysostaphin produced in *P. pastoris* and ssLys. Out of a total of 11 expressing plans, six were flexible backbone designs and five were rigid backbone designs. Rigid design mutational load ranged from two to six, and flexible from two to eight.

The activity level of the plans decreased as the number of mutations increased. This trend was better observed in flexible rather than the rigid backbone designs. Similarly, in flexible plans, it was found that the expression level decreased with an increase in the mutational load. This trend was not observed in rigid backbone designs, as their expression level remained low regardless of the mutational load. The only rigid design that had a high expression level was the two mutation design Rigid 1, which was shared between the rigid and flexible designs (also referred to as Flex 14).

### Analysis of Non-Expressing Mutants

Since less than a half of the synthesized designs expressed at a meaningful level, the non-expressing designs were analyzed for their mutational patterns. This analysis showed that expressing and non-expressing designs shared the majority of the mutations. Some mutations, such as Leu83Met, Tyr93His, and Lys95Glu, were present in most of the non-expressing plans, but only in a few expressing plans. Similarly, Arg118Thr was present in only a few expressing plans but, unlike any other mutation (the forced Ser122Asp mutation excluded), it could be found in all of the non-expressing plans (Table 8).

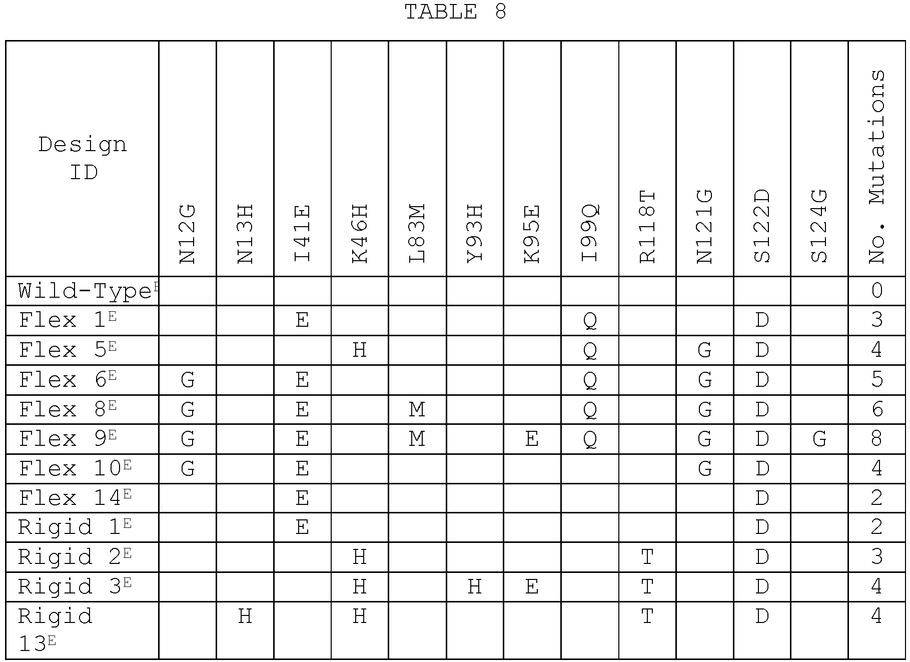

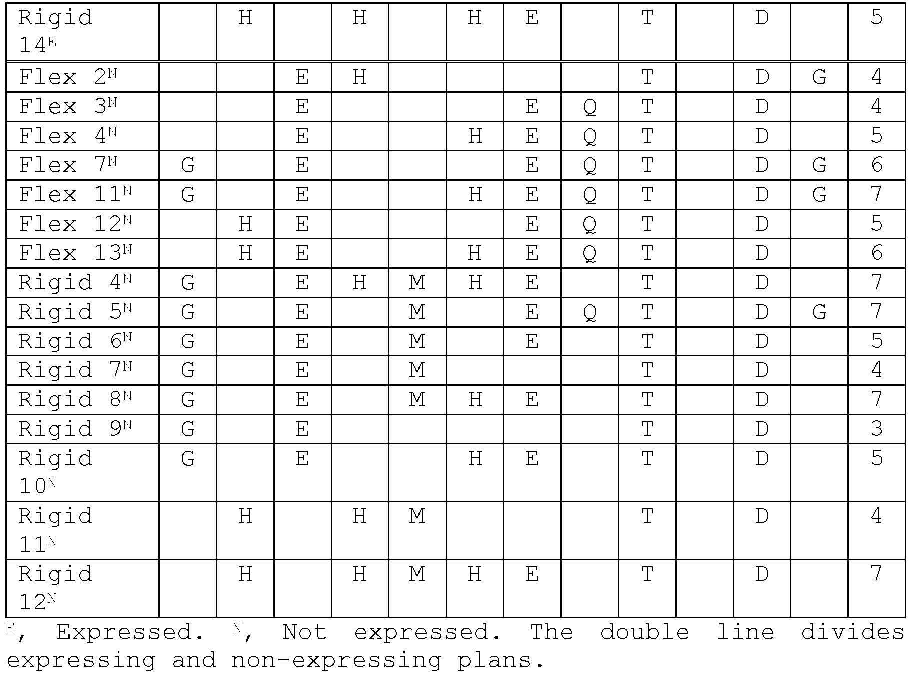

Since earlier results showed that mutation Arg118Thr had a relatively low expression level (less than 50% of wild type), it was posited that the observed lack of expression in the majority of the synthesized plans may have been due to the Arg118Thr mutation. To test this, the mutation was reverted back to wild-type in all plans having this mutation.

### Characterization of Both Original and Reverted Plans

All expressed plans were purified for further characterization of activity and stability. Furthermore, all of the designs that originally had the Arg118Thr mutation, but now had the wild-type residue at the same position (reverted plans), were evaluated. The results of this analysis are shown in Table 9.

**TABLE 9**

| Design ID | Flexible Energy | Rigid Energy | Episcore | % Wild-Type Activity | MIC (µg/mL) | Tm (°C) |
|---|---|---|---|---|---|---|
| Wild-Type | -3929 | -48.5 | 50 | 100 | 0.0351 | 59.2 |
| Flex 1 | -4130 | -47.2 | 34 | 63 | 0.0833 | 55.4 |
| Flex 5 | -3954 | -49.7 | 32 | 61 | 0.1042 | 55.7 |
| Flex 6 | -3978 | -49.3 | 28 | 58 | 0.2083 | 54.1 |
| Flex 8 | -3974 | -49.8 | 26 | 60 | 0.2083 | 52.9 |
| Flex 9 | -4005 | -50.8 | 24 | 57 | 0.1250 | 53.0 |
| Flex 10 | -3904 | -49.4 | 30 | 62 | 0.0729 | 57.5 |
| Flex 14 | -4056 | -47.4 | 36 | 69 | 0.0833 | 58.6 |
| Rigid 1 | -4056 | -47.4 | 36 | 69 | 0.0833 | 58.6 |
| Rigid 2 | -3784 | -55.5 | 34 | 72 | 0.1042 | 49.2 |
| Rigid 13 | -3699 | -57.1 | 32 | 71 | 0.0521 | 46.9 |
| Flex 2* | -4016 | -49.5 | 35 | 77 | 0.0833 | 58.4 |
| Flex 3* | -4166 | -47.7 | 33 | 61 | 0.2500 | 56.2 |
| Flex 4* | -4143 | -50.7 | 33 | 36 | 0.2083 | 57.5 |
| Flex 7* | -4084 | -49.0 | 29 | 67 | 0.2500 | 54.7 |
| Flex 11* | -4059 | -47.2 | 29 | 76 | 0.1250 | 52.8 |
| Flex 12* | -4079 | -49.4 | 31 | 54 | 0.1250 | 54.8 |
| Flex 13* | -4056 | -52.3 | 31 | 71 | 0.1667 | 54.2 |
| Rigid 2* | -3957 | -49.5 | 37 | 80 | 0.1042 | 57.1 |
| Rigid 3* | -3884 | -53.0 | 35 | 79 | 0.0833 | 57.3 |
| Rigid 4* | -3884 | -55.3 | 26 | 55 | 0.2500 | 54.1 |
| Rigid 5* | -4080 | -55.5 | 27 | 66 | 0.1667 | 52.5 |
| Rigid 6* | -4013 | -49.9 | 29 | 64 | 0.2083 | 53.3 |
| Rigid 7* | -3977 | -49.4 | 31 | 80 | 0.0729 | 55.0 |
| Rigid 9* | -3980 | -48.9 | 33 | 79 | 0.0729 | 56.5 |
| Rigid 10* | -3993 | -52.4 | 31 | 77 | 0.1458 | 55.6 |
| Rigid 11* | -3867 | -51.7 | 33 | 70 | 0.0313 | 55.7 |
| Rigid 13* | -3871 | -51.2 | 35 | 92 | 0.0625 | 56.9 |
| Rigid 14* | -3884 | -54.6 | 33 | 75 | 0.1042 | 56.1 |

| | | | | | | |
|---|---|---|---|---|---|---|
| Reverted plans are indicated with *. | | | | | | |

Out of a total of 32 plans (original and reverted), 28 were found to express. The 28 that expressed well were purified and characterized for their activity and stability. During the preliminary analysis, Rigid 3 and Rigid 14 were found to express weakly in the culture supernatant. Reverted versions of Rigid 8 and 12 did not significantly improve expression. It is possible that these four plans had low expression levels due to the presence of other mutations besides Arg118Thr, but no obvious mutational patterns could be found. All other plans in which the Arg118Thr mutation was changed back to the wild-type sequence were found to express well. As such, the Arg118Thr mutation appeared to affect expression for the majority of the plans.

The results showed that the variants had high levels of activity and stability, as compared to the wild-type enzyme. Activity values were expressed as % of the wild-type activity, and ranged from 92% (Rigid 13*) to 36% (Flex 4*). The observed MIC values were close to the wild-type, with the highest being 0.25 µg/ml (Flex 3*, Flex 7* and Rigid 4*). Tm values were also similar to that observed in the wild-type enzyme, with the lowest Tm showing a decrease in stability of 12.3°C (Rigid 13, Table 9).

This analysis indicated that an increase in the mutational load resulted in a decrease in activity/Tm values of the variants and an increase in MIC values. On average, rigid designs had a higher specific activity and lower MIC values than flexible designs. Flexible designs, however, had a better overall stability than rigid designs, as evidenced by higher average Tm values. Based on the results of a two-tailed t-test, the differences observed between the two design groups were statistically significant (p-values <0.03).

The designs were divided into the seven different groups (Table 10) for further characterization. Using the data from Table 9, the Pearson correlation coefficients between the flexible/rigid energies, mutational loads, activity, and stability for each design group were calculated.

**TABLE 10**

| | Pearson (P-value) | | | | | | |
|---|---|---|---|---|---|---|---|
| | Flexible original | Rigid original | Flexible Reverted | Rigid Reverted | All Flexible | All Rigid | All Designs |
| NM vs. Act. | -0.86 (0.01) | 0.55 (0.63) | 0.26 (0.57) | -0.80 (0.00) | -0.04 (0.88) | -0.61 (0.02) | -0.38 (0.04) |
| NM vs. MIC | 0.53 (0.22) | -0.59 (0.59) | -0.03 (0.95) | 0.73 (0.01) | 0.36 (0.21) | 0.69 (0.01) | 0.56 (0.00) |
| NM vs. Tm | -0.85 (0.02) | -0.94 (0.21) | -0.85 (0.02) | -0.12 (0.73) | -0.79 (0.00) | -0.09 (0.76) | -0.24 (0.22) |
| FE vs. Act. | -0.36 (0.43) | 0.77 (0.44) | 0.72 (0.07) | 0.20 (0.56) | 0.21 (0.47) | 0.08 (0.79) | 0.39 (0.04) |
| FE vs. MIC | 0.22 (0.63) | -0.34 (0.78) | -0.79 (0.04) | -0.35 (0.29) | -0.38 (0.18) | -0.34 (0.23) | -0.48 (0.01) |
| FE vs. Tm | -0.05 (0.92) | -1.00 (0.03) | -0.14 (0.77) | -0.02 (0.95) | -0.10 (0.74) | -0.59 (0.03) | -0.49 (0.01) |
| RE vs. Act. | 0.78 (0.04) | -0.8 (0.4) | 0.25 (0.59) | 0.52 (0.1) | 0.3 (0.3) | 0.36 (0.21) | -0.07 (0.75) |
| RE vs. MIC | -0.4 (0.35) | 0.27 (0.83) | 0.05 (0.91) | -0.4 (0.17) | -0.2 (0.56) | -0.2 (0.46) | 0.05 (0.79) |
| RE vs. Tm | 0.63 (0.13) | 1.00 (0.02) | -0.2 (0.66) | 0.09 (0.8) | 0.17 (0.57) | 0.55 (0.04) | 0.48 (0.01) |
| No. of Designs | 7 | 3 | 7 | 11 | 14 | 14 | 28 |

| | | | | | | | |
|---|---|---|---|---|---|---|---|
| NM, No. of Mutations; Act., Activity; FE, Flexible Energy; RE, Rigid Energy. | | | | | | | |

When considered together, all 28 designs showed a weak positive correlation between the number of mutations and the MIC value (Table 10, All Designs). Thus, as mutational load of the variants increased, their activity decreased (MIC value increased). A stronger positive correlation between the number of mutations and MIC was found in rigid reverted and all rigid plans.

Other correlations that were not obvious when all the plans were analyzed together become apparent when designs were evaluated separately. For instance, it was observed that a strong negative correlation existed between the mutational load and activity in the original flexible plans, reverted rigid plans, and all rigid plans. A strong negative correlation was also found between the number of mutations and Tm values in flexible original, flexible reverted, and all flexible plans.

Overall, strong correlations were not observed between energy and activity/stability terms when all designs were considered together. The only meaningful correlation observed was a weak negative correlation between the flexible energy and Tm (Table 10, All Designs). The correlations observed between the flexible energy and activity terms had opposite signs than what was expected. On the other hand, correlations between the rigid energy and activity were not significant, while the correlation between rigid energy and Tm had incorrect sign. Thus, to further examine whether energy was a reliable predictive tool, the correlations between energy components and experimentally determined activity and stability values were evaluated.

Even though flexible and rigid energies were not great predictors of activity, it was found that energy due to solvent interactions could be used instead (Table 11). As expected for a functional predictor, solvent energy had a negative (although weak) correlation with activity, and a stronger positive correlation with MIC values.

**TABLE 11**

| | Pearson Correlation Coefficient | | |
|---|---|---|---|
| | Activity | MIC | Tm |
| Flexible Energy | 0.391 | -0.479 | -0.0488 |
| Energy Due to Intermolecular Forces | 0.402 | -0.0514 | -0.0282 |
| Energy Due to Charge-Charge Interactions | 0.449 | -0.605 | -0.291 |
| Energy Due to Solvent Interactions | -0.397 | 0.556 | 0.064 |
| Rigid Energy | -0.065 | 0.054 | 0.484 |

Taken together, these results showed that flexible backbone energy was a relatively good predictive tool of stability, while energy due to solvent interactions seemed to be the best predictor for experimental activity observed using a MIC assay.

### Characterizing Immunoreactivity of Lysostaphin Variants

The predicted epitopes in the wild-type lysostaphin catalytic domain were broadly distributed throughout the lysostaphin sequence, but the majority of the epitopes could be grouped into five clusters (Figure 2). Most epitopes were predicted to have a high number of binding events.

To evaluate the relative immunogenicity of the variants, a total of 26 synthetic peptides spanning the sequence of the lysostaphin catalytic domain were designed. In particular, focus was placed on the five immunogenic clusters shown in Figure 2. For each cluster listed, a wild-type peptide and the corresponding mutated peptide were designed. The mutated peptides contained both the single mutation and mutational combination that appeared in plans.

In the context of synthetic peptides, each mutation was evaluated for its predicted potential to eliminate epitopes. EpiBars (peptides that had a Z score of 1.64 or higher for a minimum of four alleles on the EpiMatrix immunogenicity scale) were found in cluster 2 (⁴⁵VKAISSGKI⁵³, SEQ ID NO:97), cluster 3 (⁸⁰WMHLSKYNV⁸⁸, SEQ ID NO:147), and cluster 5 (¹¹⁶FQRMVNSFS¹²⁴, SEQ ID NO:88; ¹¹⁹MVNSFSNPT¹²⁷, SEQ ID NO:142; and ¹²⁰VNSFSNPTA¹²⁸, SEQ ID NO:143).

Each peptide from was experimentally evaluated for its binding potential to eight MHC II alleles in a high-throughput MHC II binding assay. The EpiSweep-generated mutations generally lessened the binding of the peptides to the MHC. Out of the 168 pair-wise comparisons, mutations decreased binding affinity in 73 cases, had no effect in 60 cases and increased binding in 35 cases. A peptide was classified as a strong binder if an IC₅₀ value of less than 0.1 µM was observed, moderate if an IC₅₀ value was in the 0.1-1 µM range, and weak if an IC₅₀ value was in the 1-10 µM range. All peptides above 10 µM were considered non-binders.

Using the 10 µM cutoff to separate binders from non-binders, EpiMatrix predictions (at 5% threshold) were compared with the MHC II binding results. The percentage of true positives (correctly predicted binders), true negatives (correctly predicted non-binders), false positives (incorrectly predicted binders), and false negatives (incorrectly predicted non-binders) were calculated. The results showed that the overall predictive success rate was 70%, a result that was slightly lower than the previously published studies, which cite the predictive rate as ~76% (Groot, et al. (2011) Immunome Res. 7:2-7; Moise, et al. (2013) Humm. Vaccin. Immunother. 9:2060-2068). Allele-specific examination revealed that the predictions were in the previously observed range for DRB1*0101, 0301, 0401, and 0701. No data could be found for 0801. It was observed that for 1301 the majority of peptides registered as either weak or non-binders. This observation would suggest that the test peptide used for 1301 was likely a really strong binder, and as such had skewed the data toward a smaller predictive success rate. Similarly, the predictive rate for 1101 and 1501 was lower than previously reported and was also thought to have contributed to the lower overall rate.

The cluster 1 wild-type (C1WT) epitope was predicted to bind four out of eight MHC II alleles tested. In accordance with the prediction, both Asn12Gly and Asn13His mutations disrupted MHC II binding. Asn13His mutation was better at removing DRB1*0801 and 1101 epitopes, and the mutations were equally good at removing DRB1*1501 epitope. However, the mutations also resulted in strong binding for DRB1*0101 (Asn12Gly), and weak binding for DRB1*1301 (Asn13His) (Table 12).

**TABLE 12**

| Peptide | IC₅₀, µM (No. of predicted eptitopes) | | | | | | | |
|---|---|---|---|---|---|---|---|---|
| | 0101 | 0301 | 0401 | 0701 | 0801 | 1101 | 1301 | 1501 |
| C1WT¹ | 214. 9 (0*) | >250 (0*) | >250 (0*) | >250 (0*) | 13.73 (2) | 0.94 (1*) | 136.70 (1) | 101.50 (1) |
| N12G² | <0.01 (0) | >250 (0*) | >250 (0*) | >250 (0*) | 58.63 (1) | 5.16 (1*) | >250 (0*) | >250 (0*) |
| N13H³ | >250 (0*) | >250 (0*) | >250 (0*) | >250 (0*) | >250 (1) | 4.13 (0) | 41.63 (1) | >250 (1) |

| | | | | | | | | |
|---|---|---|---|---|---|---|---|---|
| ¹SAQWLNNYKKGYGYG (SEQ ID NO:148). ²SAQWLGNYKKGYGYG (SEQ ID NO:221). ³SAQWLNHYKKGYGYG (SEQ ID NO:222). *Positive correlation between predicted binders and experimentally observed binders. Binding thresholds set to 5% for predictions and 10 µM for experiments. | | | | | | | | |

In the case of cluster 2 wild-type (C2WT) epitope, binding was predicted for seven MHC II alleles, with multiple epitopes for DRB1*0101, 0301, 0401, 0701, and 1501 (Table 13). The mutations were predicted to target the seven alleles except for DRB1*0101 and 0701, and for those three alleles (in addition to 0401) an increase in binding was observed. One exception was the Lys46His mutant, which showed a two-fold decrease in binding to 0101. Lys46His also reduced binding to 1101, while Ile41Glu and Ile41Glu/Lys46His resulted in strong binding. All mutants had lower affinity for 0301, as predicted. Ile41Glu and Lys46His showed reduced binding to 1501, while Ile41Glu/Lys46His emerged as a strong binder. The combination of mutations was particularly bad as it resulted in increased binding for all alleles but 0301. The high binding affinity of this cluster could be explained by the large number of epitopes. Out of a total of 13 epitopes across the eight alleles, the mutations were predicted to disrupt only four epitopes. Furthermore, this particular region contained an EpiBar, and previous studies show that these epitopes tend to be more immunogenic than epitopes that do not have EpiBars (Groot, et al. (2011) *supra*).

**TABLE 13**

| Peptide | IC₅₀, µM (No. of predicted eptitopes) | | | | | | | |
|---|---|---|---|---|---|---|---|---|
| | 0101 | 0301 | 0401 | 0701 | 0801 | 1101 | 1301 | 1501 |
| C2WT¹ | 0.22 (2*) | 60.10 (2) | 13.73 (3) | 8.19 (2*) | >250 (0*) | 10.08 (1) | 103.90 (1) | 2.37 (2*) |
| I41E² | 0.59 (2*) | >250 (1) | 3.48 (3*) | 7.51 (2*) | >250 (0*) | 146.10 (0*) | 142.60 (0*) | 7.44 (1*) |
| K46H³ | 0.49 (2*) | 151.62 (1) | 1.65 (2*) | 4.46 (2*) | 87.48 (0*) | 18.29 (1) | 212.00 (0*) | 2.55 (2*) |
| 41_46⁴ | <0.01 (2*) | >250 (1) | <0.01 (2*) | 14.33 (2) | >250 (0*) | <0.01 (1*) | >250 (0*) | <0.01 (1*) |

| | | | | | | | | |
|---|---|---|---|---|---|---|---|---|
| ¹GVDFFMNIGTPVKAISSGKIV (SEQ ID NO:223). ²GVDFFMNEGTPVKAISSGKIV (SEQ ID NO:224). ³GVDFFMNIGTPVHAISSGKIV (SEQ ID NO:225). ⁴GVDFFMNEGTPVHAISSGKIV (SEQ ID NO:226). *Positive correlation between predicted binders and experimentally observed binders. Binding thresholds set to 5% for predictions and 10 µM for experiments. | | | | | | | | |

The cluster 3 wild-type (C3WT) epitope was a predicted binder of DRB1*0101, 0701, and 1101, with multiple epitopes for 0801 and 1501 (Table 14). C3WT was experimentally shown to also weakly bind 1301. Leu83Met mutation was anticipated to disrupt binding in only 0801 and 1501. In agreement with the prediction, an approximately five-fold decrease in binding for 0801 was found. However, more than a two-fold decrease in 0701 (strong to moderate), ten-fold reduction for 1101, and a ~2000-fold drop for 0101 (strong to weak) was also observed. Increased binding was detected for 1501 (opposite of what was predicted), 1301, and 0301. For 1501, a shift from a weak to a moderate binder was observed, while 1301 remained a weak binder, and 0301 a non-binder. Thus, the Leu83Met mutation was largely productive as it reduced binding to four alleles and did not cause a change of classification for two other alleles (peptides remained weak or non-binders) .

**TABLE 14**

| Peptide | IC₅₀, µM (No. of predicted eptitopes) | | | | | | | |
|---|---|---|---|---|---|---|---|---|
| | 0101 | 0301 | 0401 | 0701 | 0801 | 1101 | 1301 | 1501 |
| C3WT¹ | <0.01 (1*) | 155.43 (0*) | 38.12 (0*) | 0.41 (1*) | 0.14 (2*) | <0.01 (1*) | 54.69 (0*) | 9.51 (2*) |
| L83M² | 32.39 (1) | 114.83 (0*) | >250 (0*) | 2.09 (1*) | 0.37 (1*) | 0.11 (1*) | 27.38 (0*) | 4.42 (1*) |

| | | | | | | | | |
|---|---|---|---|---|---|---|---|---|
| ¹GVHRQWYMHLSKYNVKVGD (SEQ ID NO:149). ²GVHRQWYMHMSKYNVKVGD (SEQ ID NO:150). *Positive correlation between predicted binders and experimentally observed binders. Binding thresholds set to 5% for predictions and 10 µM for experiments. | | | | | | | | |

The cluster 4 wild-type (C4WT) epitope was predicted to bind three out of eight MHC II alleles tested: DRB1*0101, 0401, and 0801. In addition to 0101 and 0401, binding was observed for 0301 (weak), 1101 (strong), 1301 (weak), and 1501 (strong), but not for 0801 (Table 15). In accordance with the prediction, the mutations disrupted MHC II binding. One exception was mutation Lys95Glu, which was predicted to reduce binding to 0101 and 0801, but instead resulted in stronger binding (or remained as a strong binder) across all eight alleles. The mutations significantly decreased binding to 0101: Tyr93His, Ile99Gln, and Tyr93His/Lys95Glu/Ile99Gln transformed a strong binder into a weak or non-binder, and Tyr93His/Lys95Glu turned a strong binder into a moderate one. All mutations and their combinations reduced the binding for 0301 from weak to non-binders. Similarly, all mutations but Tyr93His/Ile99Gln resulted in significant decreases in binding to 1101 (strong to weak/non-binder), and all mutants except Tyr93His/Lys95Glu lessened the binding for 1301. Lastly, only Ile99Gln and Tyr93His/Lys95Glu/Ile99Gln eliminated binding to 1501 (strong to weak/non-binder). Mutations Ile99Gln, Tyr93His/Ile99Gln, and Tyr93His/Lys95Glu slightly increased binding affinity for 0701 by turning a non-binder C4WT into a weak binder. Overall, mutations Tyr93His, Ile99Gln, Tyr93His/Lys95Glu, Lys95Glu/Ile99Gln, and Tyr93His/Lys95Glu/Ile99Gln were the most productive across all eight alleles. Tyr93His/Lys95Glu/Ile99Gln was particularly good, as it eliminated binding to all eight alleles.

**TABLE 15**

| Peptide | IC₅₀, µM (No. of predicted eptitopes) | | | | | | | |
|---|---|---|---|---|---|---|---|---|
| | 0101 | 0301 | 0401 | 0701 | 0801 | 1101 | 1301 | 1501 |
| C4WT¹ | <0.01 (1*) | 31.40 (0*) | <0.01 (1*) | 92.86 (0*) | 62.54 (1) | 1.69 (0) | 78.47 (0*) | <0.01 (0) |
| Y93H² | 102.80 (0*) | >250 (0*) | >250 (1) | 181.90 (0*) | >250 (0*) | >250 (0*) | >250 (0*) | <0.01 (0) |
| K95E³ | <0.01 (0) | 1.90 (0) | <0.01 (1*) | 4.81 (0) | 12.24 (0*) | 6.32 (0) | 25.69 (0*) | <0.01 (0) |
| I99Q⁴ | 31.74 (0*) | >250 (0*) | 6.16 (0) | 93.12 (0*) | >250 (1) | 24.32 (0*) | >250 (0*) | 66.06 (0*) |
| 93_99⁵ | <0.01 (0) | >250 (0*) | <0.01 (0) | 60.49 (0*) | >250 (0*) | >250 (0*) | >250 (0*) | <0.01 (0) |
| 93_95⁶ | 9.98 (0) | 110.20 (0*) | 25.52 (1) | 24.94 (0*) | 30.93 (0*) | 25.02 (0*) | 18.92 (0*) | 0.06 (0) |
| 95-99⁷ | <0.01 (0) | >250 (0*) | 25.77 (0*) | 103.70 (0*) | >250 (0*) | 25.43 (0*) | >250 (0*) | <0.01 (0) |
| 939599⁸ | >250 (0*) | >250 (0*) | >250 (0*) | 179.30 (0*) | >250 (0*) | >250 (0*) | >250 (0*) | >250 (0*) |

| | | | | | | | | |
|---|---|---|---|---|---|---|---|---|
| ¹DYVKAGQIIGWSGSTGY (SEQ ID NO:151). ²DHVKAGQIIGWSGSTGY (SEQ ID NO:152). ³DYVEAGQIIGWSGSTGY (SEQ ID NO:153). ⁴DYVKAGQQIGWSGSTGY (SEQ ID NO:154). ⁵DHVKAGQQIGWSGSTGY (SEQ ID NO:155). ⁶DHVEAGQIIGWSGSTGY (SEQ ID NO:156). ⁷DYVEAGQQIGWSGSTGY (SEQ ID NO:157). ⁸DHVEAGQQIGWSGSTGY (SEQ ID NO:158). *Positive correlation between predicted binders and experimentally observed binders. Binding thresholds set to 5% for predictions and 10 µM for experiments. | | | | | | | | |

The cluster five wild-type (C5WT) epitope was predicted to bind all eight alleles, with multiple epitopes for all except DRB1*0301 and 0801. Binding was experimentally observed in all but 0301 and 1301, with 0701 and 0801 measuring as weak binders (Table 16). The most deimmunizing mutations were Arg118Thr/Ser122Asp/Ser124Gly and Asn121Gly/Ser122Asp/Ser124Gly, as they each significantly reduced binding affinity to six alleles. For the 0301 allele, the IC₅₀ values of the mutants decreased slightly, but the peptides remained as non-binders. This result agreed with the EpiSweep prediction, in which the mutations disrupted binding for all eight alleles. Asn121Gly diminished binding to four alleles, while Ser124Gly and Asn121Gly/Ser122Asp reduced binding to three. Asn121Gly/Ser122Asp also slightly increased the binding to 0301 (non-binder to weak binder). Similarly, Arg118Thr and R118T/S122D lessened the affinity to two alleles, but showed an increase in binding to 0301 (non-binder to weak binder). The observed increase in binding affinity for 0301 allele in mutation combinations could be explained by the fact that Ser122Asp was predicted to introduce one epitope for the allele. In fact, it was observed that Ser122Asp changed a non-binder peptide C5WT into a moderate binder.

**TABLE 16**

| Peptide | IC₅₀, µM (No. of predicted eptitopes) | | | | | | | |
|---|---|---|---|---|---|---|---|---|
| | 0101 | 0301 | 0401 | 0701 | 0801 | 1101 | 1301 | 1501 |
| C5WT¹ | 0.15 (2*) | >250 (1) | 0.05 (3*) | 15.23 (2 | 22.01 (1) | <0.01 (3*) | >250 (3) | <0.01 (3*) |
| R118T² | <0.01 (2*) | 68.39 (1) | <0.01 (3*) | 23.14 (2) | 57.70 (1) | <0.01 (3*) | >250 (3) | <0.01 (3*) |
| N121G³ | <0.01 (2*) | >250 (1) | 6.13 (2*) | 17.40 (2) | >250 (1) | 6.41 (2*) | >250 (2) | <0.01 (3*) |
| S122D⁴ | <0.01 (1*) | 9.42 (2*) | <0.01 (2*) | >250 (0*) | 150.00 (1) | <0.01 (1*) | >250 (0*) | <0.01 (1*) |
| S124G⁵ | <0.01 (2*) | >250 (1) | 0.04 (2*) | 150.70 (2) | >250 (1) | 0.30 (2*) | >250 (3) | <0.01 (3*) |
| 18_22⁶ | <0.01 (1*) | 28.16 (1) | <0.01 (2*) | 110.60 (0*) | >250 (0*) | <0.01 (1*) | >250 (0*) | <0.01 (0) |
| 182224⁷ | 7.43 (1*) | 132.90 (0*) | 7.52 (2*) | >250 (0*) | >250 (0*) | 14.13 (1) | >250 (0*) | 0.42 (0) |
| 21_22⁸ | <0.01 (1*) | 77.42 (1) | <0.01 (2*) | >250 (0*) | 210.10 (0*) | 28.54 (1) | >250 (0*) | <0.01 (0) |
| 212224⁹ | 0.18 (1*) | 116.40 (0*) | 5.55 (2*) | >250 (0*) | >250 (0*) | 13.45 (1) | >250 (0*) | 24.20 (0*) |

| | | | | | | | | |
|---|---|---|---|---|---|---|---|---|
| ¹HLHFQRMVNSFSNPTAQ (SEQ ID NO:159). ²HLHFQTMVNSFSNPTAQ (SEQ ID NO:160). ³HLHFQRMVGSFSNPTAQ (SEQ ID NO:161). ⁴HLHFQRMVNDFSNPTAQ (SEQ ID NO:162). ⁵HLHFQRMVNSFGNPTAQ (SEQ ID NO:163). ⁶HLHFQTMVNDFSNPTAQ (SEQ ID NO:164). ⁷HLHFQTMVNDFGNPTAQ (SEQ ID NO:165). ⁸HLHFQRMVGDFSNPTAQ (SEQ ID NO:166). ⁹HLHFQRMVGDFGNPTAQ (SEQ ID NO:167). *Positive correlation between predicted binders and experimentally observed binders. Binding thresholds set to 5% for predictions and 10 µM for experiments. | | | | | | | | |

To compare immunogenicity between the full-length designs, the aggregate epitope score for each design was calculated (Figure 3). This analysis showed that the wild-type lysostaphin catalytic domain had a total of 26 binding interactions (or epitopes): 14 strong, 1 moderate, and 11 weak. The enzyme also had 14 non-binding interactions. In comparison, 18 of the designs had an epitope score lower than the wild-type, six had the same score, and only four had epitope scores that exceeded the wild-type (by maximum of two epitopes). All but three of the designs also had a higher number of non-binding interactions as compared to the wild-type. The minimum number of epitopes was 18, and it was present in three of the variants: Flex 4* (5 mutations), Flex 11* (7 mutations), and Flex 13* (6 mutations). Furthermore, 18 of the designs showed a reduction in the number of strong binders, as compared to the wild-type.

A strong negative correlation was noted between the number of mutations and the number of experimentally observed strong binders (Pearson coefficient -0.69). Similarly, a positive correlation was observed between the epitope score and the number of strong binders (Pearson coefficient 0.52). At the same time, no correlation was found between the mutational load/epitope score and the total number of binders. This result indicated that the algorithm was not only reducing binding, but was also primarily targeting the strong epitopes.

In general, more deimmunized plans were found among flexible than rigid backbone designs. On average, there were fewer strong, moderate, weak, and total binding interactions found in flexible than rigid plans. This trend may be explained in part by the fact that most rigid designs were reverted and missing one mutation. The lowest number of strong binders, eight, was observed in Flex 11* and Rigid 5*. As expected, the most aggressive plan, Flex 9, showed a significant decrease in immunogenicity, with a total of only 22 binding interactions: 9 strong, 6 moderate, and 7 weak.

### In Vitro Analysis of Flex 5 and Flex 9 Variants

Variants Flex 5 and Flex 9 were expressed, purified, and characterized in biological duplicate. As an additional control, wild-type LST was obtained from a commercial supplier and analyzed in parallel. The apparent melting temperatures of both variants were consistent with values obtained during preliminary testing, but their specific rates of bacterial lysis were found to be somewhat higher upon more rigorous analysis (Table 17). Importantly, the deimmunized variants were equivalent to or better than commercially sourced LST in both assays. The enzymes' antibacterial activity was further quantified by assessing minimal inhibitory concentration (MIC) toward four strains of *S. aureus.* The Flex 5 MIC for strain SA113 was equivalent to that of wild-type and commercial LST, and it was within a single 2-fold serial dilution for three clinical isolates, including MRSA strain 3425-3. Variant Flex 9 also retained good bactericidal/bacteriostatic activity, preventing outgrowth of all four strains at 200 ng/ml (~7 nM) or less. Given the fact that the LST^{CAT} variants encoded four or eight mutations, respectively, their high levels of anti-staphylococcal activity were striking.

**TABLE 17**

| | %WT Lytic Rate | Tm (°C) | MIC (µg/mL) | | | |
|---|---|---|---|---|---|---|
| Design ID | | | Strain SA113 | Strain 6445 | Strain 3425-1 | Strain 3425-3 |
| Wild-type | 100±30 | 59.0 ±0.4 | 0.02 ±0.00 | 0.03 ±0.01 | 0.04 ±0.02 | 0.03 ±0.02 |
| Commercial | 60±20 | 47.3 ±0.4 | 0.03 ±0.00 | 0.03 ±0.00 | 0.04 ±0.01 | 0.03 ±0.00 |
| Flex 5 | 70±30 | 55.8 ±0.2 | 0.02 ±0.01 | 0.06 ±0.04 | 0.11 ±0.09 | 0.05 ±0.03 |
| Flex 9 | 60±20 | 52.8 ±0.3 | 0.04 ±0.01 | 0.13 ±0.07 | 0.2 ±0.1 | 0.2 ±0.1 |

| | | | | | | |
|---|---|---|---|---|---|---|
| * Errors are standard deviation from a minimum of biological duplicates measured in triplicate | | | | | | |

### In Vivo Efficacy and Immunogenicity of Flex 5 and Flex 9 Variants

To assess antibacterial activities in a more clinically relevant fashion, a murine lung infection model was employed, which uses an *S. aureus* clinical isolate. Mice were infected with live bacteria via oropharyngeal aspiration, and one hour later they were treated via the same route with a solution containing 2.5 µg of wild-type LST, variant Flex 5, or variant Flex 9. Twenty-four hours post-infection, mice were sacrificed, lungs were harvested, and viable bacterial counts were determined by plating serial dilutions of lung homogenate. All three enzymes yielded a statistically significant 10-fold reduction in bacterial burden relative to a saline buffer control (one-way ANOVA P=0.007, Tukey post test), but there was no significant difference between the three treatments (Figure 4A). Thus, the deimmunized candidates retained wild-type efficacy in the infected and inflamed lung environment.

*In vivo* immunogenicity was evaluated using NOD/SCID/γ_{c}^{-/-} mice that had been surgically humanized with human immune cells, liver tissue, and thymus tissue (HUMI mice). Following transplantation of human tissues at six weeks of age, HUMI mice were allowed to mature and develop circulating repertoires of human B and T cells. At 14 weeks post-transplantation, mice were divided into three groups of four each and immunized subcutaneously with 100 µg of wild-type LST, Flex 5, or Flex 9 in adjuvant. Thirteen days post-immunization, mice were sacrificed, splenocytes were harvested and pooled for each group, and the pooled cells were subjected to *ex vivo* restimulation with their cognate proteins. Cell proliferation was measured by tritiated thymidine uptake at 72 hours. The stimulation index (protein vs. DMSO proliferative response) was less than 2-fold for wild-type LST (Figure 4B), but it bears noting that T cells from humanized mice are widely known to exhibit impaired function. In particular, humanized mouse splenocytes have been shown to exhibit poor *ex vivo* proliferative response even in the presence of potent stimulatory agents such as phytohaemagglutinin, ionomycin-PMA, and anti-CD3/anti-CD28 antibody cocktails (Watanabe, et al. (2009) Internatl. Immunol. 21:843-858). Moreover, following two to three *in vivo* immunizations with the powerful antigen keyhole limpet hemocyanin in complete Freund's adjuvant (CFA), restimulated humanized mouse splenocytes fail to produce IFN-γ or IL-4 (Watanabe, et al. (2009) Internatl. Immunol. 21:843-858) and exhibit only a 2- to 6-fold stimulation index *ex vivo* (Tonomura, et al. (2008) Blood 111:4293-6). Thus, the significant (P=0.0005, two way ANOVA) 1.6-fold stimulation index of the wild-type LST splenocytes is a reasonable indicator of an antigen specific immune response, particularly given the fact that the mice of the current study received but one immunization. Relative to the wild-type immunized group, pooled splenocytes from Flex 5 and Flex 9 immunized mice exhibited significantly reduced proliferation (Figure 4B). After background subtraction, Flex 5 pooled cells showed a 50% reduced response and Flex 9 pooled cells a 65% reduced response.

In addition to inherent immunogenicity in the context of a naive immune system, the extent to which a deimmunized protein might evade an established memory response directed against the native sequence was also considered. Due to the long time-frame of such a study and the short lifespan of HUMI mice, the memory response in transgenic DR4 mice was assessed. This homozygous strain has an intact murine immune system, with the exception that they bear a chimeric class II MHC based on the peptide binding domains from human HLA DRA and DRB1*0401 (Ito, et al. (1996) J. Exp. Med. 183:2635-44). This stable transgenic model has a normal, healthy lifespan enabling extended studies, yet its antigen presenting cells exhibit human peptide binding specificity. Ten DR4 mice were immunized and repeatedly boosted with subcutaneous injections of wild-type LST. Nineteen weeks after the final boost, they were divided into two groups of five each such that each group exhibited similar average antibody titers. Mice were then rechallenged with either 100 µg wild-type LST or 100 µg variant Flex 5. Thirteen days later, splenocytes were harvested, pooled for each group, and subjected to *ex vivo* restimulation with the cognate protein from the final rechallenge. Similar to the results in the HUMI mice, *ex vivo* restimulation of DR4 splenocytes with wild-type LST yielded a 1.8-fold stimulation index (Figure 4C). It bears noting that similarly small stimulation indices in DR2, DR3, and DQ8 transgenic mice have been shown to correlate with antigen specific antibody production and to be indicative of antigen specific immune responses (Depil, et al. (2006) Vaccine 24:2225-9). Thus, the significant (P=0.0002, two way ANOVA) 1.8-fold stimulation index seen here was a reasonable indicator of an anti-drug immune response. In contrast to wild-type LST rechallenged mice, proliferation of pooled splenocytes from the Flex 5 challenge group was at or below background levels (Figure 4C). Thus, immune cells primed to recognize wild-type LST exhibited reduced activity upon rechallenge with Flex 5, indicating that the deimmunized variant effectively evaded the memory response directed against the wild-type enzyme.

### Example 2: Deimmunization of Lysostaphin Catalytic and Cell Wall Binding Domains Against HLA Allele DRB1*0401

### Overview

This analysis was carried out to demonstrate that depletion of putative T cell epitopes in LST would mitigate the anti-drug antibody response and consequently enhance therapeutic efficacy. Epitope depleted variants were developed using two distinct computationally-guided strategies: structure-based design of individual deimmunized variants followed by empirical improvement (designated "opt" variants) and structure-based design and screening of combinatorial libraries enriched in functionally deimmunized members (designated "lib" variants). Humanized HLA-transgenic mice were used to assess the efficiency with which each method deleted putative immunogenic epitopes and thereby prevented formation of anti-LST antibodies *in vivo.* Subsequently, a recurrent bacteremia model was used to gauge the extent to which LST deimmunization enabled clearance of systemic *S*. *aureus* infections. This systematic comparison between deimmunized variants and their wild-type counterpart provides direct experimental evidence of the clinically relevant connections between putative T cell epitopes, *in vivo* immunogenicity, and therapeutic efficacy.

As a proof of concept, focus was placed on allele DRB1*0401 (hereafter DR4), which is highly prevalent in North American and European populations. At a 5% threshold (*i.e.,* peptides among the top 5% of predicted binders), the ProPred analysis tool (Singh & Raghava (2001) Bioinformatics 17:1236-7) predicted 16 DR4 restricted T cell epitopes within wild-type LST (LST^{WT} epitope score=16). The peptide epitopes were arrayed as both overlapping clusters and isolated nonamers distributed throughout the protein's sequence and structure (Figure 5). Interestingly, ProPred predicted more epitopes for DR4 than for any of the seven other representative DRB1 alleles: 0101, 0301, 0701, 0801, 1101, 1301, and 1501. Considering any single allele, therefore, the DR4 model represented a high bar for global protein redesign.

### Materials and Methods

*Materials.* Primers were ordered with standard desalting from IDT Technologies (Coralville, IA). Restriction enzymes and Phusion DNA polymerase for molecular cloning were purchased from New England Biolabs (Ipswich, MA). All other reagents and supplies were from VWR Scientific (Philadelphia, PA), unless specifically noted.

*P. pastoris* expression vector pPIC9 and *P. pastoris* strain GS115(his4) were purchased from Invitrogen (Grand Island, NY). *E. coli* DH5□ [F- Φ80*lac*ZΔM15 Δ(*lac*ZYA-*arg*F) U169 *rec*A1 *end*A1 *hsd*R17 (rK-, mK+) *pho*A *sup*E44 λ- *thi*-1 *gyr*A96 *rel*A1], *S. aureus* strain SA113, and MRSA strain USA400 were from the American Type Culture Collection (Manassas, VA).

*LST Homology Models.* As the crystal structure of lysostaphin was not available, homology models were constructed for the two domains. Three template structures from LytM were selected for the catalytic domain (2B0P:A, 2B44:A and 1QWY:A) (Firczuk, et al. (2005) J. Mol. Biol. 354:578-90; Odintsov, et al. (2004) J. Mol. Biol. 335:775-85). The three template structures share significant sequence identity (95%~97%), yet they have highly mobile loops (Firczuk, et al. (2005) J. Mol. Biol. 354:578-90) around conserved active sites. The highest sequence identity against the lysostaphin catalytic domain was 46.7%, which is sufficient to build quality model structures (Baker & Andrej (2001) Science 294:93-96). Initial homology models were built using MODELLER. A template-less region (²⁴PLGINGG³⁰; SEQ ID NO:168) was modeled using a loop modeling method, FREAD (Choi & Deane (2010) Proteins 78:1431-1440), which selected a sequence-similar loop from 1GMN:A (¹⁸⁰PRGEEGG¹⁸⁶; SEQ ID NO:169) (Lietha, et al. (2001) EMBO J. 20:5543-5555). The cell wall binding domain was constructed using a single template structure (1R77:A, cell wall targeting domain structure of ALE-1, a lysostaphin homolog) with a high sequence identity (83.5%) (Lu, et al. (2006) J. Biol. Chem. 281:549-558). The best models were selected in terms of the DOPE statistical potential function score (Shen & Sali (2006) Protein Sci. 15:2507-2524). The catalytic domain models were minimized against AMBER99sb with GB/SA in order to relax the predicted loop (Hornak, et al. (2006) Proteins 65:712-725; Still, et al. (1990) J. Am. Chem. Soc. 112:6127-6129).

*Preprocessing of Mutation Choices.* For each domain, three iterations of PSI-BLAST (Altschul, et al. (1997) Nucleic Acids Res. 25:3389-3402) were run against the non-redundant database to find homologs. Multiple sequence alignments were constructed for the identified sequences, and processed to identify those that were not too gappy (at most 25%), and were sufficiently similar to LST (at least 35%) but sufficiently different from each other (at most 90% identical). A set of 114 representative catalytic domain homologs and 23 representative cell wall binding domain sequences remained. Amino acids were identified at each position in each multiple sequence alignment and subsequently used as possible mutations for design. Particular positions and mutations deemed functionally important were not allowed to mutate (positions 32, 36, 82, 113, 115, 117, 118, 119, 125, 126, 127 were locked down; specific mutations Y33T, F38A, F38G, M39A, I41R, S124Y and R118T were disallowed). A second filtering step kept only those mutations predicted to delete at least one epitope, as determined using ProPred at a 5% threshold. To avoid presumed deleterious effects, mutations to and from proline and cysteine were also excluded.

The mutational choices were expanded for library design, since screening allows for riskier substitutions. In particular, Chou-Fasman (Chou & Fasman (1974) Biochem. 13:222-45) propensities were used to identify residues likely to be acceptable in the wild-type secondary structure environment, according to a stringent threshold of 1.5. In addition, since the library was constructed with degenerate oligonucleotides that can incorporate additional amino acids beyond the desired ones, these additions were allowed, as long as the desired to undesired ratio within a degenerate oligonucleotide remained above 2:3.

*Structure-Based Design of Individual Variants.* In order to generate epitope depleted designs, the structure-based deimmunization method EpiSweep was applied to each domain as described in Example 1. OSPREY (ver. 2.0) (Chen, et al. (2009) Proc. Natl. Acad. Sci. USA 106:7678-7678) was used to assess one- and two-body energy terms for possible rotamers (Lovell, et al. (2000) Proteins 40:389-408) for the mutational choices, according to the AMBER force field (Pearlman, et al. (1995) Comput. Phys. Commun. 91:1-41) and a reference energy (Lippow & Tidor (2007) Curr. Opin. Biotechnol. 18:305-311). ProPred (Singh & Raghava (2001) Bioinformatics 17:1236-7) at a 5% threshold was used to assess DR4 epitope (HLA Allele DRB1*0401) content, characterizing each 9mer as either a binder or a non-binder. It was found that at least seven mutations were required to deplete all the predicted DR4 epitopes in the catalytic domain; six were required to fully deplete the cell wall binding domain. A set of 20 energy optimal and near-optimal fully depleted designs were identified for each domain via the EpiSweep optimization algorithm. The most energy optimal catalytic domain design contained a mutation at Ser124, which analysis has identified as being potentially detrimental. However, the alternative double mutation Asn121Gly and Ser122Gly is predicted to remove the same epitope, yet these two mutations manifest higher experimentally determined activity than LST^{WT}. The energy difference between the most energy optimal design (containing a detrimental Ser124 mutation) and the alternative double mutation design was marginal (-294.94 and -293.9). Thus, a design (Opt1) was selected so as to possess the most energy optimal design for the cell wall binding domain and the alternative double mutation in the C-terminal portion of the catalytic domain (Asn121Gly and Ser122Gly).

*Structure-Based Design of Deimmunized Libraries.* In order to design combinatorial libraries enriched in stable, deimmunized variants, a method called EpiSOCoM was developed, which augments the SOCoM structure-based library design approach with epitope analysis, and employs a sweep-based Pareto optimization algorithm (Parker, et al. (2013) J. Comput. Biol. 20:152-65) to simultaneously optimize both energy and epitope content. Given a set of possible positions at which to mutate and possible amino acids to incorporate at those positions, along with a desired library size, EpiSOCoM selects a subset of the positions and subsets of the substitutions at those positions. It thereby specifies the construction of a library composed of all combinations of the substitutions and corresponding wild-type residues. EpiSOCoM optimizes a library for the average energy score and the average epitope score over its constituent variants, so that in general variants will be "good". Its Pareto optimization algorithm identifies all library designs (positions and substitutions) making undominated trade-offs between the two scores, in that no other library design is better for both.

In order to enable rapid assessment of structure-based energies without explicit rotameric modeling of each variant, SOCoM employs a Cluster Expansion (Grigoryan, et al. (2009) Nature 458:859-64; Grigoryan, et al. (2006) PLoS Comput. Biol. 2:e63) (CE) technique that expresses structure-based properties in terms of a protein-specific function of amino acid sequence. Here, Rosetta (Rohl, et al. (2004) Methods Enzymol. 383:66-93) was employed in a preprocessing step to model and evaluate energies of random LST variants based on the allowed mutational choices. These structural training sets allowed CE to express the energy Ψ for a possible LST variant *S* via a sum over position-specific one- and two-body sequence potentials: $Ψ \left(S\right) = {\sum }_{i} {ψ}_{i} \left({a}_{i}\right) + {\sum }_{i , j} {ψ}_{i , j} \left({a}_{i}, {a}_{j}\right)$ (1) where the sums involve amino acid *aᵢ* at position *i* and *aⱼ* at position j. A total of 9000 catalytic domain and 6000 cell wall binding domain models were used to train CE models, following the CE guidelines. These were then able to make accurate predictions for the energies of new random variants in a non-overlapping testing set (of size ~20% that of the training set), achieving correlations between the CE sequence potential and Rosetta energies of 0.8 for the catalytic domain and 0.9 for the cell wall binding domain. This modeling step thus enables fast, accurate prediction of energies within the "inner loop" of design optimization.

In order to assess a library without enumerating all its variants (not tractable when optimizing over the massive design space of possible libraries), SOCoM employs library-averaged position-specific scores. For example, if the set {Arg, Lys} were to be incorporated at one position, then the energetic contribution of that position, averaged over all variants the library, would be the average of the Arg energy and the Lys energy; similarly the pair-wise contribution from that position and another incorporating {Asp, Glu}, averaged over the library, would be the average of Arg:Asp, Arg:Glu, Lys:Asp, and Lys:Glu. Thus, given the allowed mutations, SOCoM precomputes *ψ̅ᵢ*, the average energetic contributions of possible subsets of amino acids that could be chosen at a position *i,* and *ψ̅_{i,j}*, the average for pair of positions *i* and *j*. It then evaluates the average energy Ψ̅ over a whole library ***T*** with an equation analogous to that for a single variant: $\overline{Ψ} \left(T\right) = {\sum }_{i} {\overline{ψ}}_{i} \left({T}_{i}\right) + {\sum }_{i , j} {\overline{ψ}}_{i , j} \left({T}_{i}, {T}_{j}\right)$ (2) where the sums now involve sets of amino acids *Tᵢ* at position *i* and *Tⱼ* at position j. Thus assessment of a library within the optimization is as efficient as assessment of a single variant.

To develop EpiSOCoM, it was also necessary to "lift" the epitope score to library-averaged contributions, in a manner analogous to SOCoM's treatment of energy scores. If amino acids {*Tᵢ*, *Tᵢ₊₁,..., Tᵢ₊₈*} were to be incorporated at the nine contiguous positions starting at i, then the average epitope score contribution *e̅* from the various 9mer combinations of amino acids is calculated as: ${\overline{e}}_{i} = \frac{{\sum }_{{a}_{i} ∈ {T}_{i} , {a}_{i + 1} ∈ {T}_{i + 1 ,} … , {a}_{i + 8} ∈ {T}_{i + 8}} e \left({a}_{i}{a}_{i + 1}…{a}_{i + 8}\right)}{\left|{T}_{i}\right| . \left|{T}_{i + 1}\right| … \left|{T}_{i + 2}\right|}$ (3) where the sum is over each combination of amino acids, one from each set, and the function *e*(·) gives the epitope score of the 9mer. Then the average epitope score, Ξ, of the library is simply the sum over all 9mers: $Ξ = {\sum }_{i = 1}^{n - 8} {\overline{e}}_{i}$ (4)

SOCoM uses an integer linear programming formulation to choose an optimal set of positions and sets of amino acids so as to optimize Eq. 2 subject to library size constraints. With EpiSOCoM, there are two objectives, energy (Eq. 2) and epitope score (Eq. 4). Since there is no *a priori* means to determine the best balance between these incommensurate properties, EpiSOCoM generates all Pareto optimal designs representing the best balance, enabling subsequent characterization of the trade offs and selection of suitable designs. To identify Pareto optimal designs, it employs a sweep algorithm based on that of EpiSweep. At each step in the sweep, average library energy is optimized (Eq. 2) according to a constraint on the average epitope score (Eq. 4). The constraint is successively tightened, so that each library must have a better epitope score (and thus worse energy) than the previous one. The Pareto optimization is implemented as an iterative layer over a constrained version of SOCoM, which in turn uses the IBM CPLEX integer programming solver to optimize each design.

*Protein Expression, Purification and Characterization.* LST and its derivatives were secreted from *P. pastoris.* Briefly, recombinant *Pichia* strains were cultured in a 2.5 L bioreactor (Applicon Biotechnology), and the proteins were captured from the supernatant by polyethylene glycol-6000 (PEG-6000) precipitation and purified to homogeneity by SP SEPHAROSE F.F. cation exchange chromatography. Endotoxin was removed from the protein preparation by TRITON-X114 extraction (Liu, et al. (1997) Clin. Biochem. 30:455-63). Protein expression levels were estimated by densitometry analysis of SDS-PAGE gels. The activities of proteins were assessed by determination of minimal inhibitory concentrations (MIC) against *S. aureus* strain SA113, and are reported as a normalized percentage relative to the MIC dilution determined for LST^{WT} (*i.e.,* 50% activity is 2-fold higher MIC relative to wild-type, and 25% activity is 4-fold higher MIC relative to wild type).

*Library Construction and Screening.* LST libraries were constructed by splice overlapping PCR with the primers shown in Table 18.

**TABLE 18**

| Mutations | Primer | Sequence (5'->3') | SEQ ID NO: |
|---|---|---|---|
| Library A | | | |
| Y33NDY, F38SF, T43KT | Forward | GGTATGCACDATGGTGTTGACTTCTYTATGAA CATCGGTAMGCCAGTCAAG | 170 |
| | Reverse | CTTGACTGGCKTACCGATGTTCATARAGAAGT CAACACCATHGTGCATACC | 171 |
| I70KRI, V75DEVV, W79TRSW, S84SRG | Forward | GGTTTGADAGAGAACGACGGTGWSCACAGACA AWSGTACATGCACTTGVGTAAGTAC | 172 |
| | Reverse | GTACTTACBCAAGTGCATGTACSWTTGTCTGT GSWCACCGTCGTTCTCTHTCAAACC | 173 |
| V120DV, S122TTRSA AGGSSWC | Forward | CACTTCCAAAGAATGGWTAACDSKTTCTCCAA CTCCGCT | 174 |
| | Reverse | AGCGGAGTTGGAGAAMSHGTTAWCCATTCTTT GGAAGTG | 175 |

| Library B | | | |
|---|---|---|---|
| S166TTRS, A169AG | Forward | GGTACCTTGTACAAGASMGAGTCCGSCTCCTT CACCCCAAAC | 176 |
| | Reverse | GTTTGGGGTGAAGGAGSCGGACTCKSTCTTGT ACAAGGTACC | 177 |
| S191SG, V193EDGGV V, I200TI | Forward | TCCATGCCACAARGCGGTGDMTTGAAGGCTGG TCAAACCAYTCACTACGACGAG | 178 |
| | Reverse | CTCGTCGTAGTGARTGGTTTGACCAGCCTTCA AKHCACCGCYTTGTGGCATGGA | 179 |
| N219NDY, Q222QE, N23 6KNQHED | Forward | GTDATTCCGGTSAGAGAATCTACTTGCCAGTC AGAACCTGGAACAAGTCCACCVAWAC | 180 |
| | Reverse | GTWTBGGTGGACTTGTTCCAGGTTCTGACTGG CAAGTAGATTCTCTSACCGGAATHAC | 181 |

| Library C | | | |
|---|---|---|---|
| Y33NDY, M39RMRLGV, T43KT | Forward | GGTGGTATGCACDATGGTGTTGACTTCTTTVK GAACATCGGTAMGCCAGTCAAGGCT | 182 |
| | Reverse | AGCCTTGACTGGCKTACCGATGTTCMBAAAGA AGTCAACACCATHGTGCATACCACC | 183 |
| W79TRSW, S84SRG | Forward | GGTGAGCACAGACAAWSGTACATGCACTTGVG TAAGTACAACGTCAAG | 184 |
| | Reverse | CTTGACGTTGTACTTACBCAAGTGCATGTACS WTTGTCTGTGCTCACC | 185 |
| S191SG, V193DGV | Forward | AGATCCATGCCACAARGCGGTGDCTTGAAGGC TGGTCAA | 186 |
| | Reverse | TTGACCAGCCTTCAAGHCACCGCYTTGTGGCA TGGATCT | 187 |
| N23 6KNQHED | Reverse | ATCGGAATTCTTACTTGATGGTACCCCACAAG ACACCCAAGGTWTBGGTGGACTTGTTC | 188 |

| Library D | | | |
|---|---|---|---|
| Y33NY, G34DG, T43KT, V45VL | Forward | GGTATGCACWATGRCGTTGACTTCTTTATGAA CATCGGTAMGCCAKTAAAGGCTATC | 189 |
| | Reverse | GATAGCCTTTAMTGGCKTACCGATGTTCATAA AGAAGTCAACGYCATWGTGCATACC | 190 |
| Y33NDY, N40KNTT, T43KTR | Forward | GGTATGCACDATGGTGTTGACTTCTTTATGAM WATCGGTAVACCAGTCAAG | 191 |
| | Reverse | CTTGACTGGTBTACCGATWKTCATAAAGAAGT CAACACCATHGTGCATACC | 192 |
| V35DEVV, T43TKR, V45EDGGVV | Forward | CACTATGGTGWSGACTTCTTTATGAACATCGG TAVACCAGDMAAGGCTATC | 193 |
| | Reverse | GATAGCCTTKHCTGGTBTACCGATGTTCATAA AGAAGTCSWCACCATAGTG | 194 |
| F116SCF | Forward | CCACACTTGCAC**TBC**CAAAGAATGGAT | 195 |
| | Reverse | ATCCATTCTTTG**GVA**GTGCAAGTGTGG | 196 |
| R186TR, S191AS | Forward | GGTCCATTCASGTCCATGCCACAAKCTGGTGT CTTG | 197 |
| | Reverse | CAAGACACCAGMTTGTGGCATGGACSTGAATG GACC | 198 |
| S191SG, V193DGV | Forward | TCCATGCCACAARGCGGTGDCTTGAAGGCTGG T | 199 |
| | Reverse | ACCAGCCTTCAAGHCACCGCYTTGTGGCATGG A | 200 |
| R186TTSRP RRAAGGSSC W, V193KKRRI MQQRRLLEE GGVV | Forward | GGTCCATTCNSSTCCATGCCACAAAGCGGTVD RTTGAAGGCT | 201 |
| | Reverse | AGCCTTCAAYHBACCGCTTTGTGGCATGGASS NGAATGGACC | 202 |

| Library E | | | |
|---|---|---|---|
| N28NS, I41NI, T43KT | Forward | GTATCARCGGTGGTATGCACTATGGCGTTGAC TTCTTTATGACCAWCGGTAMGCCAGT | 203 |
| | Reverse | ACTGGCKTACCGWTGGTCATAAAGAAGTCAAC GCCATAGTGCATACCACCGYTGATAC | 204 |
| | Forward | CACTATGRCGTTGACTTCTTTATGACCAWMGG TAMGCCAGTA | 205 |
| G34DG, I41KNII, T43KT | Revers e | TACTGGCKTACCKWTGGTCATAAAGAAGTCAA CGYCATAGTG | 206 |
| G34EDGG, T43KTR, I41KKRRIM EEGGVV | Forwar d | CACTATGRKGTTGACTTCTTTATGACCRDRGG TAVACCAGTA | 207 |
| | Reverse | TACTGGTBTACCYHYGGTCATAAAGAAGTCAA CMYCATAGTG | 208 |
| N28NK, G30EG, I41NI, T43KRT | Forward | GTATCAASGGTGRAATGCACTATGGCGTTGAC TTCTTTATGACCAWCGGTAVACCAGT | 209 |
| | Reverse | ACTGGTBTACCGWTGGTCATAAAGAAGTCAAC GCCATAGTGCATTYCACCSTTGATAC | 210 |
| N121NK, S124SSCW | Forward | AGAATGGAT**AAS**ACTTTC**TSS**AACTCCGCT | 211 |
| | Reverse | AGCGGAGTT**SSA**GAAAGT**STT**ATCCATTCT | 212 |
| T122TTIM, S124SSCW | Forward | AGAATGGATAACAYRTTCTSSAACTCCGCTGC T | 213 |
| | Reverse | AGCAGCGGAGTTSSAGAAYRTGTTATCCATTC T | 214 |

The PCR products were ligated into pPIC9 vector and transformed into DH5a. Constructs were sequence verified and transformed into *P. pastoris* by electroporation (Wu & Letchworth (2004) Biotechniques 36:152-4). Library construction and screening was implemented as an iterative directed evolution strategy. Active library members were identified using a moderate throughput plate halo formation assay. Briefly, *P. pastoris* transformants from each round of library construction were spread on YPM agar media (1% yeast extract, 2% peptone, 1% methanol, 1% agarose) and incubated at 30°C for 2 days. Indicating top agarose (0.5% yeast extract, 1% peptone, 1% NaCl, 0.1 OD₆₀₀ SA113, 1% low melting agarose) was poured onto the YPM yeast plates, and the plates were incubated at 37°C for 10 hours. Yeast clones expressing active enzymes were identified by their characteristic halo or zone of clearance. Approximately 10,000 clones were screened for each round. The genes encoding the 10 variants exhibiting the largest halos were PCR amplified from the genomically integrated cassette, subcloned back into pPIC9, sequenced, and retransformed into freshly prepared *P*. *pastoris* cells for functional validation by determination of MIC. The most deimmunized and functional variant was used as the starting point for the subsequent round of library construction and screening.

*In Vivo Studies.* The protocols for animal infection, treatment, and immunization were carried out to minimize animal suffering. C57Bl/6 mice were purchased from the Jackson Laboratory (Bar Harbor, ME). C57Bl/6 background Abb Knockout/Transgenic HLA-DR4 mice (B6.129S2-H2-Ab1^{tm1Gru} Tg(HLA-DRA/H2-Ea,HLA-DRB1*0401/H2-Eb)1Kito) were purchased from Taconic Farms (Germantown, NY).

*In Vivo Immunogenicity.* A 100 µl volume of 100 µg purified wild-type or variant enzyme in complete Freund's adjuvant (CFA) was injected subcutaneously in either DR4 (N=5 per group) or C57Bl/6 (N=4 per group) mice. Thirteen days following immunization, serum was collected and anti-LST IgG antibody titers (specific to wild-type or variant protein) were measured by ELISA. Briefly, wild-type or variant protein antigen was coated onto high binding ELISA plates, followed by blocking with BSA. Immune serum from mice was serially diluted into the coated plates, which were then probed using goat anti-mouse IgG-HRP conjugate (Santa Cruz Biotechnology, Dallas, TX) at a working concentration of 1:1000. Plates were subsequently developed using TMB Substrate (Santa Cruz Biotechnology) Reported titers were defined as the serum fold dilution yielding an absorbance of 1.5.

*In Vivo Efficacy.* Prior to bacterial challenge, the immune systems of DR4 mice (N=3 per group) were primed 3-times with weekly subcutaneous injections of 100 µg LST^{WT} or Lib5 variant in sterile phosphate buffered saline (PBS: 2.7 mM KCl, 1.5 mM KH₂PO₄, 8.9 mM Na₂HPO₄, 136.9 mM NaCl, pH 7.4). These immunizations and boosts contained no adjuvant. Anti-LST antibody titers were determined as described above. For the first cycle of infection and treatment, mice were challenged with intraperitoneal administration of 2x10⁸ CFU *Staphylococcus aureus* strain USA400 in a 3% suspension of porcine mucin, and one hour later were treated by intravenous tail vein administration of 500 µg of LST^{WT} or Lib5 variant in sterile PBS. Mice that were rescued by the enzyme treatment underwent subsequent infection and treatment cycles at weekly intervals. To compensate for development of innate murine antibacterial immunity following the first bacterial exposure, mice were challenged with 1x10⁹ CFU USA400 in the second and third cycles, but the treatments remained at 500 µg of the appropriate protein. As a control to verify the lethal bacterial dose in each cycle, one mouse was given a sham treatment of PBS.

### Structure-Based Design of Individual Variants

The EpiSweep algorithm (Parker, et al. (2013) J. Comput. Biol. 20:152-65) was used to optimize deimmunized variants, making the best trade-offs between predicted reduction in epitope content, as evaluated by ProPred, and predicted maintenance of protein stability, as evaluated by structure-based rotamer energy. Panels of 20 fully depleted designs (*i.e.,* DR4 epitope scores=0) were generated separately for both the catalytic and cell wall binding domains. A variant combining low energy designs from each domain, Opt1 (Table 19), was selected for experimental analysis and cloned into an optimized *Pichia pastoris* expression system. Unfortunately, this 14-mutation design failed to yield functional protein (Table 20).

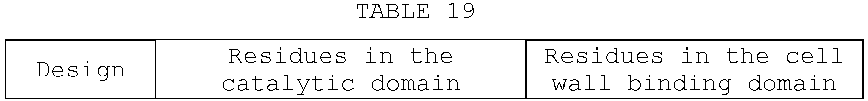

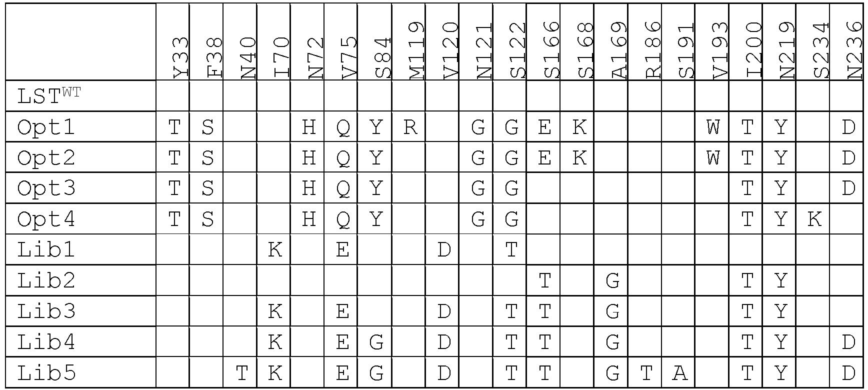

**TABLE 20**

| Design | Mutation Load | Epitope Score | Expression Level (%WT) | Activity (%WT) |
|---|---|---|---|---|
| LST^{WT} | 0 | 16 | 100 | 100 |
| Opt1 | 14 | 0 | ND^{b} | ND^{b} |
| Opt2 | 13 | 1 | ND^{b} | ND^{b} |
| Opt3 | 10 | 5 | 20 | ND^{a} |
| Opt4 | 10 | 5 | 10 | 0.25 |
| Lib1 | 4 | 13 | 80 | 100 |
| Lib2 | 4 | 12 | 80 | 100 |
| Lib3 | 8 | 8 | 60 | 50 |
| Lib4 | 10 | 6 | 50 | 50 |
| Lib5 | 13 | 3 | 50 | 50 |

| | | | | |
|---|---|---|---|---|
| ^{a} Activity measured as minimal inhibitory concentration (MIC), reported as the % fold dilution, relative to wild type, at which the MIC was achieved. ^{b} Not Detectable. | | | | |

The LST redesign work in Example 1 shows that single mutations could undermine otherwise stable and active deimmunized variants. Therefore, detrimental mutations in the Opt1 design were identified by systematically reverting mutations and mutation combinations. Analysis of isolated Opt1 mutations revealed that Met119Arg single-handedly abolished protein secretion, but reversion of this single mutation in design Opt2 (Table 19) failed to restore expression (Table 20). Ultimately, it was found that the three mutation combination Ser166Glu, Ser168Lys, and Val193Trp also undermined expression, and reversion to wild-type at these three sites as well as Met119 generated an expressible 10-mutation variant, Opt3 (Table 20).

Although Opt3 achieved reasonable expression levels (Table 20), purity analysis by SDS-PAGE revealed two bands: one at the expected 25 kDa mass and a second at approximately 30 kDa. Based on the results in Example 1, it was suspected that a latent N-linked glycosylation sequon in the C-terminal cell wall binding domain (²³²NKS²³⁴) had been activated. Therefore, the deimmunizing Asn236Asp mutation was exchanged with the Ser234Lys mutation, which deleted both the C-terminal epitope and the N-linked glycosylation sequon. The resulting 10-mutation variant, Opt4, bore only five predicted DR4 epitopes and expressed as a single 25 kDa band, albeit with lower yields than Opt3 (Table 20). Although the highly engineered Opt4 variant was produced in a folded and secretion competent state, it was subsequently found to possess only a small fraction of the wild type enzyme's antibacterial activity (Table 20).

### Structure-Based Deimmunization via Computational Library Design

In parallel to design of individual variants, an alternative combinatorial approach was pursued in which computational design was used to generate LST libraries predicted to be enriched in functional, deimmunized variants. The structure-based library design method SOCoM was augmented with epitope analysis in order to identify residue positions and mutations whose combinations yielded variants with low epitope scores, as evaluated by ProPred, along with good energies, as evaluated by a Cluster Expansion potential trained on Rosetta models. In the initial round, the designs were based on the wild-type reference, while in succeeding rounds the reference was shifted to the lead clone selected from the previous library screen.

Library A targeted nine sites in the catalytic domain, and the complementary Library B population targeted eight sites in the cell wall binding domain (Table 18). Although the deimmunized LST design space was massive, the initial libraries were constrained to fewer than 40,000 members so as to maintain some parity between library size and the screening capacity of agar plate halo formation assays. Approximately 10,000 clones were screened from both Libraries A and B, and 10 large-halo-forming colonies from each were sequenced and functionally validated. The most promising variant from Library A (clone Lib1) contained four mutations in the catalytic domain and exerted antibacterial activity equivalent to LST^{WT} (Tables 19 and 20). Likewise, the most promising variant from Library B (clone Lib2) possessed four mutations in the cell wall binding domain (Table 19) and also had wild-type antibacterial activity (Table 20).

The respective deimmunized domains of variants Lib1 and Lib2 were combined to produce variant Lib3, containing eight mutations (Table 19), a 50% reduction in predicted epitope content, and retention of 50% wild-type activity (Table 20). The structures of the two Lib3 domains were subsequently modeled and used as templates in another round of deimmunized library design. The resulting Library C targeted five sites in the catalytic domain and three sites in the cell wall binding domain (Table 19). Functional screening of 10,000 clones yielded variant Lib4, which deleted 10/16 DR4 epitopes. Relative to its Lib3 starting template, Lib4 contained one additional mutation each in the catalytic and cell wall binding domains and retained equivalent antibacterial activity (Table 20). The domains of Lib4 were used in another iterative round of modeling and deimmunized library design, yielding Library D, from which 10,000 clones were screened to isolate variant Lib5. Variant Lib5 deleted 13/16 putative DR4 epitopes (Table 19), yet retained 50% wild-type expression and 50% antibacterial activity (Table 20). Compared to variant Opt4, the best enzyme from the individual protein design efforts, Lib5 exhibited both a lower predicted epitope score and higher measured functionality.

Further library construction and screening efforts (Library E) failed to identify additional functional constructs. Therefore, the 13-mutation Lib5 variant was designated the lead candidate for further analysis. It is interesting to note that the three putative epitopes remaining in variant Lib5 (³³YGVDFFMTI⁴¹, SEQ ID NO:215; ³⁸FMTIGTPVK⁴⁶, SEQ ID NO:216; and ¹¹⁶FQRMDNTFS¹²⁴, SEQ ID NO:217) either encompass or are adjacent to amino acids responsible for active site Zn²⁺ coordination (His32, Asp36 and His115). This fact may explain the elusive nature of functional mutations within these regions; screening of diverse library populations identified only one functional substitution in region 33-46 and two in region 116-124 (Table 19).

### Epitope Depleted Designs Display Significantly Reduced Immunogenicity in vivo

The extent to which epitope depletion impacted *in vivo* immunogenicity was subsequently assessed. Anti-LST antibody responses were determined in both C57Bl/6 and transgenic DR4 mice, the latter of which are null for endogenous murine MHC II, but bear a chimeric MHC II receptor derived from human HLA DRB1*0401 (Ito, et al. (1996) J. Exp. Med. 183:2635-44). As a stringent benchmark for deimmunization, mice were immunized subcutaneously with protein in complete Freund's adjuvant, a powerful immunostimulant. Two weeks after a single immunization with LST^{WT}, all DR4 mice mounted a potent anti-LST IgG antibody response, with titers between 1:150 and 1:1700. In contrast, mice immunized with Opt4 showed a striking reduction in titers; only 2/5 mice exhibited any detectable anti-LST antibodies, and even those were substantially reduced relative to LST^{WT} immunized animals. Variant Lib5 also elicited a reduced antibody response; only one animal exhibited high antibody titers (1:1200), three showed significantly lower antibody titers (1:15 to 1:26), and 1 mouse exhibited near background levels of anti-LST antibodies. Importantly, both LST^{WT} and Opt4 were equally immunogenic in the C57Bl/6 laboratory mouse strain, while Lib5 was actually more immunogenic than LST^{WT} (IgG titers 1:4400 to 1:15,000 versus 1:560 to 1: 2100, respectively). Thus, the striking reductions in Opt4 and Lib5 immunogenicity were specifically associated with disruption of molecular recognition by human DR4, as opposed to the native murine MHC II.

It was of note that although the protein design process was blinded to all but allele DR4, ProPred predictions indicated that neither Opt4 nor Lib5 contained neoepitopes for seven other representative human DRB1 alleles (Supp. Fig. S1). In fact, over and above the 11 putative DR4 epitopes deleted from Opt4, predictions suggested that 12 epitopes associated with alleles DR1, DR3, DR7, DR13, and DR15 had also been deleted. Similarly for Lib5, in addition to deletion of 13 DR4-restricted epitopes, ProPred predicted deletion of 18 additional epitopes associated with the other seven DRB1 alleles.

### Lysostaphin Deimmunization Translates into Improved Therapeutic Efficacy

In some embodiments, therapeutic application of LST may require repeated administration to fully eradicate *S*. *aureus* infections. Therefore, to more closely mimic potential clinical applications, the immunogenicity of LST^{WT} and Lib5 were monitored during weekly dosing in the absence of adjuvant. Seven days after a third immunization, all DR4 mice receiving LST^{WT} had mounted a relatively strong immune response, with anti-LST titers in the 1:40 to 1:160 range. During the same time-frame, only one of three mice immunized with Lib5 developed high antibody titers (1:120), where the other two Lib5 mice exhibited titers only marginally above background.

Using an *S. aureus* recurrent bacteremia model, the extent to which LST immunogenicity impacted *in vivo* efficacy was subsequently evaluated. Following determination of antibody titers at week three, the above DR4 mice were infected by intraperitoneal administration of 2x10⁸ colony forming units (CFU) of methicillin-resistant *S. aureus* (MRSA) strain USA400. One hour later, mice were given a 500 µg intravenous bolus of LST^{WT} or Lib5, respectively. Both enzymes rescued their respective groups from this initial infection, whereas a control mouse given a PBS sham treatment had to be sacrificed due to excessive morbidity.

One week later, antibody titers had increased for both the LST^{WT} group (1:300 to 1:650) and the Lib5 group (1 mouse >1:1000, with the remaining two between 1:15 and 1:20), but the latter continued to exhibit a lower overall trend. Mice were now infected with 10⁹ CFU of MRSA and again treated 1 hour later with a 500 µg intravenous bolus of the respective enzyme. In this second infection cycle, where mice had developed higher antibody titers, LST^{WT} failed to rescue any of the three treated mice. Similarly, the single Lib5 mouse exhibiting high antibody titers succumbed to the infection, but the two lower titer Lib5 mice were rescued from the second MRSA challenge.

The following week, antibody titers in the two surviving Lib5 mice were found to have increased yet again (1:70 and 1:120), but notably they remained below the week four LST^{WT} titers. Following a third infection cycle with MRSA, one mouse was treated with Lib5 and survived, whereas the second mouse was given a PBS sham and succumbed to the infection. As a whole, these results showed that humanized DR4 mice mounted a strong immune response to LST^{WT}, even in the absence of adjuvant. During repeated administration, the weekly increase in anti-LST^{WT} antibody titers correlated with loss of efficacy. Conversely, immune responses were attenuated in two of three mice receiving the Lib5 deimmunized variant, and once again *in vivo* efficacy tracked with anti-LST antibody titers. Lib5 exerted potent antibacterial efficacy against as many as three consecutive challenges with MRSA.

Throughout the study, anti-LST antibody titers below 1:256 correlated with enzyme-mediated rescue from *S. aureus* infection, whereas titers above 256 were universally associated with failure of the antibacterial enzyme therapy. Variant Lib5's capacity to mitigate anti-drug antibody responses therefore manifested as enhanced efficacy relative to LST^{WT}.

### Example 3: Deimmunization of Lysostaphin Catalytic and Cell Wall Binding Domains Against HLA Alleles Representative of the HLA Binding Specificity In Human Populations

To deimmunize LST against human HLA alleles DRB1*0101, 0301, 0401, 0701, 0801, 1101, 1301, and 1501, individual variants and libraries predicted to be enriched in functional, deimmunized variants were generated. Epitope mapping of the catalytic domain (Figure 6A) and cell wall binding domain (Figure 6B) was carried out as described in Example 2. Using this information, as well as solvent accessibility and evolutionary conservation, deimmunized LST mutants were generated.

In particular, mutations in the cell wall binding domain (Table 21) were combined with mutations in the catalytic domain of the Flex 9 mutant (Example 1) or a Flex 9 derivative.

**TABLE 21**

| Residue Position | Wild-type | Single Designs | | Library Designs | |
|---|---|---|---|---|---|
| | | 1 | 2 | 1 | 2 |
| 160 | Y | | | N, H, D, Y | |
| 164 | Y | W | | | |
| 166 | S | E | E | | K,N,T,R,S,E,D,A,G |
| 168 | S | K | | | |
| 169 | A | | | E, D, A, G | |
| 186 | R | | | T, R | |
| 193 | V | W | D | E, D, G, V | D, G, V |
| 195 | K | H | H | | |
| 200 | I | T | T | T, I | T, R, I |
| 209 | D | | | | E, D, A, G |
| 214 | V | | | I, M, V, L | |
| 215 | G | | | | E, G |
| 218 | G | | | D, G | D, G |
| 224 | I | | | K, R, I | K, R, I |
| 229 | R | | | R, G | R, G |
| 232 | N | Q | Q | | |
| 236 | N | D | | | N, D |
| 237 | T | K | | | |

Variants were screened and three variants of interest were identified, F11, F12 and F13 (Table 22).

**TABLE 22**

| Variant | Mutations^{#} | SEQ ID NO: |
|---|---|---|
| F11 | N12G, I41E, L83M, K95E, I99Q, N121G, S122D, S124G, S126P*, Y160H, A169G, R186T, N232Q* | 218 |
| F12 | N12G, I41E, L83M, I99Q, N121G, S122D, S124G, S126P*, Y160H, S166N, A169G, R186T, N232Q*, N236D | 219 |
| F13 | N12G, I41E, L83M, K95E, I99Q, N121G, S122D, S124G, S126P*, Y160H, S166N, A169G, R186T, N232Q*, N236D | 220 |

| | | |
|---|---|---|
| *aglycosylation mutations for efficient production in eukaryotic host cells. These mutations are not required for expression in bacterial hosts. ^{#}Relative to wild-type lysostaphin sequence (SEQ ID NO:49). | | |

F11, F12 and F13 were screened for *in vitro* inhibitory activity against MRSA strain USA400 (Figure 7). This analysis indicated that variants F11 and F13 retained 12.5% MIC activity relative to wild-type LST, while variant F12 retained 25% MIC activity relative to wild-type LST. Further, upon heating the variants at 50°C for 1 hour prior to determining activity against MRSA strain USA400, it was found that wild-type LST retained full MIC activity, F13 retained 25% of its original MIC activity, and F12 retained 50% of its original MIC activity.

The *in vivo* efficacy of the F11 variant was further analyzed in C57BL/6 mice. Mice were challenged with an intraperitoneal injection of 2x10⁸ MRSA strain USA400, and 1 hour later the mice were treated with 100 µg wild-type LST, F11 or PBS, administered as a single bolus intravenous injection. The survival rate for both protein treatments was 1/3. The 100 µg dosage was selected because wild-type LST is known to be only partially efficacious at this dose. By using this dose, clear efficacy equivalence for the F11 variant could be demonstrated. This analysis indicated that both proteins had equivalent efficacy *in vivo* (Figure 8).

The immunogenicity of the F13 variant was compared to wild-type LST in DR4 HLA transgenic mice, *i.e.,* mice bearing partially humanized immune systems. 100 µg of wild-type LST or variant F13 were mixed with complete Freund's adjuvant and injected subcutaneously into DR4 mice. Fourteen days later, antibody titers were measured by ELISA. Variant F13 yielded more than 200-fold lower antibody titers compared to wild-type LST. To assess the impact of antibody titers on antibacterial efficacy, the mice were challenged with an intraperitoneal injection of 2x10⁸ MRSA strain USA400, and 1 hour later the mice were treated with 500 µg wild-type LST, variant F13, or PBS, administered as a single bolus intravenous injection. Both mice receiving PBS sham treatments died as did both mice treated with wild-type LST. In contrast, both mice treated with variant F13 survived. Thus, the reduced immunogenicity of F13 conferred enhanced therapeutic efficacy *in vivo.*

In a similar set of experiments, the immunogenicity and efficacy of variant F12 was compared to wild-type LST in the absence of adjuvant using DR4 HLA transgenic mice. At week 0, mice were challenged with an intraperitoneal injection of 2x10⁸ MRSA strain USA400, and 1 hour later the mice were treated with 500 µg wild-type LST or 500 µg variant F12, given as a single bolus subcutaneous injection. Subsequently, mice were challenged weekly with an intraperitoneal injection of 1x10⁹ MRSA strain USA400, and treated as above. Antibody titers were measured weekly beginning at week 2. Wild-type LST was able to rescue mice from a total of four recurrent, systemic, MRSA infections, but failed to rescue any mice from the fifth infection (Table 23). Variant F12 rescued all mice from four recurrent, systemic, MRSA infections (Table 24). The substantially lower antibody titers elicited by variant F12 indicate that this variant is more efficacious than wild-type LST.

**TABLE 23**

| Week | # Mice Infected | # Mice Treated with Enzyme | # Treated Mice Surviving | # Mice Treated with PBS | # Sham Treatment Surviving |
|---|---|---|---|---|---|
| 0 | 7 | 7 | 7 | 0 | NA |
| 1 | 7 | 6 | 6 | 1 | 0 |
| 2 | 6 | 5 | 5 | 1 | 0 |
| 3 | 5* | 4 | 4 | 1 | 0 |
| 4 | 4 | 3 | 0 | 1 | 0 |

| | | | | | |
|---|---|---|---|---|---|
| * ELISA antibody titers measured for only four representative mice out of five total. | | | | | |

**TABLE 24**

| Week | # Mice Infected | # Mice Treated with Enzyme | # Treated Mice Surviving | # Mice Treated with PBS | # Sham Treatment Surviving |
|---|---|---|---|---|---|
| 0 | 10 | 10 | 10 | 0 | NA |
| 1 | 10 | 9 | 9 | 1 | 0 |
| 2 | 9* | 8 | 8 | 1 | 0 |
| 3 | 8 | 7 | 7 | 1 | 0 |
| 4 | 7 | 6 | 6 | 1 | 0 |
| 5 | pending | pending | pending | pending | pending |

| | | | | | |
|---|---|---|---|---|---|
| * ELISA antibody titers measured for only eight of nine total. | | | | | |

## Claims

1. A deimmunized lysostaphin or N-terminal catalytic domain fragment thereof, comprising a mutation at Ser124Gly; Ser122Asp, Ser122Gly, or Ser122Thr; Asn121Gly; Va1120Asp; Arg118Thr; Ile99Gln; Lys95Glu; Tyr93His; Ser84Tyr or Ser84Gly; Leu83Met; Val75Glu or Va175Gln; Asn72His; Ile70Lys; Lys46His; Ile41Glu; Asn40Thr; Phe38Ser; Tyr33Thr; Asn13His; Asn12Gly; or a combination thereof, of SEQ ID NO:49.

2. The deimmunized lysostaphin of claim 1, wherein said lysostaphin is aglycosylated.

3. The deimmunized lysostaphin of claim 1, further comprising one or both of a Thr127Ala mutation or a Ser126Pro mutation.

4. The deimmunized lysostaphin of claim 1, further comprising one or more amino acid substitutions in the C-terminal cell wall binding domain.

5. A pharmaceutical composition comprising the deimmunized lysostaphin of claim 1 and a pharmaceutically acceptable carrier.

6. The pharmaceutical composition of claim 5, further comprising an antibiotic.

7. The pharmaceutical composition of claim 6, wherein said antibiotic comprises a β-lactam, cephalosporin, aminoglycoside, sulfonamide, antifolate, macrolide, quinolone, glycopeptide, polypeptide or a combination thereof.

8. A pharmaceutical composition of claim 5 for use in preventing or treating a microbial infection.

9. The pharmaceutical composition for use according to claim 8, wherein said infection is a bacterial infection.

10. The pharmaceutical composition for use according to claim 9, wherein said bacterial infection is caused by bacteria from the genus *Staphylococcus.*

11. The deimmunized lysostaphin of claim 4, comprising a mutation at one or more of Asn236, Ser234, Asn232, Asn219, Ile200, Ser191, Arg186, Ala169, Ser166, or Tyr160.

12. The deimmunized lysostaphin of claim 4, wherein the mutation comprises Asn236Asp; Ser234Lys; Asn232Gln; Asn219Tyr; Ile200Thr; Ser191Ala; Arg186Thr; Ala169Gly; Ser166Asn or Ser166Thr; Tyr160His; or a combination thereof.

## Patentansprüche

1. Deimmunisiertes Lysostaphin oder N-terminales katalytische-Domäne-Fragment davon,
umfassend eine Mutation bei Ser124Gly; Ser122Asp, Ser122Gly oder Ser122Thr; Asn121Gly; Val120Asp; Arg118Thr; Ile99Gln; Lys95Glu; Tyr93His; Ser84Tyr oder Ser84Gly; Leu83Met; Val75Glu oder Val75Gln; Asn72His; Ile70Lys; Lys46His; Ile41Glu; Asn40Thr; Phe38Ser; Tyr33Thr; Asnl3His; Asnl2Gly; oder eine Kombination davon, von SEQ ID NO:49.

2. Deimmunisiertes Lysostaphin nach Anspruch 1, wobei das Lysostaphin aglycosyliert ist.

3. Deimmunisiertes Lysostaphin nach Anspruch 1, ferner umfassend eine oder beide von einer Thrl27Ala-Mutation und einer Ser126Pro-Mutation.

4. Deimmunisiertes Lysostaphin nach Anspruch 1, ferner umfassend eine oder mehrere Aminosäuresubstitutionen in der C-terminalen Zellwandbindungsdomäne.

5. Pharmazeutische Zusammensetzung umfassend das deimmunisierte Lysostaphin nach Anspruch 1 und einen pharmazeutisch annehmbaren Träger.

6. Pharmazeutische Zusammensetzung nach Anspruch 5, ferner umfassend ein Antibiotikum.

7. Pharmazeutische Zusammensetzung nach Anspruch 6, wobei das Antibiotikum ein β-Lactam, Cephalosporin, Aminoglykosid, Sulfonamid, Antifolat, Makrolid, Chinolon, Glycopeptid, Polypeptid oder eine Kombination davon umfasst.

8. Pharmazeutische Zusammensetzung nach Anspruch 5 zur Verwendung bei der Prävention oder Behandlung einer mikrobiellen Infektion.

9. Pharmazeutische Zusammensetzung zur Verwendung nach Anspruch 8, wobei die Infektion eine bakterielle Infektion ist.

10. Pharmazeutische Zusammensetzung zur Verwendung nach Anspruch 9, wobei die bakterielle Infektion durch Bakterien der Gattung Staphylococcus verursacht ist.

11. Deimmunisiertes Lysostaphin nach Anspruch 4, umfassend eine Mutation an einem oder mehreren von Asn236, Ser234, Asn232, Asn219, Ile200, Ser191, Arg186, Ala169, Ser166 und Tyrl60.

12. Deimmunisiertes Lysostaphin nach Anspruch 4, wobei die Mutation Asn236Asp; Ser234Lys; Asn232Gln; Asn219Tyr; Ile200Thr; Ser191Ala; Arg186Thr; Ala169Gly; Ser166Asn oder Ser166Thr; Tyr160His; oder eine Kombination davon umfasst.

## Revendications

1. Lysostaphine déimmunisée ou fragment de domaine catalytique N-terminal de celle-ci,
comprenant une mutation au niveau de Ser124Gly; Ser122Asp, Ser122Gly, ou Ser122Thr; Asn121Gly; Val120Asp; Arg118Thr; Ile99Gln; Lys95Glu; Tyr93His; Ser84Tyr ou Ser84Gly; Leu83Met; Val75Glu ou Val75Gln; Asn72His; Ile70Lys; Lys46His; Ile41Glu; Asn40Thr; Phe38Ser; Tyr33Thr; Asn13His; Asn12Gly; ou une combinaison correspondante, de SEQ ID NO:49.

2. Lysostaphine déimmunisée selon la revendication 1, dans laquelle ladite lysostaphine est aglycosylée.

3. Lysostaphine déimmunisée selon la revendication 1, comprenant en outre l'une ou les deux d'une mutation Thr127Ala ou d'une mutation Ser126Pro.

4. Lysostaphine déimmunisée selon la revendication 1, comprenant en outre une ou plusieurs substitutions d'acides aminés dans le domaine de liaison de paroi cellulaire C-terminal.

5. Composition pharmaceutique comprenant la lysostaphine déimmunisée selon la revendication 1 et un support pharmaceutiquement acceptable.

6. Composition pharmaceutique selon la revendication 5, comprenant en outre un antibiotique.

7. Composition pharmaceutique de la revendication 6, dans laquelle ledit antibiotique comprend un β-lactame, une céphalosporine, un aminoglycoside, un sulfonamide, un antifolate, un macrolide, une quinolone, un glycopeptide, un polypeptide ou une combinaison de ceux-ci.

8. Composition pharmaceutique selon la revendication 5, destinée à être utilisée dans la prévention ou le traitement d'une infection microbienne.

9. Composition pharmaceutique pour utilisation selon la revendication 8, dans laquelle ladite infection est une infection bactérienne.

10. Composition pharmaceutique destinée à être utilisée selon la revendication 9, dans laquelle ladite infection bactérienne est provoquée par des bactéries du genre Staphylococcus.

11. Lysostaphine déimmunisée de la revendication 4, comprenant une mutation au niveau de l'un ou plusieurs parmi Asn236, Ser234, Asn232, Asn219, Ile200, Ser191, Arg186, Ala169, Ser166, ou Tyr160.

12. Lysostaphine déimmunisée de la revendication 4, dans laquelle la mutation comprend Asn236Asp; Ser234Lys; Asn232Gln; Asn219Tyr; Ile200Thr; Ser191Ala; Arg186Thr; Ala169Gly; Ser166Asn ou Ser166Thr; Tyr160His; ou une combinaison de celles-ci.
